# EUROPEAN PATENT SPECIFICATION

(11) **EP 4 355 357 B1**
(45) Date of publication and mention of the grant of the patent: **25.11.2026**
(21) Application number: 22825819.0
(22) Date of filing: 16.06.2022
(51) Int. Cl.: A61K 38/46, A61K 48/00, C12N 15/00, A61P 21/00, C12Q 1/6806

(54) **SYSTEMS AND METHODS FOR REGULATING ABERRANT GENE EXPRESSIONS**
SYSTEME UND VERFAHREN ZUR REGULIERUNG ABERRANTER GENEXPRESSIONEN
SYSTÈMES ET MÉTHODES DE RÉGULATION D'EXPRESSIONS GÉNIQUES ABERRANTES

(30) Priority: 17.06.2021 US 202163211791 P
(43) Date of publication of application: 24.04.2024
(73) Proprietor: Epicrispr Biotechnologies, Inc., South San Francisco, CA 94080 (US)
(72) Inventor: COLLIN DE L'HORTET, Alexandra Sylvie, South San Francisco, CA 94080 (US); TSOA, Rosemarie Wenting, South San Francisco, CA 94080 (US); ADHIKARI, Abhinav, South San Francisco, CA 94080 (US); BOREGOWDA, Siddaraju, South San Francisco, CA 94080 (US); HART, Daniel O., South San Francisco, CA 94080 (US); GAINOUS, Thomas Blair, South San Francisco, CA 94080 (US); CAROSSO, Giovanni, South San Francisco, CA 94080 (US); YANG, Xiao, South San Francisco, CA 94080 (US); DALEY, Timothy, South San Francisco, CA 94080 (US); LUONG, Thao Nguyen Vu, South San Francisco, CA 94080 (US); LIU, Yanxia, South San Francisco, CA 94080 (US); KO, Tengyu, South San Francisco, CA 94080 (US); SALZMAN, Amber Ruth, South San Francisco, CA 94080 (US)
(74) Representative: Mewburn Ellis LLP
(86) International application number: PCT/US2022/033797
(87) International publication number: WO 2022/266324

(56) References cited:
- WO-A1-2018/057863
- WO-A1-2020/142479
- WO-A1-2022/133062
- WO-A2-2017/180915
- FRANCESCO CHEMELLO ET AL: "Correction of muscular dystrophies by CRISPR gene editing", THE JOURNAL OF CLINICAL INVESTIGATION, vol. 130, no. 6, 1 June 2020 (2020-06-01), pages 2766 - 2776, XP055720476, DOI: 10.1172/JCI136873
- XIAO ET AL.: "Structural basis for substrate recognition and cleavage by the dimerization- dependent CRISPR-Cas12f nuclease", NUCLEIC ACIDS RESEARCH, vol. 49, no. 7, 19 April 2021 (2021-04-19), pages 4120 - 4128, XP055926469, DOI: 10.1093/nar/gkab179

## Description

### BACKGROUND

Aberrant expression of one or more genes can lead to a disease or a condition. In some cases, aberrant expression of a germinal transcription factor in a muscle cell can in a subject can lead to muscular dystrophy. For example, aberrant expression of a transcription factor in a muscle cell (e.g., aberrant expression of DUX4 in a skeletal muscle cell) can lead to Facioscapulohumeral Muscular Dystrophy (FSHD). WO 2018/057863 A1 refers to SpCas9 recruited to the FSHD locus using sgRNA targeting the DUX4 gene.

### SUMMARY

Transiently modifying aberrant expression of a target gene in a cell may not be sufficient to treat or cure a disease that is manifested by the aberrant expression of the target gene. Thus, there remains a substantial need for systems and methods to modify the aberrant expression of the target gene and sustain the modified expression level of the target gene for an extended period of time.

In an aspect, the present disclosure provides a system for regulating aberrant expression of a target gene in a muscle cell, comprising: a heterologous polypeptide comprising a deactivated nuclease, wherein the deactivated nuclease has a length that is less than or equal to 750 amino acids and is a modified variant of Un1Cas12f1; and a guide nucleic acid molecule configured to form a complex with the heterologous polypeptide, wherein the guide nucleic acid molecule exhibits specific binding to a target polynucleotide sequence at or adjacent to, a D4Z4 repeat array in the muscle cell, wherein, upon formation of the complex, the complex is capable of binding the target polynucleotide sequence, to effect modification of an expression level and/or a methylation level of the target gene in the muscle cell, wherein the target gene is within the D4Z4 repeat array.

In some embodiments of any of the systems disclosed herein, upon formation of the complex, the modified expression level and/or methylation level of the target gene in the muscle cell is sustained for at least about 2 days. In some embodiments of any of the systems disclosed herein, the modified expression level and/or methylation level of the target gene is sustained for at least about 3 days, 4 days, 5 days, 6 days, 1 week, 2 weeks, 2 weeks, 4 weeks, or 2 months. In some embodiments of any of the systems disclosed herein, the modified expression level and/or methylation level of the target gene is sustained for at least about 17 days. In some embodiments of any of the systems disclosed herein, the modified expression level and/or methylation level of the target gene is sustained for at least about 18 days.

In some embodiments of any of the systems disclosed herein, the muscle cell is in a subject having or is suspected of having facioscapulohumeral muscular dystrophy (FSHD). In some embodiments of any of the systems disclosed herein, the target gene is Dux4.

The nuclease is a modified variant of Un1Cas12f1. In some embodiments of any of the systems disclosed herein, the nuclease comprises an amino acid sequence that is at least about 80%, at least about 90%, at least about 95%, or at least about 99% identical to the polypeptide sequence of SEQ ID NO: 43. In some embodiments of any of the systems disclosed herein, the nuclease comprises an amino acid sequence that is at least about 80%, at least about 90%, at least about 95%, or at least about 99% identical to the polypeptide sequence of SEQ ID NO: 44.

In some embodiments of any of the systems disclosed herein, the heterologous polypeptide further comprises a transcriptional regulator. In some embodiments of any of the systems disclosed herein, the transcriptional regulator comprises at least one methyltransferases. In some embodiments of any of the systems disclosed herein, the transcriptional regulator comprises at least one DNA Methyltransferases (DNMT). In some embodiments of any of the systems disclosed herein, the transcriptional regulator comprises DNMT-A or DNMT-L. In some embodiments of any of the systems disclosed herein, the transcriptional regulator comprises (i) DNMT-A or DNMT-L and (ii) KRAB or a variant of KRAB. In some embodiments of any of the systems disclosed herein, the transcriptional regulator comprises (i) DNMT-L and (ii) KRAB or a variant of KRAB. In some embodiments of any of the systems disclosed herein, the transcriptional regulator comprises DNMT-A, DNMT-L, and KRAB or a variant of KRAB. In some embodiments of any of the systems disclosed herein, the transcriptional regulator comprises DNMT-L or KRAB or variant of KRAB. In some embodiments of any of the systems disclosed herein, the transcriptional regulator comprises KRAB or a variant of KRAB. In some embodiments of any of the systems disclosed herein, the transcriptional regulator comprises a plurality of different transcriptional regulators.

In some embodiments of any of the systems disclosed herein, the modification of the expression level and/or the methylation level of the target gene effects downregulation of a downstream gene of the target gene, wherein the downstream gene comprises one or more members selected from the group consisting of ZSCAN4, LEUTX, MBD3L2, TRIM48, and TRIM43.

In some embodiments of any of the systems disclosed herein, the modification of the expression level and/or the methylation level of the target gene effects downregulation of an apoptosis marker in the muscle cell. In some embodiments of any of the systems disclosed herein, the apoptosis marker comprises Caspase 3.

In some embodiments of any of the systems disclosed herein, the complex effects the modification of the expression level of the target gene in the muscle gene. In some embodiments of any of the systems disclosed herein, the modification of the expression level results in downregulation of the target gene.

In some embodiments of any of the systems disclosed herein, the complex effects the modification of the methylation level of the target gene in the muscle gene. In some embodiments of any of the systems disclosed herein, the modification of the methylation level results in downregulation of the target gene.

In some embodiments of any of the systems disclosed herein, the nuclease is a deactivated nuclease.

In another aspect, the present disclosure provides a composition comprising any of the systems disclosed herein.

In another aspect, the present disclosure provides a viral vector comprising any of the systems or any of the compositions disclosed herein.

In some embodiments of any of the viral vectors disclosed herein, the viral vector comprises an adeno-associated virus (AAVs), a retrovirus, a lentivirus, a poxvirus, or an adenovirus. In some embodiments of any of the viral vectors disclosed herein, the AAV comprises a AAV serotype RH74 AAV.

In another aspect, the present disclosure provides an *in vitro* or *ex vivo* method for regulating aberrant expression of a target gene in a muscle cell, the method comprising (a) contacting the muscle cell with a complex comprising (i) a heterologous polypeptide comprising a deactivated nuclease, wherein the deactivated nuclease has a length that is less than or equal to 750 amino acids and is a modified variant of Un1Cas12f1 and (ii) a guide nucleic acid molecule exhibiting specific binding to a target polynucleotide sequence at or adjacent to, a D4Z4 repeat array in the muscle cell; and (b) upon the contacting, binding the target gene with the complex to effect modification of an expression level and/or a methylation level of the target gene in the muscle cell, wherein the target gene is within the D4Z4 repeat array.

In another aspect, the present disclosure provides a complex comprising (i) a heterologous polypeptide comprising a deactivated nuclease, wherein the deactivated nuclease has a length that is less than or equal to 750 amino acids and is a modified variant of Un1Cas12f1 and (ii) a guide nucleic acid molecule exhibiting specific binding to a target polynucleotide sequence at or adjacent to, a D4Z4 repeat array in the muscle cell, for use in a method for regulating aberrant expression of a target gene in a muscle cell, the method comprising (a) contacting the muscle cell with the complex of the heterologous polypeptide and the guide nucleic acid molecule; and (b) upon the contacting, binding the target gene with the complex to effect modification of the expression level and/or the methylation level of the target gene in the muscle cell.

In some embodiments of any of the methods disclosed herein, upon formation of the complex, the modified expression level and/or methylation level of the target gene in the muscle cell is sustained for at least about 2 days. In some embodiments of any of the methods disclosed herein, the modified expression level and/or methylation level of the target gene is sustained for at least about 3 days, 4 days, 5 days, 6 days, 1 week, 2 weeks, 2 weeks, 4 weeks, or 2 months. In some embodiments of any of the methods disclosed herein, the modified expression level and/or methylation level of the target gene is sustained for at least about 17 days. In some embodiments of any of the methods disclosed herein, the modified expression level and/or methylation level of the target gene is sustained for at least about 18 days.

In some embodiments of any of the methods disclosed herein, the contacting comprises injecting a composition comprising the complex to a subject in need thereof, wherein the subject has or is suspected of having facioscapulohumeral muscular dystrophy (FSHD). In some embodiments of any of the methods disclosed herein, the target gene is Dux4.

As set out in the claims, the nuclease of any of the methods disclosed herein has a length that is less than or equal to about 750 amino acids.

As set out in the claims, the nuclease of any of the methods disclosed herein is a modified variant of Un1Cas12f1.

In some embodiments of any of the methods disclosed herein, the nuclease comprises an amino acid sequence that is at least about 80%, at least about 90%, at least about 95%, or at least about 99% identical to the polypeptide sequence of SEQ ID NO: 43. In some embodiments of any of the methods disclosed herein, the nuclease comprises an amino acid sequence that is at least about 80%, at least about 90%, at least about 95%, or at least about 99% identical to the polypeptide sequence of SEQ ID NO: 44.

In some embodiments of any of the methods disclosed herein, the heterologous polypeptide further comprises a transcriptional regulator. In some embodiments of any of the methods disclosed herein, the transcriptional regulator comprises at least one methyltransferases. In some embodiments of any of the methods disclosed herein, the transcriptional regulator comprises at least one DNA Methyltransferases (DNMT). In some embodiments of any of the methods disclosed herein, the transcriptional regulator comprises DNMT-A or DNMT-L. In some embodiments of any of the methods disclosed herein, the transcriptional regulator comprises (i) DNMT-A or DNMT-L and (ii) KRAB or a variant of KRAB. In some embodiments of any of the methods disclosed herein, the transcriptional regulator comprises (i) DNMT-L and (ii) KRAB or a variant of KRAB. In some embodiments of any of the methods disclosed herein, the transcriptional regulator comprises DNMT-A, DNMT-L, and KRAB or variant of KRAB. In some embodiments of any of the methods disclosed herein, the transcriptional regulator comprises DNMT-L or KRAB or a variant of KRAB. In some embodiments of any of the methods disclosed herein, the transcriptional regulator comprises KRAB or a variant of KRAB. In some embodiments of any of the methods disclosed herein, the transcriptional regulator comprises a plurality of different transcriptional regulators.

In some embodiments of any of the methods disclosed herein, the modification of the expression level and/or the methylation level of the target gene effects downregulation of a downstream gene of the target gene, wherein the downstream gene comprises one or more members selected from the group consisting of ZSCAN4, LEUTX, MBD3L2, TRIM48, and TRIM43.

In some embodiments of any of the methods disclosed herein, the modification of the expression level and/or the methylation level of the target gene effects downregulation of an apoptosis marker in the muscle cell. In some embodiments of any of the methods disclosed herein, the apoptosis marker comprises Caspase 3.

In some embodiments of any of the methods disclosed herein, the complex effects the modification of the expression level of the target gene in the muscle gene.

In some embodiments of any of the methods disclosed herein, the modification of the expression level results in downregulation of the target gene.

In some embodiments of any of the methods disclosed herein, the complex effects the modification of the methylation level of the target gene in the muscle gene. In some embodiments of any of the methods disclosed herein, the modification of the methylation level results in downregulation of the target gene.

As set out in the claims, in the methods disclosed herein, the nuclease is a deactivated nuclease.

Disclosed herein is a system for regulating aberrant expression of a target gene in a muscle cell, the system comprising: a heterologous actuator moiety coupled to a gene regulator, wherein the heterologous actuator moiety is capable of forming a complex with the target gene in the muscle cell, and wherein the gene regulator is capable of modifying an expression level and/or a methylation level of the target gene in the muscle cell, wherein, upon formation of the complex, the modified expression level and/or methylation level of the target gene in the muscle cell is sustained for at least about 2 days.

In some embodiments of any of the systems disclosed herein, the sustained modified expression level and/or the methylation level of the target gene is characterized by maintaining at least about 70%, 75%, 80%, 85%, 90%, 95%, 99%, or 100% of the modified expression level and/or methylation level of the target gene.

In some embodiments of any of the systems disclosed herein, the modified expression level and/or methylation level of the target gene is sustained for at least about 3 days, 4 days, 5 days, 6 days, 1 week, 2 weeks, 3 weeks, 4 weeks, or 2 months.

In some embodiments of any of the systems disclosed herein, the modified expression level of the target gene is a decreased expression level of the target gene.

In some embodiments of any of the systems disclosed herein, the modified methylation level of the target gene is an increased degree of methylation of the target gene.

In some embodiments of any of the systems disclosed herein, the gene regulator comprises an epigenetic regulator. In some embodiments of any of the systems disclosed herein, the epigenetic regulator comprises a chromatin modifier. In some embodiments of any of the systems disclosed herein, the epigenetic regulator comprises at least one methyltransferases. In some embodiments of any of the systems disclosed herein, the epigenetic regulator comprises at least one DNA Methyltransferases (DNMT). In some embodiments of any of the systems disclosed herein, the epigenetic regulator comprises DNMT-A or DNMT-L. In some embodiments of any of the systems disclosed herein, the epigenetic regulator comprises (i) DNMT-A or DNMT-L and (ii) KRAB or a variant of KRAB. In some embodiments of any of the systems disclosed herein, the epigenetic regulator comprises DNMT-A, DNMT-L, and KRAB or a variant of KRAB. In some embodiments of any of the systems disclosed herein, the epigenetic regular comprises of KRAB or a variant of KRAB. In some embodiments of any of the systems disclosed herein, the gene regulator comprises a plurality of different gene regulators.

In some embodiments of any of the systems disclosed herein, the system further comprises a guide nucleic acid molecule capable of directing the heterologous actuator moiety to the target gene, to form the complex.

In some embodiments of any of the systems disclosed herein, the heterologous actuator moiety is capable of forming a complex with a first portion of the muscle-regulating gene, and wherein the system further comprises an additional heterologous actuator moiety coupled to an additional gene regulator, wherein the additional heterologous actuator moiety is capable of forming a complex with a second portion of the muscle-regulating gene.

In some embodiments of any of the systems disclosed herein, the system further comprises an additional guide nucleic acid molecule capable of directing the additional heterologous actuator moiety to the second portion of the muscle-regulating gene.

In some embodiments of any of the systems disclosed herein, the target gene is a transcription factor.

As set out in the claims, the target gene is within a D4Z4 repeat array. In some embodiments of any of the systems disclosed herein, the target gene encodes DUX4.

In some embodiments of any of the systems disclosed herein, the target gene is not C9orf72.

In some embodiments of any of the systems disclosed herein, the muscle cell is a skeletal muscle cell.

In some embodiments of any of the systems disclosed herein, the heterologous actuator moiety or the additional heterologous actuator moiety comprises an endonuclease. In some embodiments of any of the systems disclosed herein, the heterologous actuator moiety or the additional heterologous actuator moiety comprises a CRISPR-Cas protein. In some embodiments of any of the systems disclosed herein, the heterologous actuator moiety or the additional heterologous actuator moiety comprises a dCas protein. In some embodiments of any of the systems disclosed herein, the guide nucleic acid molecule or the additional guide nucleic acid molecule comprises a guide RNA molecule.

In another aspect, the present disclosure provides a complex of the heterologous polypeptide and a guide nucleic acid molecule as set forth in the claims for use in a method for regulating aberrant expression of a target gene in a muscle cell, the method comprising: (a) contacting the muscle cell with a heterologous actuator moiety coupled to a gene regulator, wherein the heterologous actuator moiety is capable of forming a complex with the target gene in the muscle cell, and wherein the gene regulator is capable of modifying an expression level and/or a methylation level of the target gene in the muscle cell; and (b) upon formation of the complex, sustaining the modified expression level and/or methylation level of the target gene in the muscle cell for at least about 2 days.

In some embodiments of any of the methods disclosed herein, the sustained modified expression level and/or the methylation level of the target gene is characterized by maintaining at least about 70%, 75%, 80%, 85%, 90%, 95%, 99%, or 100% of the modified expression level and/or methylation level of the target gene.

In some embodiments of any of the methods disclosed herein, the modified expression level and/or methylation level of the target gene is sustained for at least about 3 days, 4 days, 5 days, 6 days, 1 week, 2 weeks, 3 weeks, 4 weeks, or 2 months.

In some embodiments of any of the methods disclosed herein, the modified expression level of the target gene is a decreased expression level of the target gene.

In some embodiments of any of the methods disclosed herein, the modified methylation level of the target gene is an increased degree of methylation of the target gene.

In some embodiments of any of the methods disclosed herein, the gene regulator comprises an epigenetic regulator. In some embodiments of any of the methods disclosed herein, the epigenetic regulator comprises a chromatin modifier. In some embodiments of any of the methods disclosed herein, the epigenetic regulator comprises at least one methyltransferases. In some embodiments of any of the methods disclosed herein, the epigenetic regulator comprises at least one DNA Methyltransferases (DNMT). In some embodiments of any of the methods disclosed herein, the epigenetic regulator comprises DNMT-A or DNMT-L. In some embodiments of any of the methods disclosed herein, the epigenetic regulator comprises (i) DNMT-A or DNMT-L and (ii) KRAB or a variant of KRAB. In some embodiments of any of the methods disclosed herein, the epigenetic regulator comprises DNMT-A, DNMT-L, and KRAB or a variant of KRAB. In some embodiments of any of the methods disclosed herein, the epigenetic regular comprises of KRAB or a variant of KRAB. In some embodiments of any of the methods disclosed herein, the gene regulator comprises a plurality of different gene regulators.

In some embodiments of any of the methods disclosed herein, the method further comprises contacting the muscle cell with a guide nucleic acid molecule capable of directing the heterologous actuator moiety to the target gene, to form the complex.

In some embodiments of any of the methods disclosed herein, the heterologous actuator moiety is capable of forming a complex with a first portion of the muscle-regulating gene, and wherein the method further comprises contacting the muscle cell with an additional heterologous actuator moiety coupled to an additional gene regulator, wherein the additional heterologous actuator moiety is capable of forming a complex with a second portion of the muscle-regulating gene.

In some embodiments of any of the methods disclosed herein, the method further comprises contacting the muscle cell with an additional guide nucleic acid molecule capable of directing the additional heterologous actuator moiety to the second portion of the muscle-regulating gene.

In some embodiments of any of the methods disclosed herein, the target gene is a transcription factor.

As set out in the claims, in the methods disclosed herein, the target gene is within a D4Z4 repeat array. In some embodiments of any of the methods disclosed herein, the target gene encodes DUX4.
In some embodiments of any of the methods disclosed herein, the target gene is not C9orf72.

In some embodiments of any of the methods disclosed herein, the muscle cell is a skeletal muscle cell.

In some embodiments of any of the methods disclosed herein, the heterologous actuator moiety or the additional heterologous actuator moiety comprises an endonuclease. In some embodiments of any of the methods disclosed herein, the heterologous actuator moiety or the additional heterologous actuator moiety comprises a CRISPR-Cas protein. In some embodiments of any of the methods disclosed herein, the heterologous actuator moiety or the additional heterologous actuator moiety comprises a dCas protein. In some embodiments of any of the methods disclosed herein, the guide nucleic acid molecule or the additional guide nucleic acid molecule comprises a guide RNA molecule.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the invention are utilized, and the accompanying drawings of which:

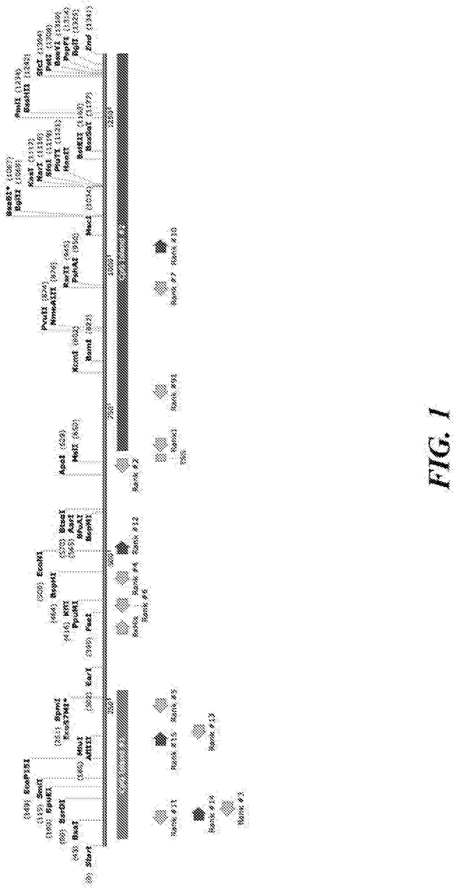

**FIG. 1** provides different target polynucleotide sequences (e.g., Rank #1 through Rank #91) between two CpG islands within a D4Z4 repeat array that encodes DUX4.

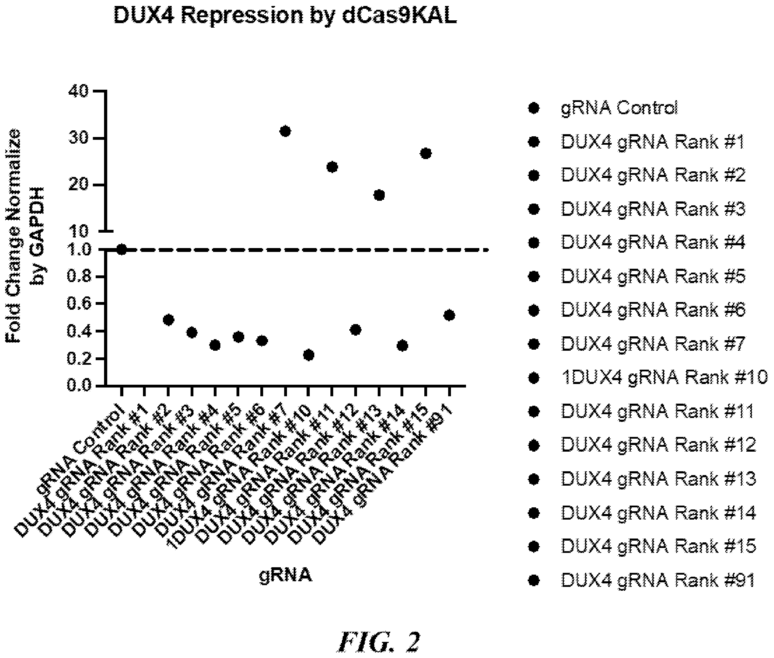

**FIG. 2** provides regulation of DUX4 expression in a target cell population (e.g., lymphoblasts) by a heterologous actuator moiety coupled to a gene regulator (e.g., dCas-KRAB-DNMT3A-DNMT3L) that is complexed with various guide RNA molecules target polynucleotide sequences (e.g., Rank #1 through Rank #91) within the D4Z4 repeat array that encodes DUX4.

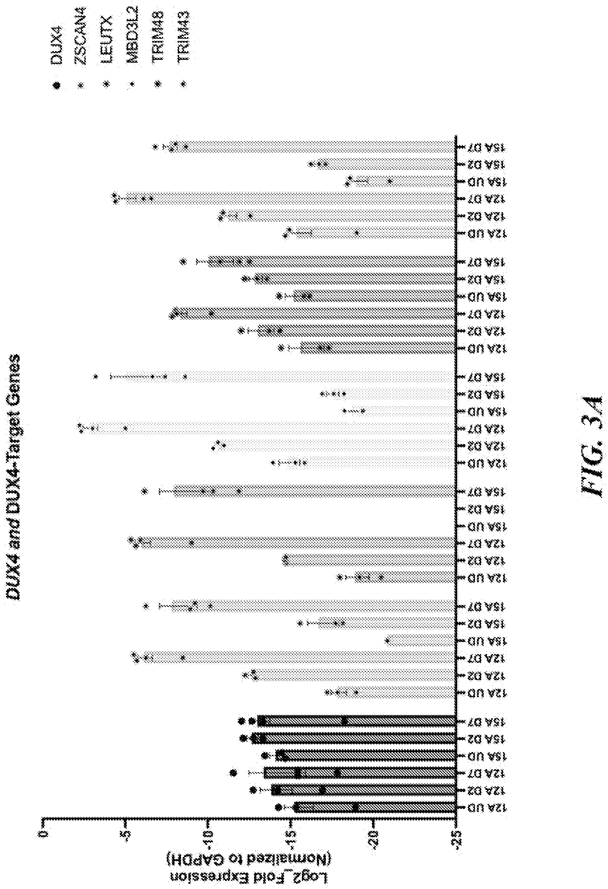

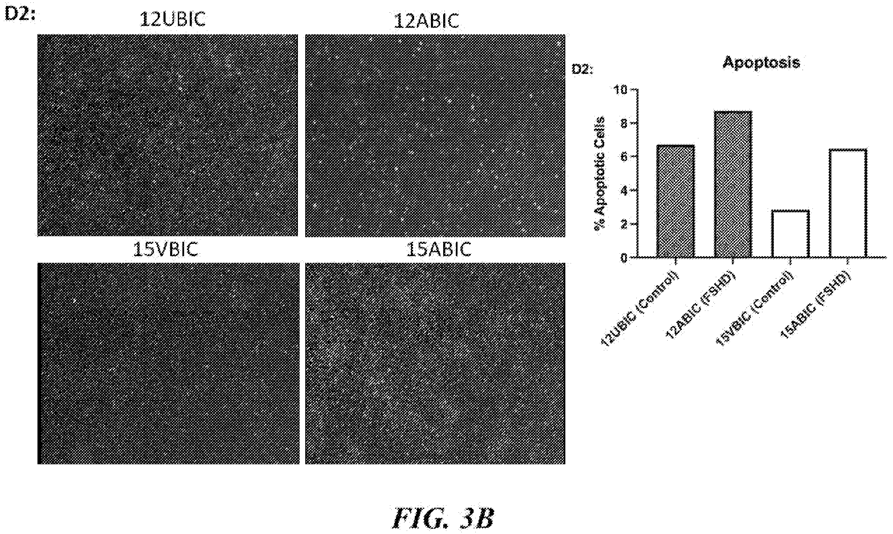

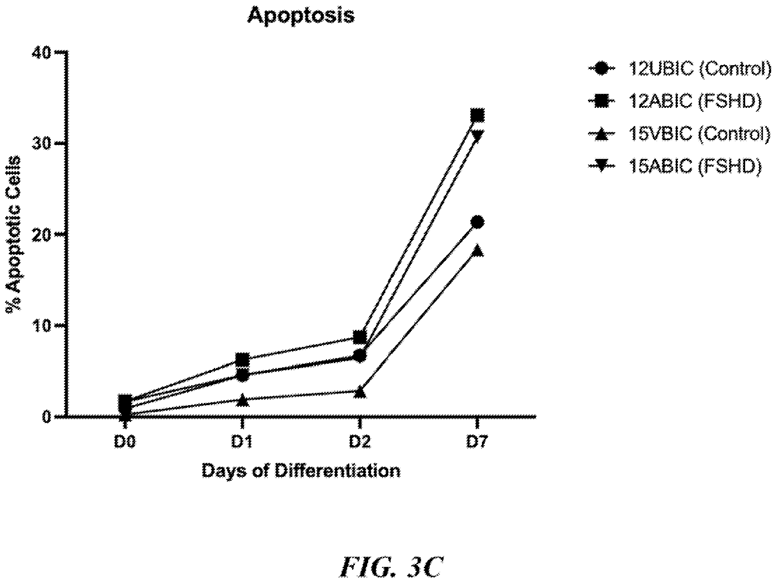

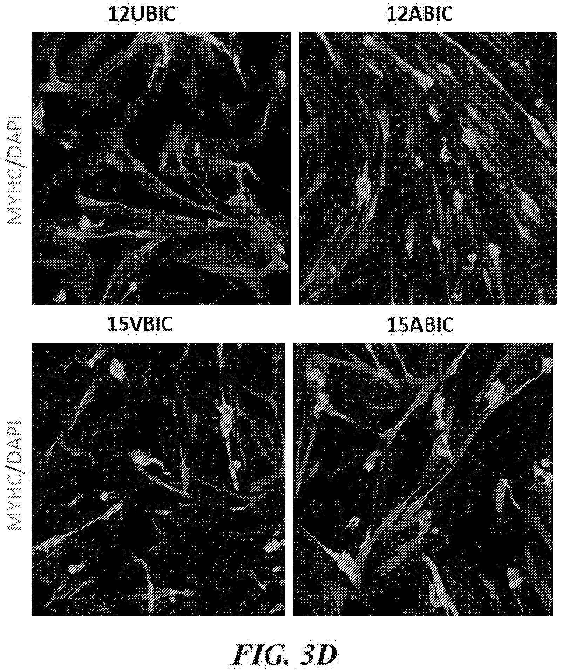

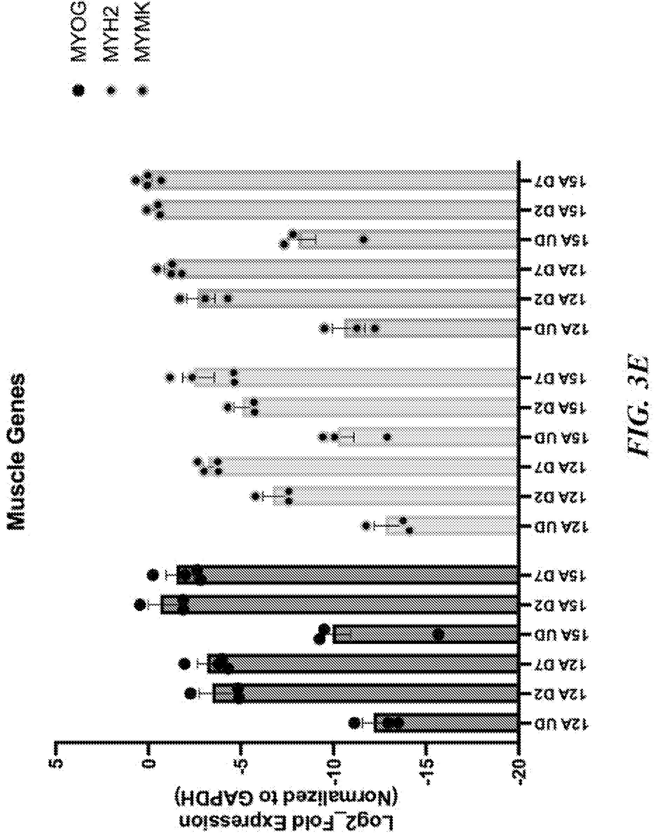

**FIG. 3A** depicts the gene expression of DUX4 and DUX4-target genes in immortalized patient-derived human FSHD skeletal myoblasts (SkM) cells (12ABIC/12A and 15ABIC/15A). The gene expression of DUX4 and DUX4-target genes is measured in 12ABIC and 15ABIC undifferentiated cells, 12ABIC and 15ABIC cells after 2 days of differentiation, and 12ABIC and 15ABIC cells after 7 days of differentiation. Each shade of gray on the graph depicts the gene expression for a different gene corresponding to the legend on the right. **FIG. 3B** depicts the proportion of apoptotic cells in FSHD myoblasts 12ABIC and 15ABIC (right column) compared to their healthy sibling control myoblasts, 12UBIC and 15VBIC, respectively (left column) after two days of differentiation. The white dots in the images on the left represent apoptotic cells. The graph on the right depicts the percentage of apoptotic cells in the 12ABIC, 15ABIC, 12UBIC, and 15VBIC cell cultures after two days of differentiation shown in the images on the left. DAPI stain is used to stain the for nuclei. **FIG. 3C** depicts the percentage of apoptotic cells in 12ABIC, 15ABIC, 12UBIC, and 15VBIC cells after seven days of differentiation. Percentage of apoptotic cells are measured on day 0, day 1, day 2, and day 7 of differentiation. **FIG. 3D** depicts the expression of MYHC in 12ABIC, 15ABIC, 12UBIC, and 15 VBIC cells after 7 days of differentiation. Myosin Heavy Chain (MYHC) is a marker for muscle cell differentiation. The white dots indicate expression of MYHC. **FIG. 3E** depicts the expression level of MYOG, MYH2, and MYMK in 12ABIC, 15ABIC, 12UBIC, and 15VBIC cells after 7 days of differentiation. MYOG is a myogenic regulatory factor that regulates skeletal muscle differentiation and MyoMaker (MYMK) is a marker for muscle cell differentiation. DAPI stain is used to stain the for nuclei. Expression level for the 12ABIC and 15ABIC cells is measured on day 2 and day 7 of differentiation. 12A UD and 15A UD: undifferentiated, proliferating control myoblasts. Dark gray bars depicts MYOG expression levels, light gray bars depicts MYH2 expression levels, and gray bars depicts MYMK expression levels.

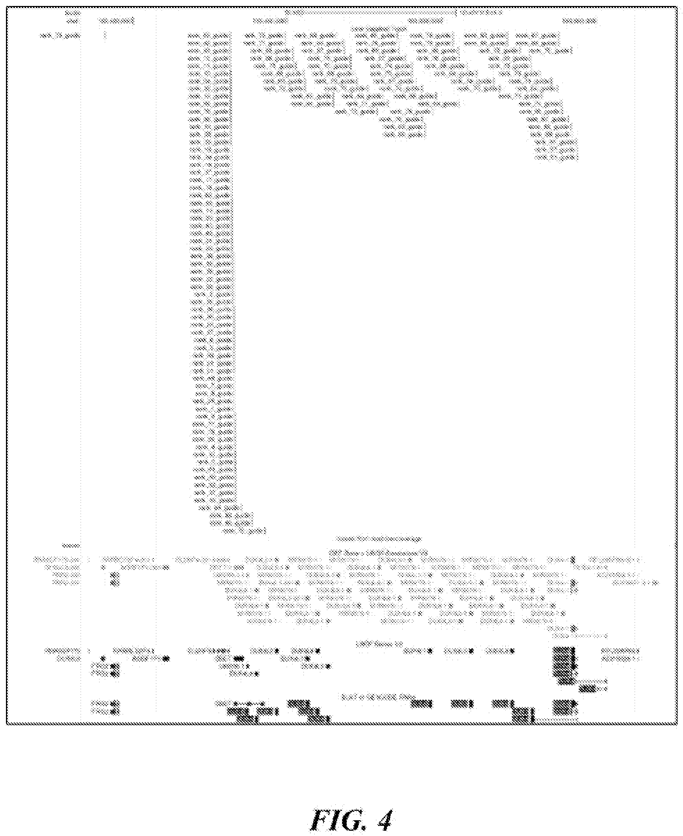

**FIG. 4** depicts the design of multiple gRNAs in relation to the D4Z4 repeat region. The multiple DUX4-targeting gRNAs are designed to span across the DZ4Z repeat region. The DZ4Z repeat region and the DUX4 gene locations in relation to each other is shown at the bottom of **FIG. 4****.** The newly designed gRNAs are shown at the top of **FIG. 4****.**

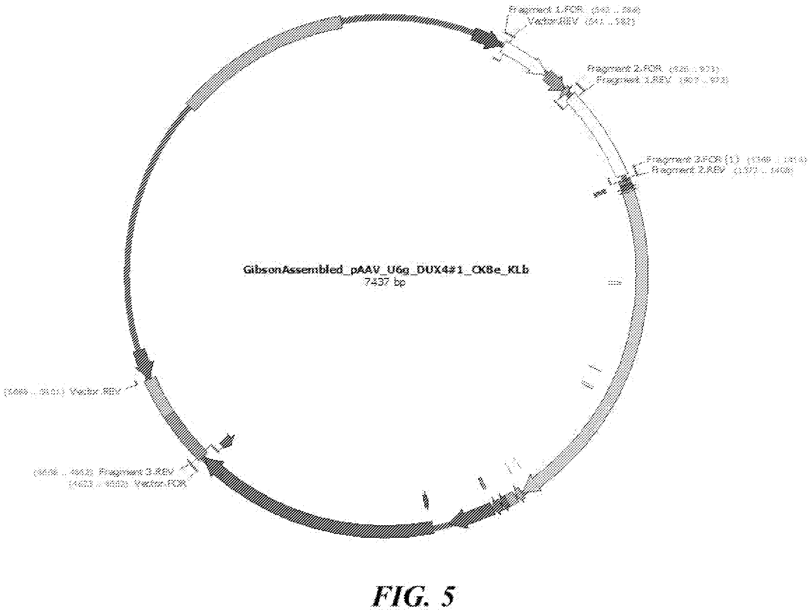

**FIG. 5** depicts the Cas12f effector-modulator vector design. The expression of the Cas12f variant, KRAB domain, and DNMT3L domain are under the control of a muscle-specific promoter, CK8e. The expression of sgRNA spacer sequence with scaffold driven by RNA polymerase III is under the control of a human U6g promoter. The vector additionally includes a modified WPRE and polyadenylation regulatory sequences.

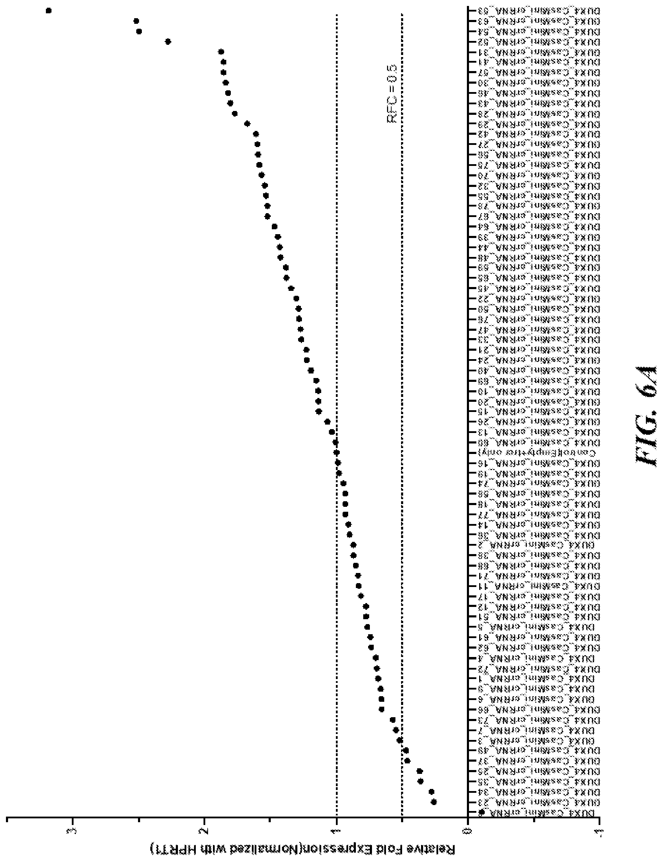

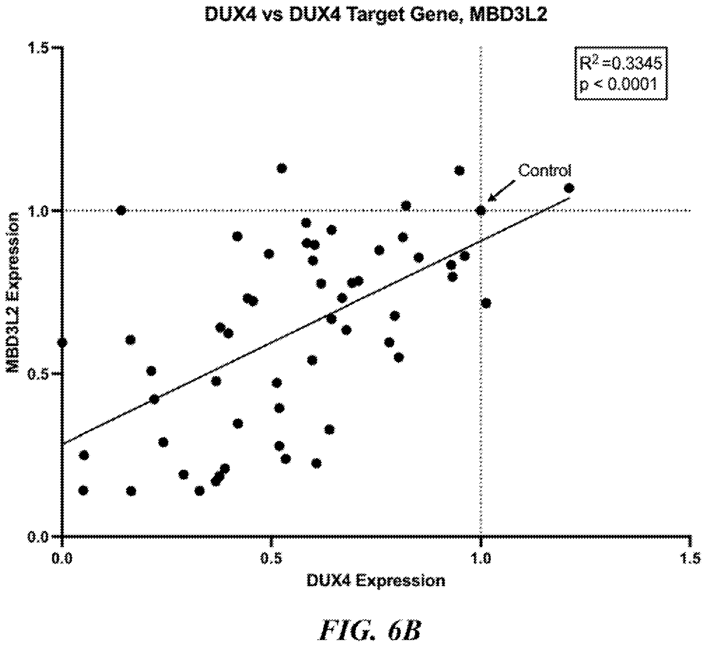

**FIG. 6A** depicts the relative expression level of DUX4 in 12ABIC FSHD myoblasts that stably express the Cas12f-KRAB effector-modulator after 78 gRNAs were nucleofected into the 12ABIC myoblasts. Following nucleofection, the cells are cultured in differentiation conditions for 7 days before the gene expression of DUX4 is measured. The 78 gRNAs tested are listed on the x-axis and the y-axis represents the relative fold expression of DUX4. The expression level of DUX4 was normalized with the expression of control gene HPRT1. **FIG. 6B** depicts the relative expression level of DUX4 in 12ABIC FSHD myoblasts that stably express the Cas12f-KRAB effector-modulator after 78 gRNAs were nucleofected into the 12ABIC myoblasts. Following nucleofection, the cells are cultured in differentiation conditions for 7 days before the gene expression of DUX4 and the DUX4-target gene, MBD3L2, is measured.

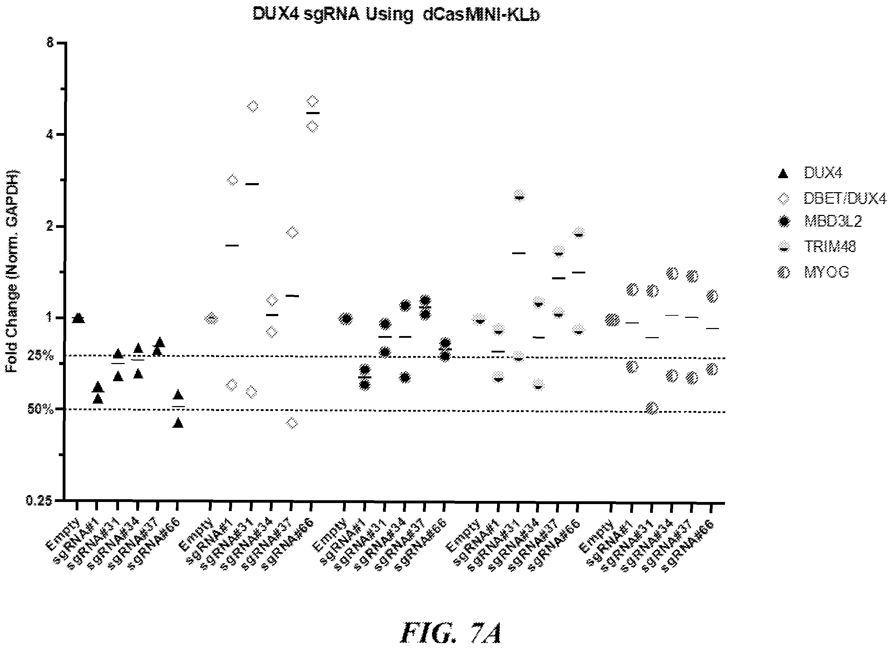

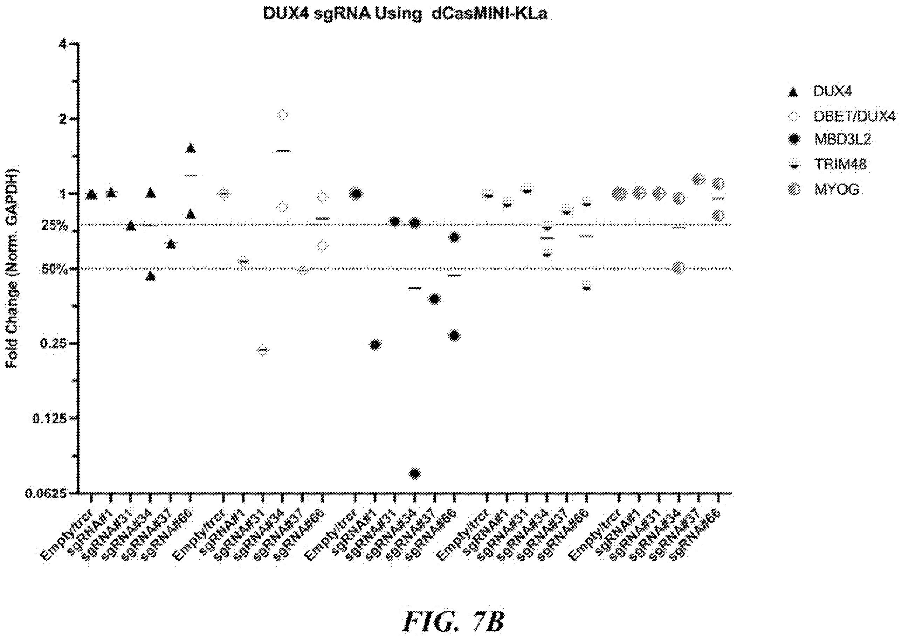

**FIG. 7A** depicts the repression of DUX4 and DUX4-target genes, DBET/DUX4, MBD3L2, and TRIM48, in immortalized patient-derived FSHD myoblasts transfected with six gRNAs and a Cas12f effector-modulator. The Cas12f effector-modulator expresses a Cas12f variant, a KRAB domain, and a DNMT-KLa domain. One of the six sgRNA is a control sgRNA (Empty/trcr) which did not target the DZ4Z repeat region. Expression level of MYOG is measured in the cells to assay if the differentiation ability of DUX4 sgRNA transfected cells is similar to control sgRNA transfected myoblasts. Expression level of DUX4, DUX4-target genes, and MYOG is measured 17 days post transfection. **FIG. 7B** depicts the repression of DUX4 and DUX4-target genes, DBET/DUX4, MBD3L2, and TRIM48, in immortalized patient-derived FSHD myoblasts transfected with six gRNAs and a Cas12f effector-modulator. The Cas12f effector-modulator expresses a Cas12f variant, a KRAB domain, and a DNMT-KLb domain. One of the six sgRNA is a control sgRNA (Empty) which did not target the DZ4Z repeat region. Expression level of MYOG is measured in the cells to assay if the differentiation ability of DUX4 sgRNA transfected cells is similar to control sgRNA transfected myoblasts. Expression level of DUX4, DUX4-target genes, and MYOG is measured 18 days post transfection.

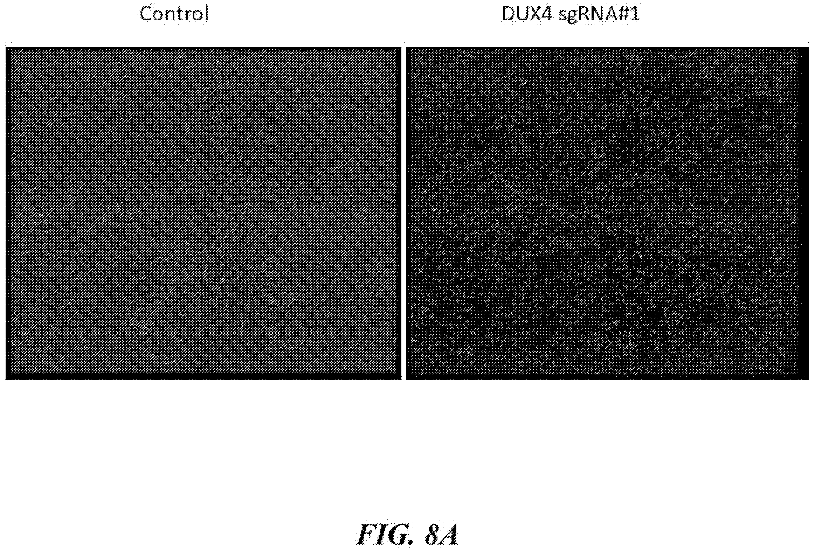

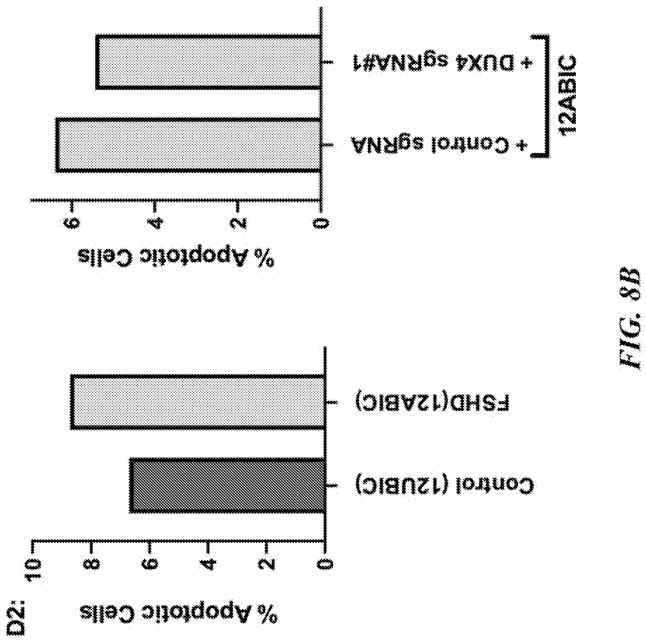

**FIGs. 8A** and **8B** depict the apoptosis level of FSHD-patient derived myoblasts transfected with Cas12f effector-modulator and DUX4-targeting gRNA. The percentage of apoptotic-positive cells is measured after two days of differentiation following transfection. The images in **FIG. 8A** depicts the proportion of apoptotic cells in control 12UBIC cells and 12ABIC cells transfected with the Cas12f effector-modulator and DUX4-targeting gRNA. The white dots depict apoptotic cells. The graphs in **FIG. 8B** depict the percentage of apoptotic cells measured in the images on the left, as well as the percentage of apoptotic cells in 12ABIC cells transfected with either a DUX4-targeting gRNA or a control gRNA, which does not target DUX4. DAPI stain is used to stain the for nuclei.

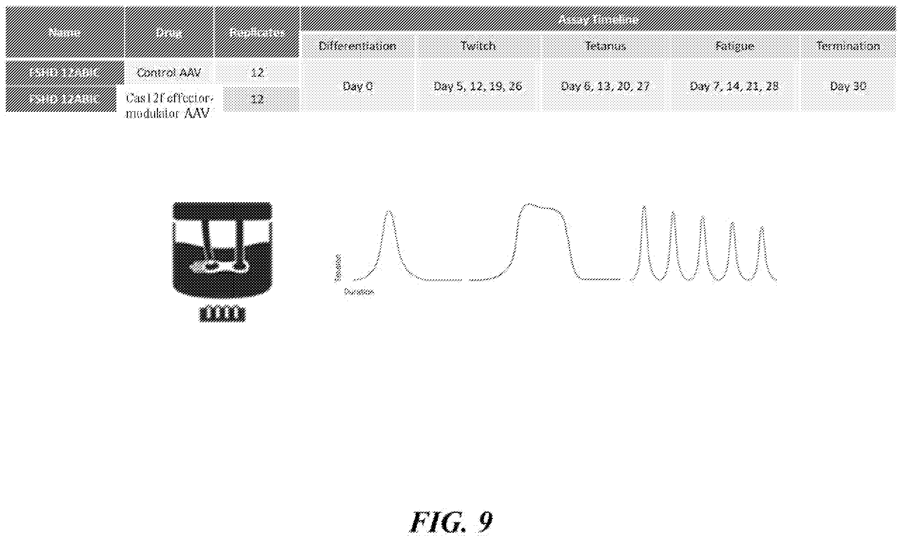

**FIG. 9** depicts the workflow for an *ex vivo* FSHD model. The *ex vivo* model cultures immortalized healthly sibling control cells and FSHD skeletal myoblasts and then engineers the cells into 3D tissues. The 3D tissues are treated with either a control AAV or a AAV with the Cas12f effector-modulator vector. The 3D tissues are then tested for phenotypic differences in mechanical force, tetanic force, and fatigue, in addition to measuring 3D tissue morphology and gene expression profile.

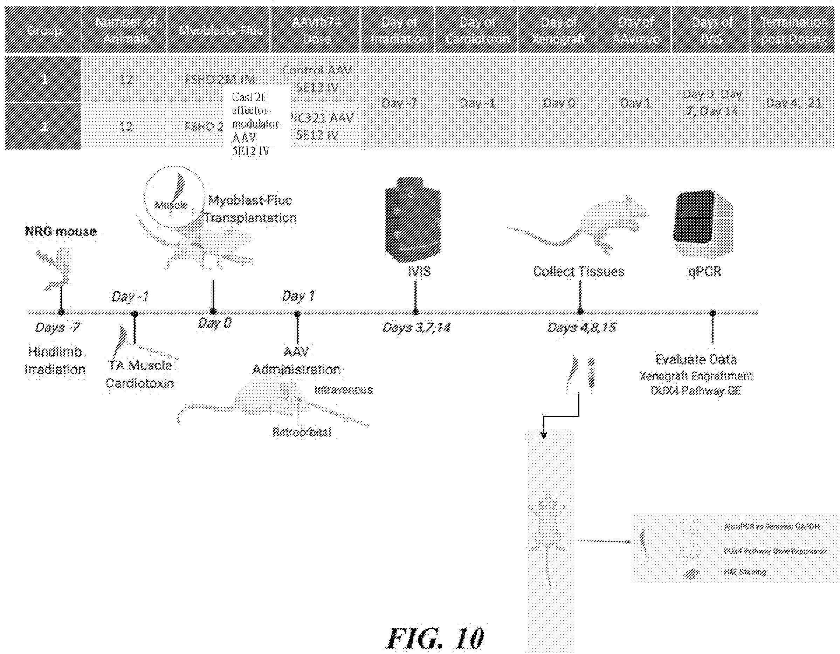

**FIG. 10** depicts the workflow for an *in vivo* xenograft model. The *in vivo* model begins with treating mice legs with irradiation and TA muscle cardiotoxin to prepare for the transplantation of human myoblast cells into the mice's leg. Following transplantation, the mice are treated with either a control AAV or a AAV with the Cas12f effector-modulator vector. At designated time points, the mice are euthanized, and the xenograft and tissue samples are collected for analysis. The collected xenograft is fixed, sectioned, and stained with Hematoxylin and eosin. The remaining tissues are used for gene expression assays, as well as determining AAV tropism within the mice.

### DETAILED DESCRIPTION

Aberrant expression of one or more genes can lead to a disease or a condition. The aberrant expression can be characterized by aberrantly low expression level of the gene(s). Alternatively, the aberrant expression can be characterized by aberrantly high expression level of the gene(s). In some cases, the gene(s) can be genetically modified (e.g., via action of endonucleases, such as CRISPR-Cas enzymes) to reverse the aberrant expression (e.g., for treatment of Duchenne muscular dystrophy (DMD)). Alternatively, the aberrant expression can be transiently modified without genetically modifying such gene(s) of interest, e.g., by targeting the gene(s) with gene effectors (e.g., deactivated CRISPR-Cas enzyme that is coupled to a gene effector). Transiently modifying aberrant expression of a target gene in a cell may not be sufficient to treat or cure a disease that is manifested by the aberrant expression of the target gene. Thus, disclosed herein are systems and methods for modifying the aberrant expression of the target gene, such that the modified expression level of the target gene may be sustained for an extended period of time.

### MODIFICATION OF ABERRANT EXPRESSION OF A TARGET GENE

The present disclosure provides compositions, systems, and methods thereof for regulating aberrant expression of a target gene in a muscle cell. As set out in the claims, the target gene is within a D4Z4 repeat array. The target gene can encode at least a portion of DUX4. The compositions, systems, and methods disclosed herein utilize at least a heterologous polypeptide (e.g., a heterologous actuator moiety)and a guide nucleic acid molecule) to modify an expression level and/or a methylation level of the target gene. For example, the compositions, systems, and methods disclosed herein can utilize a heterologous actuator moiety that is operatively coupled (e.g., covalently or non-covalently coupled) to a heterologous gene effector or regulator (e.g., gene actuator, gene repressor, etc.) to modify an expression level and/or a epigenetic modification level of the target gene.

As set out in the claims, the cell is a muscle cell. A muscle cell as disclosed herein can be any classification of muscle cells at any state of development. The muscle cell can comprise undifferentiated muscle cells (e.g., mononucleated cells, such as muscle stem cells, muscle satellite cells, myoblasts, etc.). Alternatively or in addition to, the muscle cell can comprise differentiated muscle cells (e.g. multinucleated muscle cells, such as myotubes). The muscle cell can be a skeletal muscle cell, a cardiac muscle cell, or a smooth muscle cell. For example, the skeletal muscle cell can be a primary myoblast (e.g., an immortalized primary myoblast cell line).

In some cases, the target gene can be in chromosome number 4 of the cell as disclosed herein. In some cases, the target gene can be in chromosome number 10 of the cell, such as a distal portion of the q (long) arm of the chromosome number 10 of the cell.

Prior to the modification of the target gene as disclosed herein, the aberrant expression of the target gene can be characterized by an expression level and/or epigenetic modification level (e.g., methylation level) of the target gene that is higher than that in a control cell (e.g., a healthy cell in a healthy subject) by at least about 1%, 5%, 10%, 20%, 50%, 100%, 150%, 200%, 300%, 400%, 500%, or more. The aberrant expression can be characterized by an expression level and/or epigenetic modification level (e.g., methylation level) of the target gene that is higher than that in a control cell (e.g., a healthy cell in a healthy subject) by at most about 500%, 400%, 300%, 200%, 150%, 100%, 50%, 20%, 10%, 5%, 1%, or less.

Prior to the modification of the target gene as disclosed herein, the aberrant expression of the target gene can be characterized by an expression level and/or epigenetic modification level (e.g., methylation level) of the target gene that is lower than that in a control cell (e.g., a healthy cell in a healthy subject) by at least about 1%, 5%, 10%, 20%, 30%, 40%, 50%, 70%, 99%, or more. The aberrant expression can be characterized by an expression level and/or epigenetic modification level (e.g., methylation level) of the target gene that is lower than that in a control cell (e.g., a healthy cell in a healthy subject) by at most about 100%, 70%, 50%, 40%, 30%, 20%, 10%, 5%, 1%, or less.

Prior to the modification of the target gene as disclosed herein, the aberrant expression of the target gene can be characterized by a duration of an expression level and/or epigenetic modification level (e.g., methylation level) of the target gene that is longer than that in a control cell (e.g., a healthy cell in a healthy subject) by at least about 1%, 5%, 10%, 20%, 50%, 100%, 150%, 200%, 300%, 400%, 500%, or more. The aberrant expression can be characterized by a duration of an expression level and/or epigenetic modification level (e.g., methylation level) of the target gene that is longer than that in a control cell (e.g., a healthy cell in a healthy subject) by at most about 500%, 400%, 300%, 200%, 150%, 100%, 50%, 20%, 10%, 5%, 1%, or less.

Prior to the modification of the target gene as disclosed herein, the aberrant expression of the target gene can be characterized by a duration of an expression level and/or epigenetic modification level (e.g., methylation level) of the target gene that is shorter than that in a control cell (e.g., a healthy cell in a healthy subject) by at least about 1%, 5%, 10%, 20%, 30%, 40%, 50%, 70%, 99%, or more. The aberrant expression can be characterized by a duration of an expression level and/or epigenetic modification level (e.g., methylation level) of the target gene that is shorter than that in a control cell (e.g., a healthy cell in a healthy subject) by at most about 100%, 70%, 50%, 40%, 30%, 20%, 10%, 5%, 1%, or less.

Subsequent to the modification of the target gene as disclosed herein, modification of the aberrant expression of the target gene can be characterized by an increased expression level and/or epigenetic modification level (e.g., methylation level) of the target gene by at least about 1%, 5%, 10%, 20%, 50%, 100%, 150%, 200%, 300%, 400%, 500%, or more, as compared to a control (e.g., without the modification). The modification of the aberrant expression of the target gene can be characterized by an increased expression level and/or epigenetic modification level (e.g., methylation level) of the target gene by at most about 500%, 400%, 300%, 200%, 150%, 100%, 50%, 20%, 10%, 5%, 1%, or less, as compared to a control.

Subsequent to the modification of the target gene as disclosed herein, modification of the aberrant expression of the target gene can be characterized by a decreased expression level and/or epigenetic modification level (e.g., methylation level) of the target gene by at least about 1%, 5%, 10%, 20%, 30%, 40%, 50%, 70%, 99%, or more, as compared to a control (e.g., without the modification). The modification of the aberrant expression of the target gene can be characterized by a decreased expression level and/or epigenetic modification level (e.g., methylation level) of the target gene by at most about 100%, 70%, 50%, 40%, 30%, 20%, 10%, 5%, 1%, or less.

Subsequent to the modification of the target gene as disclosed herein, modification of the aberrant expression of the target gene can be characterized by an increased duration of an expression level and/or epigenetic modification level (e.g., methylation level) of the target gene by at least about 1%, 5%, 10%, 20%, 50%, 100%, 150%, 200%, 300%, 400%, 500%, or more, as compared to a control (e.g., without the modification). The modification of the aberrant expression of the target gene can be characterized by an increased duration of an expression level and/or epigenetic modification level (e.g., methylation level) of the target gene by at most about 500%, 400%, 300%, 200%, 150%, 100%, 50%, 20%, 10%, 5%, 1%, or less, as compared to a control.

Subsequent to the modification of the target gene as disclosed herein, modification of the aberrant expression of the target gene can be characterized by a decreased duration of an expression level and/or epigenetic modification level (e.g., methylation level) of the target gene by at least about 1%, 5%, 10%, 20%, 30%, 40%, 50%, 70%, 99%, or more, as compared to a control (e.g., without the modification). The modification of the aberrant expression of the target gene can be characterized by a decreased duration of an expression level and/or epigenetic modification level (e.g., methylation level) of the target gene by at most about 100%, 70%, 50%, 40%, 30%, 20%, 10%, 5%, 1%, or less.

Subsequent to the modification of the target gene as disclosed herein, the modified expression level and/or epigenetic modification level (e.g., methylation level) of the target gene (e.g., the aberrantly expressed target gene) can be sustained for at least about 1 day, 2 days, 3 days, 4 days, 5 days, 6 days, 7 days, 2 weeks, 3 weeks, 4 weeks, 2 months, 3 months, 4 months, 5 months, 6 months, 7 months, 8 months, 9 months, 10 months, 11 months, 12 months, 2 years, 3 years, 4 years, 5 years, or more. Subsequent to the modification of the target gene as disclosed herein, the modified expression level and/or epigenetic modification level (e.g., methylation level) of the target gene (e.g., the aberrantly expressed target gene) can be sustained for at most about 5 years, 4 years, 3 years, 2 years, 12 months, 11 months, 10 months, 9 months, 8 months, 7 months, 6 months, 5 months, 4 months, 3 months, 2 months, 4 weeks, 3 weeks, 2 weeks, 7 days, 6 days, 5 days, 4 days, 3 days, 2 days, 1 day, or less.

Subsequent to the modification of the target gene as disclosed herein, the modified expression level and/or epigenetic modification level (e.g., methylation level) of the target gene (e.g., the aberrantly expressed target gene) can be sustained for at least about 1 cell division, at least about 2 cell divisions, at least about 3 cell divisions, at least about 4 cell divisions, at least about 5 cell divisions, at least about 6 cell divisions, at least about 7 cell divisions, at least about 8 cell divisions, at least about 9 cell divisions, at least about 10 cell divisions, at least about 15 cell divisions, at least about 20 cell divisions, at least about 25 cell divisions, at least about 30 cell divisions, at least about 40 cell divisions, at least about 50 cell divisions, or at least about 100 cell divisions. Subsequent to the modification of the target gene as disclosed herein, the modified expression level and/or epigenetic modification level (e.g., methylation level) of the target gene (e.g., the aberrantly expressed target gene) can be sustained for at most about 100 cell divisions, at most about 50 cell divisions, at most about 40 cell divisions, at most about 30 cell divisions, at most about 25 cell divisions, at most about 20 cell divisions, at most about 15 cell divisions, at most about 10 cell divisions, at most about 9 cell divisions, at most about 8 cell divisions, at most about 7 cell divisions, at most about 6 cell divisions, at most about 5 cell divisions, at most about 4 cell divisions, at most about 3 cell divisions, at most about 2 cell division, or at most about 1 cell division.

As disclosed herein, non-limiting examples of the epigenetic modification can include methylation, acetylation, phosphorylation, ADP-ribosylation, glycosylation, SUMOylation, ubiquitination, modification of histone structure (e.g., via an ATP hydrolysis-dependent process). For example, the epigenetic modification can result in a modified methylation level of one or more target genes.

As disclosed herein, the sustained modified expression level and/or epigenetic modification level (e.g., methylation level) of the target gene can be characterized by maintaining at least about 70%, 75%, 80%, 85%, 90%, 95%, 96%, 97%, 98%, 99%, or 100% of the modified expression level and/or methylation level of the target gene. The sustained modified expression level and/or epigenetic modification level (e.g., methylation level) of the target gene can be characterized by maintaining at most about 100%, 99%, 98%, 97%, 96%, 95%, 90%, 85%, 80%, 75%, or 70% of the modified expression level and/or methylation level of the target gene.

The systems, compositions, and methods as disclosed herein can be used to treat or ameliorate a disease (e.g., muscular dystrophy, such as Facioscapulohumeral Muscular Dystrophy (FSHD)) of a subject.

### HETEROLOGOUS POLYPEPTIDES

As set forth in the claims, the system comprises a heterologous polypeptide comprising a deactivated nuclease, wherein the deactivated nuclease has a length that is less than or equal to 750 amino acids and is a modified variant of Un1Cas12f1, which upon forming a complex with a guide RNA that exhibits specific binding to a target polynucleotide sequence at or adjacent to, a D4Z4 repeat array in the muscle cell, is capable of binding the target polynucleotide sequence, to effect modification of an expression level and/or a methylation level of the target gene in the muscle cell, wherein the target gene is within the D4Z4 repeat array.
Also disclosed herein is a heterologous polypeptide which, either alone or in conjunction with one or more co-agents such as a heterologous polynucleotide (e.g., a guide nucleic acid) can be configured to specifically bind a target polynucleotide sequence, to modulate an expression level and/or an epigenetic level of the target gene (e.g., the D4Z4 repeat array) in the target cell, as disclosed herein. The target polynucleotide sequence can be at (e.g., within) the target gene. Alternatively, the target polynucleotide sequence can be adjacent to the target gene. For example, the target polynucleotide sequence can be adjacent to an end (e.g., a 5' end or a 3' end) of the target gene. The target polynucleotide sequence can be at least about 5 nucleobases, at least about 10 nucleobases, at least about 20 nucleobases, at least about 30 nucleobases, at least about 40 nucleobases, at least about 50 nucleobases, at least about 100 nucleobases, at least about 150 nucleobases, at least about 200 nucleobases, at least about 250 nucleobases, at least about 300 nucleobases, at least about 400 nucleobases, at least about 500 nucleobases, at least about 1,000 nucleobases, at least about 1,500 nucleobases, at least about 2,000 nucleobases, at least about 3,000 nucleobases, at least about 4,000 nucleobases, or at least about 5,000 nucleobases away from the end of the target gene. The target polynucleotide sequence can be at most about 5,000 nucleobases, at most about 4,000 nucleobases, at most about 3,000 nucleobases, at most about 2,000 nucleobases, at most about 1,500 nucleobases, at most about 1,000 nucleobases, at most about 500 nucleobases, at most about 400 nucleobases, at most about 300 nucleobases, at most about 200 nucleobases, at most about 150 nucleobases, at most about 100 nucleobases, at most about 50 nucleobases, at most about 40 nucleobases, at most about 30 nucleobases, at most about 20 nucleobases, at most about 10 nucleobases, or at most about 5 nucleobases away from the end of the target gene.

Without wishing to be bound by theory, when the target polynucleotide sequence is not within the target gene, the target polynucleotide sequence can interact (e.g., via direct or indirect binding) with at least a portion of the target gene (e.g., a promoter sequence of the target gene), such that binding or targeting of the target polynucleotide sequence by at least the heterologous polypeptide (e.g., by a complex comprising the heterologous polypeptide and the heterologous polynucleotide as disclosed herein) can target the at least the portion of the target gene (e.g., the promoter sequence), to effect the modulation of the expression level and/or the epigenetic level of the target gene in the cell.

In some cases, the target polynucleotide sequence can comprise a plurality of target polynucleotide sequences. The plurality of target polynucleotide sequences can be within the target gene. Alternatively, the plurality of target polynucleotide sequences can be outside but adjacent to the target gene, as disclosed herein. Yet in another alternative, the plurality of target polynucleotide sequences can comprise at least one target polynucleotide sequence within the target gene (e.g., within the D4Z4 repeat domain) and at least one additional target polynucleotide sequence that is outside of but adjacent to the target gene. In such case, targeting both the at least one target polynucleotide sequence and the at least one additional target polynucleotide sequence may yield a greater effect (e.g., greater degree of modulation of the expression and/or epigenetic level of the target gene) (e.g., by at least 0.1-fold, at least 0.5-fold, at least 1-fold, at least 2-fold, at least 3-fold, at least 4-fold, at least 5-fold, at least 10-fold, at least 15-fold, at least 20-fold, or more) as compared to that by targeting only one of the at least one target polynucleotide sequence and the at least one additional target polynucleotide sequence.

The heterologous polypeptide as disclosed herein can comprise one or more heterologous gene effectors (e.g., gene effectors that are heterologous to a cell comprising the gene effectors and/or another component in a complex of the disclosure). Heterologous gene effectors can comprise domains that are capable of, or are candidates for, modulating expression of a target gene (e.g., a target endogenous gene), for example, activating, repressing, upregulating, downregulating, or stabilizing an expression level or activity level of the gene. Heterologous gene effectors can be heterologous with respect to another component that is present in a complex, for example, a guide moiety (e.g., nuclease and/or guide nucleic acid, as disclosed herein). In some cases, heterologous gene effectors can be heterologous with respect to a host cell they are introduced to.

A heterologous gene effector can be or can comprise a sequence from any suitable source, for example, an amino acid sequence from a human protein, viral protein, or other protein as disclosed herein. A heterologous gene effector can be or can comprise a sequence from a protein that primarily localized to the nucleus, for example, a member of the human nuclear proteome. A heterologous gene effector can be or can comprise one or more natural amino acid residues. A heterologous gene effector can be or can comprise one or more synthetic amino acid residues.

A heterologous gene effector can be or can comprise a sequence from a mammalian protein. A heterologous gene effector can be or can comprise a sequence from a human protein. A heterologous gene effector can be or can comprise a sequence from a viral protein. A heterologous gene effector can be or can comprise a sequence from a non-human primate protein. A heterologous gene effector can be or can comprise a sequence from a non-human mammal protein. A heterologous gene effector can be or can comprise a sequence from a non-rodent mammal protein. A heterologous gene effector can be or can comprise a sequence from a plant protein. A heterologous gene effector can be or can comprise a sequence from a pig protein. A heterologous gene effector can be or can comprise a sequence from a lagomorph protein. A heterologous gene effector can be or can comprise a sequence from a canine protein. A heterologous gene effector can be or can comprise a sequence from an avian protein. A heterologous gene effector can be or can comprise a sequence from a reptilian protein. A heterologous gene effector can be or can comprise a sequence from a bacterial protein. A heterologous gene effector can be or can comprise a sequence from an archaeal protein.

For example, the amino acid sequence of the heterologous gene effector as disclosed herein may not and need not be derived from a bacterial protein (e.g., may be derived from an archaeal protein). Without wishing to be bound by theory, a subject in need thereof may be treated with a composition comprising the non-bacterial protein-derived heterologous gene effector, such that the composition may not (i) induce a bacterial stimulus in the subject and/or (ii) elicit a bacterial immune response in the subject.

As set forth in the claims, the heterologous polypeptide comprises a deactivated nuclease, wherein the deactivated nuclease has a length that is less than or equal to 750 amino acids and is a modified variant of Un1Cas12f1. Also disclosed herein is a heterologous actuator moiety that can comprise a nuclease (e.g., an endonuclease). For example, the nuclease can be a CRISPR/Cas protein. The nuclease can have a length that is less than a threshold length. The threshold length can be at most about 1,000 amino acids, at most about 950 amino acids, at most about 900 amino acids, at most about 850 amino acids, at most about 800 amino acids, at most about 750 amino acids, at most about 700 amino acids, at most about 650 amino acids, at most about 600 amino acids, at most about 550 amino acids, at most about 500 amino acids, at most about 450 amino acids, at most about 400 amino acids, at most about 350 amino acids, or at most about 300 amino acids. The threshold length can be at least about 300 amino acids, at least about 350 amino acids, at least about 400 amino acids, at least about 450 amino acids, at least about 500 amino acids, at least about 550 amino acids, at least about 600 amino acids, at least about 650 amino acids, at least about 700 amino acids, at least about 750 amino acids, at least about 800 amino acids, at least about 850 amino acids, at least about 900 amino acids, at least about 950 amino acids, or at least about 1,000 amino acids.

Without wishing to be bound by theory, using a size of the nuclease to be less than the threshold length can have one or advantages over using a control nuclease having a size greater than the threshold length. When using a delivery vehicle having a limited size (e.g., a limited physical size to entrap the nuclease or a limited expression cassette size, such as a viral genome) can leave sufficient room (or sufficient space within the expression cassette) for on or more co-agents, such as one or more gene regulators (e.g., transcriptional regulator) and/or one or more heterologous polynucleotides (e.g., one or more guide nucleic acid molecules). Alternatively or in addition to, using the nuclease having a size less than or equal to the threshold size can elicit a greater effect on the modulation of the expression level and/or the epigenetic level of the target gene, as compared to the effect on the modulation of the expression level and/or the epigenetic level of the target gene by a control nuclease having a size greater than the threshold size.

In some examples, the degree of modulation (e.g., increase or decrease) of the expression level and/or the epigenetic level of the target gene by the nuclease as disclosed herein (e.g., having a size less than or equal to the threshold size) can be greater than that by the control nuclease (e.g., having a size greater than the threshold size) by at least or up to about 0.1-fold, at least or up to about 0.5-fold, at least or up to about 1-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 25-fold, at least or up to about 30-fold, at least or up to about 40-fold, at least or up to about 50-fold, at least or up to about 60-fold, at least or up to about 70-fold, at least or up to about 80-fold, at least or up to about 90-fold, or at least or up to about 100-fold.

In some examples, the degree of modulation (e.g., increase or decrease) of the expression level and/or the epigenetic level of the target gene by the nuclease as disclosed herein (e.g., having a size less than or equal to the threshold size) can persist longer than that by the control nuclease (e.g., having a size greater than the threshold size) by at least or up to about 0.1-fold, at least or up to about 0.5-fold, at least or up to about 1-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 25-fold, at least or up to about 30-fold, at least or up to about 40-fold, at least or up to about 50-fold, at least or up to about 60-fold, at least or up to about 70-fold, at least or up to about 80-fold, at least or up to about 90-fold, or at least or up to about 100-fold.

In some examples, the degree of modulation (e.g., increase or decrease) of the expression level and/or the epigenetic level of the target gene by the nuclease as disclosed herein (e.g., having a size less than or equal to the threshold size) can persist longer than (or sustained longer than) that by the control nuclease (e.g., having a size greater than the threshold size) by at least or up to about 1 cell division, at least or up to about 2 cell divisions, at least or up to about 3 cell divisions, at least or up to about 4 cell divisions, at least or up to about 5 cell divisions, at least or up to about 6 cell divisions, at least or up to about 7 cell divisions, at least or up to about 8 cell divisions, at least or up to about 9 cell divisions, at least or up to about 10 cell divisions, at least or up to about 11 cell divisions, at least or up to about 12 cell divisions, at least or up to about 13 cell divisions, at least or up to about 14 cell divisions, at least or up to about 15 cell divisions, at least or up to about 16 cell divisions, at least or up to about 17 cell divisions, at least or up to about 18 cell divisions, at least or up to about 19 cell divisions, at least or up to about 20 cell divisions, at least or up to about 25 cell divisions, at least or up to about 30 cell divisions, at least or up to about 40 cell divisions, at least or up to about 50 cell divisions, or at least about 100 cell divisions. As disclosed herein, a cell division can be characterized by a division of a parent cell into two daughter cells with substantially the same genetic material as the parent cell.

A heterologous gene effector can be or can comprise a sequence from a chromatic regulator (CR). Chromatin regulators include functional domains from various classes of histone and DNA modifying enzymes (e.g., DNMTs, HATs, HMTs, etc.).

A heterologous gene effector can comprise two or more domains from chromatin regulators, e.g., located at a C-terminus, an N-terminus, or within a polypeptide sequence, in tandem or separate.

In some embodiments, a heterologous gene effector that facilitates heterochromatin formation. Non-limiting examples of proteins that can facilitate heterochromatin formation include HP1α, HP1β, KAP1, KRAB, SUV39H1, and G9a.

In some embodiments, a heterologous gene effector modulates histones through methylation. In some embodiments, a heterologous gene effector modulates histones through acetylation. In some embodiments, a heterologous gene effector modulates histones through phosphorylation. In some embodiments, a heterologous gene effector modulates histones through ADP-ribosylation. In some embodiments, a heterologous gene effector modulates histones through glycosylation. In some embodiments, a heterologous gene effector modulates histones through SUMOylation. In some embodiments, a heterologous gene effector modulates histones through ubiquitination. In some embodiments, a heterologous gene effector modulates histones by remodeling histone structure, e.g., via an ATP hydrolysis-dependent process.

In some embodiments, a heterologous gene effector facilitates spatial positioning of proteins on or near the target polynucleotide, e.g., transcriptional repressors, transcription factors, histones, etc. In some embodiments, a heterologous gene effector is useful for manipulating the spatiotemporal organization of genomic DNA and RNA components in the nucleus and/or cytoplasm, e.g., for regulating diverse cellular functions.

In some embodiments, a heterologous gene effector is from a histone acetyltransferase. Non-limiting examples of histone acetyltransferases include GNAT subfamily, MYST subfamily, p300/CBP subfamily, HAT1 subfamily, GCN5, PCAF, Tip60, MOZ, MORF, MOF, HBO1, p300, CBP, HAT1, ATF-2, SRC1, and TAFII250.

In some embodiments, a heterologous gene effector is from a histone lysine methyltransferase. Non-limiting examples of histone lysine methyltransferases include EZH subfamily, Non-SET subfamily, Other SET subfamily, PRDM subfamily, SET1 subfamily, SET2 subfamily, SUV39 subfamily, SYMD subfamily, ASH1L, EHMT1, EHMT2, EZH1, EZH2, MLL, MLL2, MLL3, MLL4, MLL5, NSD1, NSD2, NSD3, PRDM1, PRDM10, PRDM11, PRDM12, PRDM13, PRDM14, PRDM15, PRDM16, PRDM2, PRDM4, PRDM5, PRDM6, PRDM7, PRDM8, PRDM9, SET1, SET1L, SET2L, SETD2, SETD3, SETD4, SETD5, SETD6, SETD7, SETD8, SETDB1, SETDB2, SETMAR, SUV39H1, SUV39H2, SUV420H1, SUV420H2, SYMD1, SYMD2, SYMD3, SYMD4, and SYMD5.

In some embodiments, a heterologous gene effector is from a component of a chromatin remodeling complex. In some embodiments, a heterologous gene effector is a component of BAF, for example, Actin, ARIDA/B, BAF155, BAF170, BAF45 A/B/C/D, BAF53 A/B, BAF57, BAF60 A/B/C, BRG1/BRM, INI1, or SS18.

In some embodiments, a heterologous gene effector is from a component of PBAF, for example, Actin, ARID2, BAF155, BAF170, BAF180, BAF45 A/B/C/D, BAF53 A/B, BAF57, BAF60 A/B/C, BRD7, BRG1, or INI1.

In some embodiments, a heterologous gene effector is from a component of an ISWI family chromatin remodeling complex, for example, ACF subfamily, RSF subfamily, CERF subfamily, CHRAC subfamily, NURF subfamily, NoRC subfamily, WICH subfamily, b-WICH subfamily, ACF1, ATPase, BPTF, CECR2, CHRAC15, CHRAC17, CSB, DEK, MYBBP1A, NM1, RBAP46/48, RHII/Gua, RSF1, SAP155, SNF2H, SNF2H/L, SNF2L, TIP5, or WSTF.

In some embodiments, a heterologous gene effector is from a component of a CHD family complex, for example, a NuRD complex, NuRD-like complex, or CHD complex. In some embodiments, a heterologous gene effector is from CHD1/2/6/7/8/9, CHD3/4, CHD5, GATAD2 A/B, GATAD2 B, HDAC1, HDAC2, HDAC2, MBD2/3, MTA1/2/3, MTA3, or RBAP46, RBAP46/48.

In some embodiments, a heterologous gene effector is from a component of an INO80 family complex, for example, from an INO80 complex, Tip60/p400 complex, SRCAP complex, AMIDA, ARP6, BAF53, BAF53, BAF53A, BRD8, DMAP1, DMAP1, EPC1/2, FLJ11730, GAS41, GAS41, IES2, IES6, ING3, IN080, INO80E, MCRS1, MRG15, MRGBP, MRGX, NFRKB, p400, RUVBL1/2, RUVBL1/2, RUVBL1/2, SRCAP, Tip60, TRRAP, UCH37, YL-1, YL-1, YY1, or ZnF-HIT1.

A heterologous gene effector can be or can comprise a sequence from a transcriptional regulator (TR). TR gene effectors include transcriptional regulatory domains from various families of transcription factors (e.g. KRAB, p65, MED, GTFs, etc.).

A heterologous gene effector can comprise a transcriptional activator domain. A heterologous gene effector can comprise can comprise two or more tandem transcriptional activation domains, e.g., located at a C-terminus, an N-terminus, or within a polypeptide sequence.

Non-limiting examples of transcriptional activation domains include GAL4, herpes simplex activation domain VP16, VP64 (a Tetramer of the herpes simplex activation domain VP16), NF-KB p65 subunit, Epstein-Barr virus R transactivator (Rta). In some embodiments, such transcriptional activation domains are used as controls in methods of the disclosure. In some embodiments, such transcriptional activation domains are used as one heterologous gene effector in a complex that comprises at least one additional heterologous gene effector (e.g., a different effector).

A heterologous gene effector can comprise a transcriptional repressor domain. A heterologous gene effector can comprise two or more transcriptional repressor domains, e.g., located at a C-terminus, an N-terminus, or within a polypeptide sequence, in tandem or separate.

Non-limiting examples of transcriptional repressor domains include the KRAB (Kruppel-associated box) domain of Koxl, the Mad mSIN3 interaction domain (SID), and ERF repressor domain (ERD). In some embodiments, such transcriptional repressor domains are used as controls in methods of the disclosure. In some embodiments, such transcriptional repressor domains are used as one heterologous gene effector in a complex that comprises at least one additional heterologous gene effector (e.g., a different effector).

In some embodiments, a heterologous gene effector is from a gene product that is a transcription factor.

In some embodiments, a heterologous gene effector is from a gene product that is a hematopoietic stem cell transcription factor. Non-limiting examples of hematopoietic stem cell transcription factors include AHR, Aiolos/IKZF3, CDX4, CREB, DNMT3A, DNMT3B, EGR1, FoxO3, GATA-1, GATA-2, GATA-3, Helios, HES-1, HHEX, HIF-1 alpha/HIF1A, HMGB1/HMG-1, HMGB3, Ikaros, c-Jun, LMO2, LMO4, c-Maf, MafB, MEF2C, MYB, c-Myc, NFATC2, NFIL3/E4BP4, Nrf2, p53, PITX2, PRDM16/MEL1, Prox1, PU.1/Spi-1, RUNX1/CBFA2, SALL4, SCL/Tal1, Smad2, Smad2/3, Smad4, Smad7, Spi-B, STAT Activators, STAT Inhibitors, STAT3, STAT4, STAT5a, STAT6, and TSC22.

In some embodiments, a heterologous gene effector is from a gene product that is a mesenchymal stem cell transcription factor. Non-limiting examples of mesenchymal stem cell transcription factors include DUX4, DUX4/DUX4c, DUX4c, EBF-1, EBF-2, EBF-3, ETV5, FoxC2, FoxF1, GATA-4, GATA-6, HMGA2, c-Jun, MYF-5, Myocardin, MyoD, Myogenin, NFATC2, p53, Pax3, PDX-1/IPF1, PLZF, PRDM16/MEL1, RUNX2/CBFA1, Smad1, Smad3, Smad4, Smad5, Smad8, Smad9, Snail, SOX2, SOX9, SOX11, STAT Activators, STAT Inhibitors, STAT1, STAT3, TBX18, Twist-1, and Twist-2.

In some embodiments, a heterologous gene effector is from a gene product that is an embryonic stem cell transcription factor. Non-limiting examples of embryonic stem cell transcription factors include Brachyury, EOMES, FoxC2, FoxD3, FoxF1, FoxH1, FoxO1/FKHR, GATA-2, GATA-3, GBX2, Goosecoid, HES-1, HNF-3 alpha/FoxA1, c-Jun, KLF2, KLF4, KLF5, c-Maf, Max, MEF2C, MIXL1, MTF2, c-Myc, Nanog, NFkB/IkB Activators, NFkB/IkB Inhibitors, NFkB1, NFkB2, Oct-3/4, Otx2, p53, Pax2, Pax6, PRDM14, Rex-1/ZFP42, SALL1, SALL4, Smad1, Smad2, Smad2/3, Smad3, Smad4, Smad5, Smad8, Snail, SOX2, SOX7, SOX15, SOX17, STAT Activators, STAT Inhibitors, STAT3, SUZ12, TBX6, TCF-3/E2A, THAP11, UTF1, WDR5, WT1, ZNF206, and ZNF281.

In some embodiments, a heterologous gene effector is from a gene product that is an induced pluripotent stem cell (iPSC) transcription factor. Non-limiting examples of iPSC transcription factors include KLF2, KLF4, c-Maf, c-Myc, Nanog, Oct-3/4, p53, SOX1, SOX2, SOX3, SOX15, SOX18, and TBX18.

In some embodiments, a heterologous gene effector is from a gene product that is an epithelial stem cell transcription factor. Non-limiting examples of epithelial stem cell transcription factors include ASCL2/Mash2, CDX2, DNMT1, ELF3, Ets-1, FoxM1, FoxN1, GATA-6, Hairless, HNF-4 alpha/NR2A1, IRF6, c-Maf, MITF, Miz-1/ZBTB17, MSX1, MSX2, MYB, c-Myc, Neurogenin-3, NFATC1, NKX3.1, Nrf2, p53, p63/TP73L, Pax2, Pax3, RUNX1/CBFA2, RUNX2/CBFA1, RUNX3/CBFA3, Smad1, Smad2, Smad2/3, Smad4, Smad5, Smad7, Smad8, Snail, SOX2, SOX9, STAT Activators, STAT Inhibitors, STAT3, SUZ12, TCF-3/E2A, and TCF7/TCF1.

In some embodiments, a heterologous gene effector is from a gene product that is a cancer stem cell transcription factor. Non-limiting examples of cancer stem cell transcription factors include Androgen R/NR3C4, AP-2 gamma, beta-Catenin, beta-Catenin Inhibitors, Brachyury, CREB, ER alpha/NR3A1, ER beta/NR3A2, FoxM1, FoxO3, FRA-1, GLI-1, GLI-2, GLI-3, HIF-1 alpha/HIF1A, HIF-2 alpha/EPAS1, HMGA1B, c-Jun, JunB, KLF4, c-Maf, MCM2, MCM7, MITF, c-Myc, Nanog, NFkB/IkB Activators, NFkB/IkB Inhibitors, NFkB1, NKX3.1, Oct-3/4, p53, PRDM14, Snail, SOX2, SOX9, STAT Activators, STAT Inhibitors, STAT3, TAZ/WWTR1, TBX3, Twist-1, Twist-2, WT1, and ZEB1.

In some embodiments, a heterologous gene effector is from a gene product that is a cancer-related transcription factor. Non-limiting examples of cancer-related transcription factors include ASCL1/Mash1, ASCL2/Mash2, ATF1, ATF2, ATF4, BLIMP1/PRDM1, CDX2, CDX4, DLX5, DNMT1, E2F-1, EGR1, ELF3, Ets-1, FosB/G0S3, FoxC1, FoxC2, FoxF1, GADD153, GATA-2, HMGA2, HMGB1/HMG-1, HNF-3 alpha/FoxA1, HNF-6/ONECUT1, HSF1, ID1, ID2, JunD, KLF10, KLF12, KLF17, LMO2, MEF2C, MYCL1/L-Myc, NFkB2, Oct-1, p63/TP73L, Pax3, PITX2, Prox1, RAP80, Rex-1/ZFP42, RUNX1/CBFA2, RUNX3/CBFA3, SALL4, SCL/Tal1, Sirtuin 2/SIRT2, Smad3, Smad4, Smad5, SOX11, STAT5a/b, STAT5a, STAT5b, TCF7/TCF1, TORC1, TORC2, TRIM32, TRPS1, and TSC22.

In some embodiments, a heterologous gene effector is from a gene product that is an immune cell transcription factor. Non-limiting examples of immune cell transcription factors include AP-1, Bcl6, E2A, EBF, Eomes, FoxP3, GATA3, Id2, Ikaros, IRF, IRF1, IRF2, IRF3, IRF3, IRF7, NFAT, NFkB, Pax5, PLZF, PU.1, ROR-gamma-T, STAT, STAT1, STAT2, STAT3, STAT4, STATS, STAT5A, STAT5B, STAT6, T-bet, TCF7, and ThPOK.

In some embodiments, a heterologous gene effector is from a gene product that is a RNA polymerase related protein. In some embodiments, a heterologous gene effector is from a transcription factor with a basic domain. In some embodiments, a heterologous gene effector is from a transcription factor with a zinc-coordinated DNA binding domain. In some embodiments, a heterologous gene effector is from a transcription factor with a helix-turn-helix domain. In some embodiments, a heterologous gene effector is from a transcription factor with an alpha helical DNA binding domain. In some embodiments, a heterologous gene effector is from a transcription factor with an alpha helix exposed by beta structures. In some embodiments, a heterologous gene effector is from a transcription factor with an immunoglobulin fold. In some embodiments, a heterologous gene effector is from a transcription factor with a with a beta-Hairpin exposed by an alpha/beta-scaffold. In some embodiments, a heterologous gene effector is from a transcription factor with a beta sheet binding to DNA. In some embodiments, a heterologous gene effector is from a transcription factor with a beta barrel DNA binding domain.

In some embodiments, a heterologous gene effector is from a gene product that is a nuclear receptor, for example, a nuclear hormone receptor. Non-limiting examples of nuclear hormone receptors include those encoded by NROB1, NROB2, NR1A1, NR1A2, NR1B1, NR1B2, NR1B3, NR1C1, NR1C2, NR1C3, NR1D1, NR1D2, NR1F1, NR1F2, NR1F3, NR1H4, NR1H5, NR1H3, NR1H2, NR1I1, NR1I2, NR1I3, NR2A1, NR2A2, NR2B1, NR2B2, NR2B3, NR2C1, NR2C2, NR2E1, NR2E3, NR2F1, NR2F2, NR2F6, NR3A1, NR3A2, NR3B1, NR3B2, NR3B3, NR3C4, NR3C1, NR3C2, NR3C3, NR4A1, NR4A2, NR4A3, NR5A1, NR5A2, and NR6A1.

In some embodiments, a heterologous gene effector is from a gene product that is involved in nucleosome assembly. In some embodiments, a heterologous gene effector is from a gene product that is involved in DNA metabolism. In some embodiments, a heterologous gene effector is from a gene product that is involved in nucleotide metabolism. In some embodiments, a heterologous gene effector is from a gene product that is involved in ribosome biogenesis. In some embodiments, a heterologous gene effector is from a gene product that is involved in protein folding. In some embodiments, a heterologous gene effector is from a gene product that is involved in translation. In some embodiments, a heterologous gene effector is from a gene product that is involved in signaling. In some embodiments, a heterologous gene effector is from a gene product that is involved in proteolysis. In some embodiments, a heterologous gene effector is from a gene product that is involved in negative regulation of endopeptidase activity.

In some embodiments, a heterologous gene effector or gene regulator, as used interchangeably herein, can comprise a polypeptide sequence that exhibits at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or substantially about 100% sequence identity to any of the heterologous gene effector amino acid sequences provided in **Table 3.**

**Table 3. Heterologous gene effector amino acid sequences**

| **SEQ** ID **NO:** | **Heterologous gene effector amino acid sequences** |
|---|---|
| SEQ ID NO: 15 | NNSQGRVTFEDVTVNFTQGEWQRLNPEQRNLYRDVMLENYSNLVSVGQGETTKPDV ILRLEQGKEPWLEEEEVLGSGRAEKNGDI |
| SEQ ID NO: 16 | SGHPGSWEMNSVAFEDVAVNFTQEEWALLDPSQKNLYRDVMQETFRNLASIGNKGE DQSIEDQYKNSSRNLRHIISHSGNNPYGC |
| SEQ ID NO: 17 | AAATLRTPTQGTVTFEDVAVHFSWEEWGLLDEAQRCLYRDVMLENLALLTSLDVHHQ KQHLGEKHFRSNVGRALFVKTCTFHVSG |
| SEQ ID NO: 18 | TTFKEAMTFKDVAVVFTEEELGLLDLAQRKLYRDVMLENFRNLLSVGHQAFHRDTFHF LREEKIWMMKTAIQREGNSGDKIQTEM |
| SEQ ID NO: 19 | VPAETSSSGLLEEQKMMKSQGLVSFKDVAVDFTQEEWQQLDPSQRTLYRDVMLENYS HLVSMGYPVSKPDVISKLEQGEEPWIIK |
| SEQ ID NO: 20 | MKSQGLVSFKDVAVDFTQEEWQQLDPSQRTLYRDVMLENYSHLVSMGYPVSKPDVIS KLEQGEEPWIIKGDISNWIYPDEYQADG |
| SEQ ID NO: 21 | AEGSVMFSDVSIDFSQEEWDCLDPVQRDLYRDVMLENYGNLVSMGLYTPKPQVISLLE QGKEPWMVGRELTRGLCSDLESMCETK |
| SEQ ID NO: 22 | AAALRDPAQVPVAADLLTDHEEGYVTFEDVAVYFSQEEWRLLDDAQRLLYRNVMLEN FTLLASLGKVLTPHPSILSWARLFLLFL |
| SEQ ID NO: 23 | AAAALRDPAQVPVAADLLTDHEEQGYVTFEDVAVYFSQEEWRLLDDAQRLLYRNVML ENFTLLASLGLASSKTHEITQLESWEEP |
| SEQ ID NO: 24 | AAAALRDPAQVPVAADLLTDHEEQGYVTFEDVAVYFSQEEWRLLDDAQRLLYRNVML ENFTLLASLGCWHGAEAEEAPEQIASVG |
| SEQ ID NO: 25 | AAAALRDPAQQGYVTFEDVAVYFSQEEWRLLDDAQRLLYRNVMLENFTLLASLGCWH GAEAEEAPEQIASVGLLSSNIQQHQKQH |
| SEQ ID NO: 26 | AAAALRDPAQVPVAADLLTDHEEGYVTFEDVAVYFSQEEWRLLDDAQRLLYRNVMLE NFTLLASLGKVLTPHPSILSWARLFLLF |
| SEQ ID NO: 27 | YVTFEDVAVYFSQEEWRLLDDAQRLLYRNVMLENFTLLASLGLASSKTHEITQLESWEE PFMPAWEVVTSAIPRGSWWVELREV |
| SEQ ID NO: 28 | AAAALRDPAQGYVTFEDVAVYFSQEEWRLLDDAQRLLYRNVMLENFTLLASLGLASSK THEITQLESWEEPFMPAWEVVTSAIPR |
| SEQ ID NO: 29 | AAAALRDPAQGYVTFEDVAVYFSQEEWRLLDDAQRLLYRNVMLENFTLLASLGCWHG AEAEEAPEQIASVGLLSSNIQQHQKQHC |
| SEQ ID NO: 30 | AAAALRDPAQQGYVTFEDVAVYFSQEEWRLLDDAQRLLYRNVMLENFTLLASLGLASS KTHEITQLESWEEPFMPAWEVVTSAIP |
| SEQ ID NO: 31 | AAAALRDPAQVPVAADLLTDHEEGYVTFEDVAVYFSQEEWRLLDDAQRLLYRNVMLE NFTLLASLGLASSKTHEITQLESWEEPF |
| SEQ ID NO: 32 | VTCAHLGRRARLPAAQPSACPGTCFSQEERMAAGYLPRWSQELVTFEDVSMDFSQEE WELLEPAQKNLYREVMLENYRNVVSLEA |
| SEQ ID NO: 33 | LVTFEDVSMDFSQEEWELLEPAQKNLYREVMLENYRNVVSLEALKNQCTDVGIKEGPL SPAQTSQVTSLSSWTGYLLFQPVASSH |
| SEQ ID NO: 34 | KNATIVMSVRREQGSSSGEGSLSFEDVAVGFTREEWQFLDQSQKVLYKEVMLENYINL VSIGYRGTKPDSLFKLEQGEPPGIAEG |
| SEQ ID NO: 35 | SSGEGSLSFEDVAVGFTREEWQFLDQSQKVLYKEVMLENYINLVSIGYRGTKPDSLFKLE QGEPPGIAEGAAHSQICPDADFLE |
| SEQ ID NO: 36 | GPLQFRDVAIEFSLEEWHCLDTAQRNLYRNVMLENYSNLVFLGITVSKPDLITCLEQGRK PLTMKRNEMIAKPSVSFLQVHSESQ |
| SEQ ID NO: 37 | GPLQFRDVAIEFSLEEWHCLDTAQRNLYRNVMLENYSNLVFLGITVSKPDLITCLEQGRK PLTMKRNEMIAKPSVMCSHFAQDLW |
| SEQ ID NO: 38 | APPSAPLPAQGPGKARPSRKGRRPRALKFVDVAVYFSPEEWGCLRPAQRALYRDVM RETYGHLGALGCAGPKPALISWLERNTD |
| SEQ ID NO: 39 | QTNTKDWTVTPEHVLPESQSLLTFEEVAMYFSQEEWELLDPTQKALYNDVMQENYET VISLALFVLPKPKVISCLEQGEEPWVQV |
| SEQ ID NO: 40 | AAATLRDPAQQGYVTFEDVAVYFSQEEWRLLDDAQRLLYRNVMLENFTLLASLGLASS KTHEITQLESWEEPFMPAWEVVTSAIL |
| SEQ ID NO: 41 | AAATLRDPAQGYVTFEDVAVYFSQEEWRLLDDAQRLLYRNVMLENFTLLASLGLASSK THEITQLESWEEPFMPAWEVVTSAILR |
| SEQ ID NO: 42 | DSVAFEDVAVNFTQEEWALLDPSQKNLYREVMQETLRNLTSIGKKWNNQYIEDEHQN PRRNLRRLIGERLSESKESHQHGEVLTQ |

The heterologous polynucleotide as disclosed herein can comprise one or more guide moieties (e.g., one or more guide nucleic acid molecules) to direct a heterologous gene effector to a target gene (e.g., target endogenous gene) or a target gene regulatory sequence. A guide moiety can confer an ability to recognize and specifically bind to the target gene or the target gene regulatory sequence. The guide moiety can be configured to form a complex with the heterologous polypeptide (e.g., a guide nucleic acid forming a complex with a nuclease, such as a CRISPR/Cas protein), and the complex can be configured to exhibit specific binding to the target polypeptide sequence as disclosed herein, to modify the expression level and/or the epigenetic modification level of the target gene.

A guide moiety can comprise a guide nucleic acid. A guide moiety can comprise a nuclease and a guide nucleic acid as disclosed herein. A guide moiety can comprise a nuclease or a part thereof, for example, an endonuclease, such as a heterologous endonuclease. The nuclease can be, e.g., a DNA nuclease and/or RNA nuclease, a modified nuclease that is nuclease-deficient or has reduced nuclease activity compared to a wild-type nuclease, a derivative thereof, a variant thereof, or a fragment thereof. In some embodiments, the guide moiety has minimal nuclease activity.

Any suitable nuclease, fragment or derivative thereof can be used in a guide moiety. Suitable nucleases include, but are not limited to, CRISPR-associated (Cas) proteins or Cas nucleases including type I CRISPR-associated (Cas) polypeptides, type II CRISPR-associated (Cas) polypeptides, type III CRISPR-associated (Cas) polypeptides, type IV CRISPR-associated (Cas) polypeptides, type V CRISPR-associated (Cas) polypeptides, and type VI CRISPR-associated (Cas) polypeptides; zinc finger nucleases (ZFN); transcription activator-like effector nucleases (TALEN); meganucleases; RNA-binding proteins (RBP); CRISPR-associated RNA binding proteins; recombinases; flippases; transposases; Argonaute (Ago) proteins (e.g., prokaryotic Argonaute (pAgo), archaeal Argonaute (aAgo), and eukaryotic Argonaute (eAgo)); any derivative thereof; any variant thereof and any fragment thereof.

In some embodiments, the guide moiety comprises a DNA nuclease such as an engineered (e.g., programmable or targetable) DNA nuclease that is nuclease-deficient. In some embodiments, the guide moiety comprises a nuclease-null DNA binding protein derived from a DNA nuclease that does not induce transcriptional activation or repression of a target DNA sequence unless it is present in a complex with one or more heterologous gene effectors of the disclosure. In some embodiments, the guide moiety comprises a nuclease-null DNA binding protein derived from a DNA nuclease that can induce transcriptional activation or repression of a target DNA sequence (e.g., which can be altered or augmented by the presence of a heterologous gene effector of the disclosure).

In some embodiments, the guide moiety comprises an RNA nuclease such as an engineered (e.g., programmable or targetable) RNA nuclease. In some embodiments, the guide moiety comprises a nuclease-null RNA binding protein derived from an RNA nuclease that does not induce transcriptional activation or repression of a target RNA sequence unless it is present in a complex with one or more heterologous gene effectors of the disclosure. In some embodiments, the guide moiety comprises a nuclease-null RNA binding protein derived from a RNA nuclease that can induce transcriptional activation or repression of a target RNA sequence (e.g., which can be altered or augmented by the presence of a heterologous gene effector of the disclosure).

In some embodiments, the guide moiety comprises a nucleic acid-guided targeting system. In some embodiments, the guide moiety comprises a DNA-guided targeting system. In some embodiments, the guide moiety comprises an RNA-guided targeting system. A guide moiety can comprise and utilize, for example, a guide nucleic acid sequence that facilitates specific binding of a CRISPR-Cas system (e.g., a nuclease deficient form thereof, such as dCas9) to a target gene (e.g., target endogenous gene) or target gene regulatory sequence. Binding specificity can be determined by use of a guide nucleic acid, such as a single guide RNA (sgRNA) or a part thereof. In some embodiments, the use of different sgRNAs allows the compositions and methods of the disclosure to be used with (e.g., targeted to) different target genes (e.g., target endogenous genes) or target gene regulatory sequences.

Prokaryotic CRISPR-Cas (Clustered regularly interspaced short palindromic repeats-CRISPR associated) systems, for example, Class II CRISPR-Cas systems such as Cas9 and Cpfl, can be repurposed as a tool for regulation of gene expression, epigenome editing, and chromatin looping in compositions and methods of the disclosure. Nuclease-deactivated Cas (dCas) proteins complexed with heterologous gene effectors can allow for regulation of expression of target genes (e.g., target endogenous genes) adjacent to a site bound by the dCas.

In some embodiments, the guide moiety comprises a CRISPR-associated (Cas) protein or a Cas nuclease that functions in a non-naturally occurring CRISPR (Clustered Regularly Interspaced Short Palindromic Repeats)/Cas (CRISPR-associated) system. In bacteria, this system can provide adaptive immunity against foreign DNA.

In a wide variety of organisms including diverse mammals, animals, plants, microbes, and yeast, a CRISPR/Cas system (e.g., modified and/or unmodified) can be utilized as a genome engineering tool, or can be modified to direct specific binding of engineered proteins to target loci as disclosed herein. A CRISPR/Cas system can comprise a guide nucleic acid such as a guide RNA (gRNA) complexed with a Cas protein for targeted regulation of gene expression and/or activity or nucleic acid binding. An RNA-guided Cas protein (e.g., a Cas nuclease such as a Cas9 nuclease) can specifically bind a target polynucleotide (e.g., DNA) in a sequence-dependent manner. The Cas protein, if possessing nuclease activity, can cleave the DNA.

In some cases, the Cas protein is mutated and/or modified to yield a nuclease deficient protein or a protein with decreased nuclease activity relative to a wild-type Cas protein. A nuclease deficient protein can retain the ability to bind DNA, but may lack or have reduced nucleic acid cleavage activity.

In some embodiments, the guide moiety comprises a Cas protein that forms a complex with a guide nucleic acid, such as a guide RNA or a part thereof. In some embodiments, the guide moiety comprises a Cas protein that forms a complex with a single guide nucleic acid, such as a single guide RNA (sgRNA). In some embodiments, the guide moiety comprises a RNA-binding protein (RBP) optionally complexed with a guide nucleic acid, such as a guide RNA (e.g., sgRNA), which is able to form a complex with a Cas protein. In some embodiments, the guide moiety comprises a nuclease-null DNA binding protein derived from a DNA nuclease that can induce transcriptional activation or repression of a target DNA sequence. In some embodiments, the guide moiety comprises a nuclease-null RNA binding protein derived from a RNA.

In some embodiments, a guide nucleic acid used in compositions and methods of the disclosure can be, for example, at least 1, at least 2, at least 3, at least 4, at least 5, at least 6, at least 7, at least 8, at least 9, at least 10, or more nucleotide(s).

In some embodiments, a guide nucleic acid used in compositions and methods of the disclosure is at most at most 10, at most 9, at most 8, at most 7, at most 6, at most 5, at most 4, at most 3, at most 2, or at most 1 nucleotide(s).

A guide nucleic acid can be a guide RNA or a part thereof.

Any suitable CRISPR/Cas system can be used. A CRISPR/Cas system can be referred to using a variety of naming systems. A CRISPR/Cas system can be a type I, a type II, a type III, a type IV, a type V, a type VI system, or any other suitable CRISPR/Cas system. A CRISPR/Cas system as used herein can be a Class 1, Class 2, or any other suitably classified CRISPR/Cas system. Class 1 or Class 2 determination can be based upon the genes encoding the effector module. Class 1 systems generally have a multi-subunit crRNA-effector complex, whereas Class 2 systems generally have a single protein, such as Cas9, Cpfl, C2c1, C2c2, C2c3 or a crRNA-effector complex. A Class 1 CRISPR/Cas system can use a complex of multiple Cas proteins to effect regulation. A Class 1 CRISPR/Cas system can comprise, for example, type I (e.g., I, IA, IB, IC, ID, IE, IF, IU), type III (e.g., III, IIIA, IIIB, IIIC, IIID), and type IV (e.g., IV, IVA, IVB) CRISPR/Cas type. A Class 2 CRISPR/Cas system can use a single large Cas protein to effect regulation. A Class 2 CRISPR/Cas systems can comprise, for example, type II (e.g., II, IIA, IIB) and type V CRISPR/Cas type. CRISPR systems can be complementary to each other, and/or can lend functional units in trans to facilitate CRISPR locus targeting.

When a guide moiety comprises a Cas protein or derivative thereof, the Cas protein or derivative thereof can be a Class 1 or a Class 2 Cas protein. A Cas protein can be a type I, type II, type III, type IV, type V Cas protein, or type VI Cas protein. A Cas protein can comprise one or more domains. Non-limiting examples of domains include, guide nucleic acid recognition and/or binding domain, nuclease domains (e.g., DNase or RNase domains, RuvC, HNH), DNA binding domain, RNA binding domain, helicase domains, protein-protein interaction domains, and dimerization domains. A guide nucleic acid recognition and/or binding domain can interact with a guide nucleic acid. A nuclease domain can comprise catalytic activity for nucleic acid cleavage. A nuclease domain can lack catalytic activity to prevent nucleic acid cleavage. A Cas protein can be a chimeric Cas protein or fragment thereof that is fused to other proteins or polypeptides. A Cas protein can be a chimera of various Cas proteins, for example, comprising domains from different Cas proteins.

Non-limiting examples of Cas proteins include c2c1, C2c2, c2c3, Casl, Cas1B, Cas2, Cas3, Cas4, Cas5, Cas5e (CasD), Cash, Cas6e, Cas6f, Cas7, Cas8a, Cas8a1, Cas8a2, Cas8b, Cas8c, Cas9 (Csnl or Csx12), Cas10, CaslOd, Cas10, CaslOd, CasF, CasG, CasH, Cpfl, Csyl, Csy2, Csy3, Csel (CasA), Cse2 (CasB), Cse3 (CasE), Cse4 (CasC), Cscl, Csc2, Csa5, Csn2, Csm2, Csm3, Csm4, Csm5, Csm6, Cmrl, Cmr3, Cmr4, Cmr5, Cmr6, Csbl, Csb2, Csb3, Csx17, Csx14, Csx10, Csx16, CsaX, Csx3, Csxl, Csx15, Csfl, Csf2, Csf3, Csf4, Cul966, Cas13a, Cas13b, Cas13c, Cas13d, Cas13X, Cas13Y, and homologs or modified versions thereof.

In some cases, the Cas protein as disclosed herein may not and need not be Cas9 or Cas12a. The Cas protein as disclosed herein can have a smaller size as compared to Cas9 or Cas12a. The Cas protein as disclosed herein can be derived from Un1Cas12f1 (or Cas14a1). For example, the Cas protein as disclosed herein can comprise an amino acid sequence that is at least about 50%, at least about 60%, at least about 70%, at least about 75% at least about 80%, at least about 85%, at least about 90%, at least about 95%, at least about 96%, at least about 97%, at least about 98%, at least about 99%, or substantially about 100% identical to the polypeptide sequence of SEQ ID NO. 43 In another example, the Cas protein as disclosed herein can comprise an amino acid sequence that is at least about 50%, at least about 60%, at least about 70%, at least about 75% at least about 80%, at least about 85%, at least about 90%, at least about 95%, at least about 96%, at least about 97%, at least about 98%, at least about 99%, or substantially about 100% identical to the polypeptide sequence of SEQ ID NO. 44 As disclosed herein, SEQ ID NO: 43 encodes the polypeptide sequence of Un1Cas12f1 (or Cas14a1). As disclosed herein, SEQ ID NO: 44 encodes an engineered variant of Un1Cas12f1 with reduced nuclease activity.
SEQ ID NO: 43 (Un1Cas12f1) 
1 MAKNTITKTL KLRIVRPYNS AEVEKIVADE KNNREKIALE KNKDKVKEAC
51 SKHLKVAAYC TTQVERNACL FCKARKLDDK FYQKLRGQFP DAVFWQEISE
101 IFRQLQKQAA EIYNQSLIEL YYEIFIKGKG IANASSVEHY LSDVCYTRAA
151 ELFKNAAIAS GLRSKIKSNF RLKELKNMKS GLPTTKSDNF PIPLVKQKGG
201 QYTGFEISNH NSDFIIKIPF GRWQVKKEID KYRPWEKFDF EQVQKSPKPI
251 SLLLSTQRRK RNKGWSKDEG TEAEIKKVMN GDYQTSYIEV KRGSKIGEKS
301 AWMLNLSIDV PKIDKGVDPS IIGGIDVGVK SPLVCAINNA FSRYSISDND
351 LFHFNKKMFA RRRILLKKNR HKRAGHGAKN KLKPITILTE KSERFRKKLI
401 ERWACEIADF FIKNKVGTVQ MENLESMKRK EDSYFNIRLR GFWPYAEMQN
451 KIEFKLKQYG IEIRKVAPNN TSKTCSKCGH LNNYFNFEYR KKNKFPHFKC
501 EKCNFKENAD YNAALNISNP KLKSTKEEP
SEQ ID NO: 44 (deactivated nuclease variant of Un1Cas12f1)

1 MAKNTITKTL KLRIVRPYNS AEVEKIVADE KNNREKIALE KNKDKVKEAC
51 SKHLKVAAYC TTQVERNACL FCKARKLDDK FYQKLRGQFP DAVFWQEISE
101 IFRQLQKQAA EIYNQSLIEL YYEIFIKGKG IANASSVEHY LSRVCYRRAA
151 ELFKNAAIAS GLRSKIKSNF RLKELKNMKS GLPTTKSDNF PIPLVKQKGG

201 QYTGFEISNH NSDFIIKIPF GRWQVKKEID KYRPWEKFDF EQVQKSPKPI
251 SLLLSTQRRK RNKGWSKDEG TEAEIKKVMN GDYQTSYIEV KRGSKICEKS
301 AWMLNLSIDV PKIDKGVDPS IIGGIAVGVR SPLVCAINNA FSRYSISDND
351 LFHFNKKMFA RRRILLKKNR HKRAGHGAKN KLKPITILTE KSERFRKKLI
401 ERWACEIADF FIKNKVGTVQ MENLESMKRK EDSYFNIRLR GFWPYAEMQN
451 KIEFKLKQYG IEIRKVAPNN TSKTCSKCGH LNNYFNFEYR KKNKFPHFKC
501 EKCNFKENAA YNAALNISNP KLKSTKERP

A Cas protein or fragment or derivative thereof can be from any suitable organism. Non-limiting examples include Streptococcus pyogenes, *Streptococcus thermophilus, Streptococcus sp., Staphylococcus aureus, Nocardiopsis dassonvillei, Streptomyces pristinae spiralis, Streptomyces viridochromo genes, Streptomyces viridochromogenes, Streptosporangium roseum, Streptosporangium roseum, AlicyclobacHlus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas nap hthalenivorans, Polaromonas sp., Crocosphaera watsonii, Cyanothece sp., Microcystis aeruginosa, Pseudomonas aeruginosa, Synechococcus sp., Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, Acaryochloris marina, Leptotrichia shahii,* and *Francisella novicida.* In some aspects, the organism is *Streptococcus pyogenes* (*S. pyogenes*)*.* In some aspects, the organism is *Staphylococcus aureus* (*S. aureus*)*.* In some aspects, the organism is *Streptococcus thermophilus* (*S. thermophilus*)*.*

A Cas protein can be derived from a variety of bacterial species including, but not limited to, *Veillonella atypical, Fusobacterium nucleatum, Filifactor alocis, Solobacterium moorei, Coprococcus catus, Treponema denticola, Peptoniphilus duerdenii, Catenibacterium mitsuokai, Streptococcus mutans, Listeria innocua, Staphylococcus pseudintermedius, Acidaminococcus intestine, Olsenella uli, Oenococcus kitaharae, Bifidobacterium bifidum, Lactobacillus rhamnosus, Lactobacillus gasseri, Finegoldia magna, Mycoplasma mobile, Mycoplasma gallisepticum, Mycoplasma ovipneumoniae, Mycoplasma canis, Mycoplasma synoviae, Eubacterium rectale, Streptococcus thermophilus, Eubacterium dolichum, Lactobacillus coryniformis subsp. Torquens, Ilyobacter polytropus, Ruminococcus albus, Akkermansia muciniphila, Acidothermus cellulolyticus, Bifidobacterium longum, Bifidobacterium dentium, Corynebacterium diphtheria, Elusimicrobium minutum, Nitratifractorsalsuginis, Sphaerochaeta globus, Fibrobacter succinogenes subsp. Succinogenes, Bacteroides fragilis, Capnocytophaga ochracea, Rhodopseudomonas palustris, Prevotella micans, Prevotella ruminicola, Flavobacterium columnare, Aminomonas paucivorans, Rhodospirillum rubrum, Candidatus Puniceispirillum marinum, Verminephrobacter eiseniae, Ralstonia syzygii, Dinoroseobacter shibae, Azospirillum, Nitrobacter hamburgensis, Bradyrhizobium, Wolinellasuccinogenes, Campylobacter jejuni subsp. Jejuni, Helicobacter mustelae, Bacillus cereus, Acidovorax ebreus, Clostridium perfringens, Parvibaculum lavamentivorans, Roseburia intestinalis, Neisseria meningitidis, Pasteurella multocida subsp. Multocida, Sutterella wadsworthensis, proteobacterium, Legionella pneumophila, Parasutterella excrementihominis, Wolinella succinogenes,* and *Francisella novicida.*

A Cas protein as used herein can be a wildtype or a modified form of a Cas protein. A Cas protein can be an active variant, inactive variant, or fragment of a wild type or modified Cas protein. A Cas protein can comprise an amino acid change such as a deletion, insertion, substitution, variant, mutation, fusion, chimera, or any combination thereof relative to a wild-type version of the Cas protein. A Cas protein can be a polypeptide with at least about 5%, at least about 10%, at least about 20%, at least about 30%, at least about 40%, at least about 50%, at least about 60%, at least about 70%, at least about 80%, at least about 90%, at least about 91%, at least about 92%, at least about 93%, at least about 94%, at least about 95%, at least about 96%, at least about 97%, at least about 98%, at least about 99%, or 100% sequence identity or sequence similarity to a wild type Cas protein. A Cas protein can be a polypeptide with at most about 5%, at most about 10%, at most about 20%, at most about 30%, at most about 40%, at most about 50%, at most about 60%, at most about 70%, at most about 80%, at most about 90%, or at most about 100% sequence identity and/or sequence similarity to a wild type exemplary Cas protein. Variants or fragments can comprise at least about 5%, at least about 10%, at least about 20%, at least about 30%, at least about 40%, at least about 50%, at least about 60%, at least about 70%, at least about 80%, at least about 90%, at least about 91%, at least about 92%, at least about 93%, at least about 94%, at least about 95%, at least about 96%, at least about 97%, at least about 98%, at least about 99%, or 100% sequence identity or sequence similarity to a wild type or modified Cas protein or a portion thereof. Variants or fragments can be targeted to a nucleic acid locus in complex with a guide nucleic acid while lacking nucleic acid cleavage activity.

A Cas protein can comprise one or more nuclease domains, such as DNase domains. For example, a Cas9 protein can comprise a RuvC-like nuclease domain and/or an HNH-like 20 nuclease domain. The in a nuclease active form of Cas9, RuvC and HNH domains can each cut a different strand of double-stranded DNA to make a double-stranded break in the DNA. A Cas protein can comprise only one nuclease domain (e.g., Cpfl comprises RuvC domain but lacks HNH domain). In some embodiments, nuclease domains are absent. In some embodiments, nuclease domains are present but inactive or have reduced or minimal activity. In some embodiments, nuclease domains are present and active.

One or a plurality of the nuclease domains (e.g., RuvC, HNH) of a Cas protein can be deleted or mutated so that they are no longer functional or comprise reduced nuclease activity. For example, in a Cas protein comprising at least two nuclease domains (e.g., Cas9), if one of the nuclease domains is deleted or mutated, the resulting Cas protein, known as a nickase, can generate a single-strand break at a CRISPR RNA (crRNA) recognition sequence within a double- stranded DNA but not a double-strand break. Such a nickase can cleave the complementary strand or the non-complementary strand, but may not cleave both. If all of the nuclease domains of a Cas protein (e.g., both RuvC and HNH nuclease domains in a Cas9 protein; RuvC nuclease domain in a Cpfl protein) are deleted or mutated, the resulting Cas protein can have a reduced or no ability to cleave both strands of a double-stranded DNA. An example of a mutation that can convert a Cas9 protein into a nickase is a D10A (aspartate to alanine at position 10 of Cas9) mutation in the RuvC domain of Cas9 from *S. pyogenes.* H939A (histidine to alanine at amino acid position 839) or H840A (histidine to alanine at amino acid position 840) in the HNH domain of Cas9 from *S. pyogenes* can convert the Cas9 into a nickase. An example of a mutation that can convert a Cas9 protein into a dead Cas9 is a D10A (aspartate to alanine at position 10 of Cas9) mutation in the RuvC domain and H939A (histidine to alanine at amino acid position 839) or H840A (histidine to alanine at amino acid position 840) in the HNH domain of Cas9 from *S. pyogenes.*

A nuclease dead Cas protein (e.g., one derived from any Cas protein, such as Un1Cas12f1) can comprise one or more mutations relative to a wild-type version of the protein. The mutation can result in no more than 90%, no more than 80%, no more than 70%, no more than 60%, no more than 50%, no more than 40%, no more than 30%, no more than 20%, no more than 10%, no more than 5%, or no more than 1% of the nucleic acid-cleaving activity in one or more of the plurality of nucleic acid-cleaving domains of the wild-type Cas protein. The mutation can result in one or more of the plurality of nucleic acid-cleaving domains retaining the ability to cleave the complementary strand of the target nucleic acid but reducing its ability to cleave the non-complementary strand of the target nucleic acid. The mutation can result in one or more of the plurality of nucleic acid-cleaving domains retaining the ability to cleave the non-complementary strand of the target nucleic acid but reducing its ability to cleave the complementary strand of the target nucleic acid. The mutation can result in one or more of the plurality of nucleic acid-cleaving domains lacking the ability to cleave the complementary strand and the non-complementary strand of the target nucleic acid. The residues to be mutated in a nuclease domain can correspond to one or more catalytic residues of the nuclease. For example, residues in the wild type exemplary *S. pyogenes* Cas9 polypeptide such as Asp10, His840, Asn854 and Asn856 can be mutated to inactivate one or more of the plurality of nucleic acid-cleaving domains (e.g., nuclease domains). The residues to be mutated in a nuclease domain of a Cas protein can correspond to residues Asp10, His840, Asn854 and Asn856 in the wild type *S. pyogenes* Cas9 polypeptide, for example, as determined by sequence and/or structural alignment.

As non-limiting examples, residues D10, G12, G17, E762, H840, N854, N863, H982, H983, A984, D986, and/or A987 (or the corresponding mutations of any of the Cas proteins) can be mutated. For example, e.g., D 10A, G12A, G17A, E762A, H840A, N854A, N863A, H982A, H983A, A984A, and/or D986A. Mutations other than alanine substitutions can be suitable.

A D10A mutation can be combined with one or more of H840A, N854A, or N856A mutations to produce a Cas9 protein substantially lacking DNA cleavage activity (e.g., a dead Cas9 protein). A H840A mutation can be combined with one or more of D10A, N854A, or N856A mutations to produce a site-directed polypeptide substantially lacking DNA cleavage activity. An N854A mutation can be combined with one or more of H840A, D1OA, or N856A mutations to produce a site-directed polypeptide substantially lacking DNA cleavage activity. A N856A mutation can be combined with one or more of H840A, N854A, or D10A mutations to produce a site-directed polypeptide substantially lacking DNA cleavage activity.

In some embodiments, a Cas protein is a Class 2 Cas protein. In some embodiments, a Cas protein is a type II Cas protein. In some embodiments, the Cas protein is a Cas9 protein, a modified version of a Cas9 protein, or derived from a Cas9 protein. For example, a Cas9 protein lacking cleavage activity. In some embodiments, the Cas9 protein is a Cas9 protein from *S. pyogenes* (e.g., SwissProt accession number Q99ZW2). In some embodiments, the Cas9 protein is a Cas9 from *S. aureus* (e.g., SwissProt accession number J7RUA5). In some embodiments, the Cas9 protein is a modified version of a Cas9 protein from *S. pyogenes* or *S. Aureus.* In some embodiments, the Cas9 protein is derived from a Cas9 protein from *S. pyogenes* or *S. Aureus.* For example, a *S. pyogenes* or *S. Aureus* Cas9 protein lacking cleavage activity.

In some embodiments, Cas9 can generally refer to a polypeptide with at least about 5%, at least about 10%, at least about 20%, at least about 30%, at least about 40%, at least about 50%, at least about 60%, at least about 70%, at least about 80%, at least about 90%, or about 100% sequence identity and/or sequence similarity to a wild type exemplary Cas9 polypeptide (e.g., Cas9 from *S. pyogenes*)*.* In some embodiments, Cas9 can refer to a polypeptide with at most about 5%, at most about 10%, at most about 20%, at most about 30%, at most about 40%, at most about 50%, at most about 60%, at most about 70%, at most about 80%, at most about 90%, or about 100% sequence identity and/or sequence similarity to a wild type Cas9 polypeptide (e.g., from *S. pyogenes*)*.* Cas9 can refer to the wildtype or a modified form of the Cas9 protein that can comprise an amino acid change such as a deletion, insertion, substitution, variant, mutation, fusion, chimera, or any combination thereof.

A Cas protein can comprise an amino acid sequence having at least about 5%, at least about 10%, at least about 20%, at least about 30%, at least about 40%, at least about 50%, at least about 60%, at least about 70%, at least about 80%, at least about 90%, at least about 91%, at least about 92%, at least about 93%, at least about 94%, at least about 95%, at least about 96%, at least about 97%, at least about 98%, at least about 99%, or 100% sequence identity or sequence similarity to a nuclease domain (e.g., RuvC domain, HNH domain) of a wild-type Cas protein.

A Cas protein, variant or derivative thereof can be modified to enhance regulation of gene expression by compositions and methods of the disclosure, e.g., as part of a complex disclosed herein. A Cas protein can be modified to increase or decrease nucleic acid binding affinity, nucleic acid binding specificity, enzymatic activity, and/or binding to other factors, such as heterodimerization or oligomerization domains and induce ligands. Cas proteins can also be modified to change any other activity or property of the protein, such as stability. For example, one or more nuclease domains of the Cas protein can be modified, deleted, or inactivated, or a Cas protein can be truncated to remove domains that are not essential for the desired function of the protein or complex. A Cas protein can be modified to modulate (e.g., enhance or reduce) the activity of the Cas protein for regulating gene expression by a complex of the disclosure that comprises a heterologous gene effector.

For example, a Cas protein can be coupled (e.g., fused, covalently coupled, or non-covalently coupled) to a heterologous gene effector (e.g., an epigenetic modification domain, a transcriptional activation domain, and/or a transcriptional repressor domain). A Cas protein can be coupled (e.g., fused, covalently coupled, or non-covalently coupled) to an oligomerization or dimerization domain as disclosed herein (e.g., a heterodimerization domain). A Cas protein can be coupled (e.g., fused, covalently coupled, or non-covalently coupled) to a heterologous polypeptide that provides increased or decreased stability. A Cas protein can be coupled (e.g., fused, covalently coupled, or non-covalently coupled) to a sequence that can facilitate degradation of the Cas protein or a complex containing the Cas protein, for example, a degron, such as an inducible degron (e.g., auxin inducible).

A Cas protein can be coupled (e.g., fused, covalently coupled, or non-covalently coupled) to any suitable number of partners, for example, at least one, at least two, at least three, at least four, or at least five, at least six, at least seven, or at least 8 partners. In some embodiments, a Cas protein of the disclosure is coupled (e.g., fused, covalently coupled, or non-covalently coupled) to at most two, at most three, at most four, at most five, at most six, at most seven, at most eight, or at most ten partners. In some embodiments, a Cas protein of the disclosure is coupled (e.g., fused, covalently coupled, or non-covalently coupled) to 1 - 5, 1 - 4, 1 - 3, 1 - 2, 2 - 5, 2 - 4, 2 - 3, 3 - 5, 3 - 4, or 4 - 5 partners. In some embodiments, a Cas protein of the disclosure is coupled (e.g., fused, covalently coupled, or non-covalently coupled) to one partner. In some embodiments, a Cas protein of the disclosure is coupled (e.g., fused, covalently coupled, or non-covalently coupled) to two partners. In some embodiments, a Cas protein of the disclosure is coupled (e.g., fused, covalently coupled, or non-covalently coupled) to three partners. In some embodiments, a Cas protein of the disclosure is coupled (e.g., fused, covalently coupled, or non-covalently coupled) to four partners. In some embodiments, a Cas protein of the disclosure is coupled (e.g., fused, covalently coupled, or non-covalently coupled) to five partners. In some embodiments, a Cas protein of the disclosure is coupled (e.g., fused, covalently coupled, or non-covalently coupled) to six partners.

A Cas protein can be a fusion protein. The fused domain or heterologous polypeptide can be located at the N-terminus, the C-terminus, or internally within the Cas protein.

A Cas protein can be provided in any form. For example, a Cas protein can be provided in the form of a protein, such as a Cas protein alone or complexed with a guide nucleic acid as a ribonucleoprotein. A Cas protein can be provided in a complex, for example, complexed with a guide nucleic acid and/or one or more heterologous gene effectors of the disclosure. A Cas protein can be provided in the form of a nucleic acid encoding the Cas protein, such as an RNA (e.g., messenger RNA (mRNA)), or DNA. The nucleic acid encoding the Cas protein can be codon optimized for efficient translation into protein in a particular cell or organism.

Nucleic acids encoding Cas proteins, fragments, or derivatives thereof can be stably integrated in the genome of a cell. Nucleic acids encoding Cas proteins can be operably linked to a promoter, for example, a promoter that is constitutively or inducibly active in the cell. Nucleic acids encoding Cas proteins can be operably linked to a promoter in an expression construct. Expression constructs can include any nucleic acid constructs capable of directing expression of a gene or other nucleic acid sequence of interest (e.g., a Cas gene) and which can transfer such a nucleic acid sequence of interest to a target cell.

In some embodiments, a Cas protein, variant or derivative thereof is a nuclease dead Cas (dCas) protein. A dead Cas protein can be a protein that lacks nucleic acid cleavage activity.

A Cas protein can comprise a modified form of a wild type Cas protein. The modified form of the wild type Cas protein can comprise an amino acid change (e.g., deletion, insertion, or substitution) that reduces the nucleic acid-cleaving activity of the Cas protein. For example, the modified form of the Cas protein can have no more than 90%, no more than 80%, no more than 70%, no more than 60%, no more than 50%, no more than 40%, no more than 30%, no more than 20%, no more than 10%, no more than 5%, or no more than 1% of the nucleic acid-cleaving activity of the wild-type Cas protein (e.g., Cas9 from *S. pyogenes*)*.* The modified form of Cas protein can have no substantial nucleic acid-cleaving activity. When a Cas protein is a modified form that has no substantial nucleic acid-cleaving activity, it can be referred to as enzymatically inactive, "deactivated" and/or "dead" (abbreviated by "d"). A dead Cas protein (e.g., dCas, dCas9) can bind to a target polynucleotide but may not cleave or minimally cleaves the target polynucleotide. In some aspects, a dead Cas protein is a dead Cas9 protein.

A dCas9 polypeptide can associate with a single guide RNA (sgRNA) to activate or repress transcription of a target gene (e.g., target endogenous gene), for example, in combination with heterologous gene effector(s) disclosed herein. sgRNAs can be introduced into cells expressing the Cas or guide moiety component of the disclosure. In some cases, such cells can contain one or more different sgRNAs that target the same target gene (e.g., target endogenous gene) or target gene regulatory sequence. In other cases, the sgRNAs target different nucleic acids in the cell (e.g., different target genes, different target gene regulatory sequences, or different sequences within the same target gene or target gene regulatory sequence).

Enzymatically inactive can refer to a nuclease that can bind to a nucleic acid sequence in a polynucleotide in a sequence-specific manner, but will not cleave a target polynucleotide or will cleave it at a substantially reduced frequency. An enzymatically inactive guide moiety can comprise an enzymatically inactive domain (e.g. nuclease domain). Enzymatically inactive can refer to no activity. Enzymatically inactive can refer to substantially no activity. Enzymatically inactive can refer to essentially no activity. Enzymatically inactive can refer to an activity no more than 1%, no more than 2%, no more than 3%, no more than 4%, no more than 5%, no more than 6%, no more than 7%, no more than 8%, no more than 9%, or no more than 10% activity compared to a comparable wild-type activity (e.g., nucleic acid cleaving activity, wild-type Cas9 activity).

In some embodiments, the guide moiety does not contain a nucleic acid-guided targeting system. For example, guide moieties can include proteins that bind to a target gene (e.g., target endogenous gene) or target gene regulatory sequence based on protein structural features, such as certain nucleases disclosed herein.

In some embodiments, a guide moiety comprises a zinc finger nuclease (ZFN) or a variant, fragment, or derivative thereof. ZFN can refer to a fusion between a cleavage domain, such as a cleavage domain of Fokl, and at least one zinc finger motif (e.g., at least 2, at least 3, at least 4, or at least 5 zinc finger motifs) which can bind polynucleotides such as DNA and RNA. In some embodiments, a ZFN is used in a targeting moiety of the disclosure to bind a polynucleotide (e.g., target gene or target gene regulatory sequence), but the ZFN does not cleave or substantially does not cleave the polynucleotide, e.g., a nuclease dead ZFN. A ZFN or a variant, fragment, or derivative thereof can be fused to or associated with one of more heterologous gene effectors to form a complex of the disclosure.

The heterodimerization at certain positions in a polynucleotide of two individual ZFNs in certain orientation and spacing can lead to cleavage of the polynucleotide in nuclease-active ZFN. For example, a ZFN binding to DNA can induce a double-strand break in the DNA. In order to allow two cleavage domains to dimerize and cleave DNA, two individual ZFNs can bind opposite strands of DNA with their C-termini at a certain distance apart. In some cases, linker sequences between the zinc finger domain and the cleavage domain can require the 5' edge of each binding site to be separated by about 5-7 base pairs. In some cases, a cleavage domain is fused to the C-terminus of each zinc finger domain.

In some embodiments, the cleavage domain of a guide moiety comprising a ZFN comprises a modified form of a wild type cleavage domain. The modified form of the cleavage domain can comprise an amino acid change (e.g., deletion, insertion, or substitution) that reduces the nucleic acid-cleaving activity of the cleavage domain. For example, the modified form of the cleavage domain can have no more than 90%, no more than 80%, no more than 70%, no more than 60%, no more than 50%, no more than 40%, no more than 30%, no more than 20%, no more than 10%, no more than 5%, or no more than 1% of the nucleic acid-cleaving activity of the corresponding wild-type cleavage domain. The modified form of the cleavage domain can have no substantial nucleic acid-cleaving activity. In some embodiments, the cleavage domain is enzymatically inactive.

In some embodiments, a guide moiety comprises a "TALEN" or "TAL-effector nuclease" or a variant, fragment, or derivative thereof. TALENs refer to engineered transcription activator-like effector nucleases that generally contain a central domain of DNA-binding tandem repeats and a cleavage domain. TALENs can be produced by fusing a TAL effector DNA binding domain to a DNA cleavage domain. In some cases, a DNA-binding tandem repeat comprises 33-35 amino acids in length and contains two hypervariable amino acid residues at positions 12 and 13 that can recognize at least one specific DNA base pair. A transcription activator-like effector (TALE) protein can be fused to a nuclease such as a wild-type or mutated Fok1 endonuclease or the catalytic domain of Fok1. In some embodiments, a TALEN is used in a targeting moiety of the disclosure to bind a polynucleotide (e.g., target gene or target gene regulatory sequence), but the TALEN does not cleave or substantially does not cleave the polynucleotide, e.g., a nuclease dead TALEN. A TALEN or a variant, fragment, or derivative thereof can be fused to or associated with one of more heterologous gene effectors to form a complex of the disclosure.

In some embodiments, a TALEN is engineered for reduced nuclease activity. In some embodiments, the nuclease domain of a TALEN comprises a modified form of a wild type nuclease domain. The modified form of the nuclease domain can comprise an amino acid change (e.g., deletion, insertion, or substitution) that reduces the nucleic acid-cleaving activity of the nuclease domain. For example, the modified form of the nuclease domain can have no more than 90%, no more than 80%, no more than 70%, no more than 60%, no more than 50%, no more than 40%, no more than 30%, no more than 20%, no more than 10%, no more than 5%, or no more than 1% of the nucleic acid-cleaving activity of the wild-type nuclease domain. The modified form of the nuclease domain can have no substantial nucleic acid-cleaving activity. In some embodiments, the nuclease domain is enzymatically inactive. A TALEN or a variant, fragment, or derivative thereof can be fused to or associated with one of more heterologous gene effectors to form a complex of the disclosure.

Several mutations to Fok1 have been made for its use in TALENs, which, for example, improve cleavage specificity or activity. Such TALENs can be engineered to bind any desired DNA sequence. TALENs can be used to generate gene modifications (e.g., nucleic acid sequence editing) by creating a double-strand break in a target DNA sequence, which in turn, undergoes NHEJ or HDR.

A TALE or a variant, fragment, or derivative thereof can be fused to or associated with one of more heterologous gene effectors to form a complex of the disclosure. In some embodiments, the transcription activator-like effector (TALE) protein is fused to a heterologous gene effector and does not comprise a nuclease. In some embodiments, a TALEN does not cleave or substantially does not cleave the polynucleotide, e.g., a nuclease dead TALE. A TALE or a variant, fragment, or derivative thereof can be fused to or associated with one of more heterologous gene effectors to form a complex of the disclosure.

In some embodiments, the complex of the transcription activator-like effector (TALE) protein and the heterologous gene effector is designed to function as a transcriptional activator. In some embodiments, the complex of the transcription activator-like effector (TALE) protein and the heterologous gene effector is designed to function as a transcriptional repressor. For example, the DNA-binding domain of the transcription activator-like effector (TALE) protein can be fused (e.g., linked) to one or more heterologous gene effectors that comprise transcriptional activation domains, or to one or more heterologous gene effectors that comprise transcriptional repression domains.

In some embodiments, a guide moiety comprises a meganuclease. Meganucleases generally refer to rare-cutting endonucleases or homing endonucleases that can be highly sequence specific. Meganucleases can recognize DNA target sites ranging from at least 12 base pairs in length, e.g., from 12 to 40 base pairs, 12 to 50 base pairs, or 12 to 60 base pairs in length. Meganucleases can be modular DNA-binding nucleases such as any fusion protein comprising at least one catalytic domain of an endonuclease and at least one DNA binding domain or protein specifying a nucleic acid target sequence. The DNA-binding domain can contain at least one motif that recognizes single- or double-stranded DNA. A nuclease-active meganuclease can generate a double-stranded break. In some embodiments, a meganuclease is used in a targeting moiety of the disclosure to bind a polynucleotide (e.g., target gene or target gene regulatory sequence), but the meganuclease does not cleave or substantially does not cleave the polynucleotide, e.g., a nuclease dead meganuclease. A meganuclease or a variant, fragment, or derivative thereof can be fused to or associated with one of more heterologous gene effectors to form a complex of the disclosure.

The meganuclease can be monomeric or dimeric. In some embodiments, the meganuclease is naturally-occurring (found in nature) or wild-type, and in other instances, the meganuclease is non-natural, artificial, engineered, synthetic, rationally designed, or man-made. In some embodiments, the meganuclease of the present disclosure includes an I-CreI meganuclease, I-CeuI meganuclease, I-Msol meganuclease, I-SceI meganuclease, variants thereof, derivatives thereof, and fragments thereof.

In some embodiments, the nuclease domain of a meganuclease comprises a modified form of a wild type nuclease domain. The modified form of the nuclease domain can comprise an amino acid change (e.g., deletion, insertion, or substitution) that reduces or eliminates the nucleic acid-cleaving activity of the nuclease domain. For example, the modified form of the nuclease domain can have no more than 90%, no more than 80%, no more than 70%, no more than 60%, no more than 50%, no more than 40%, no more than 30%, no more than 20%, no more than 10%, no more than 5%, or no more than 1% of the nucleic acid-cleaving activity of the wild-type nuclease domain. The modified form of the nuclease domain can have no substantial nucleic acid-cleaving activity. In some embodiments, the nuclease domain is enzymatically inactive. In some embodiments, a meganuclease can bind DNA but cannot cleave the DNA. In some embodiments, a nuclease-inactive meganuclease is fused to or associated with one or more heterologous gene effectors to generate a complex of the disclosure.

In some embodiments, the guide moiety can regulate expression and/or activity of a target gene (e.g., target endogenous gene). In some embodiments, the guide moiety can edit the sequence of a nucleic acid (e.g., a gene and/or gene product). A nuclease-active Cas protein can edit a nucleic acid sequence by generating a double-stranded break or single-stranded break in a target polynucleotide.

In some embodiments, a guide moiety comprising a nuclease can generate a double-strand break in a target polynucleotide, such as DNA. A double-strand break in DNA can result in DNA break repair which allows for the introduction of gene modification(s) (e.g., nucleic acid editing). In some embodiments, a nuclease induces site-specific single-strand DNA breaks or nicks, thus resulting in HDR.

A double-strand break in DNA can result in DNA break repair which allows for the introduction of gene modification(s) (e.g., nucleic acid editing). DNA break repair can occur via non-homologous end joining (NHEJ) or homology-directed repair (HDR). In HDR, a donor DNA repair template or template polynucleotide that contains homology arms flanking sites of the target DNA can be provided.

In some embodiments, a guide moiety or complex comprising a nuclease does not generate a double-strand break in a target polynucleotide, such as DNA.

### III. COMPLEXES

Disclosed herein, in some aspects, are one or more complexes that comprise a heterologous polypeptide and a heterologous polynucleotide. In some cases, a complex can comprise a heterologous gene effector and a guide moiety, for example, a guide nucleic acid and/or a nuclease, such as an endonuclease that lacks or substantially lacks cleavage activity.

Complexes of the disclosure can be useful, for example, for bringing one or more heterologous gene effectors into close proximity with a target gene (e.g., target endogenous gene) or target gene regulatory sequence, thereby facilitating modulation of an expression, epigenetic modification, or activity level of the target gene.

In some embodiments, a complex of the disclosure binds to DNA, e.g., genomic DNA. In some embodiments, a complex of the disclosure binds to RNA, e.g., mRNA, microRNA, siRNA, or non-coding RNA. In some embodiments, a complex of the disclosure binds to DNA and RNA.

In some embodiments, a complex can modulate (e.g., increase or decrease) expression and/or activity of a target gene (e.g., target endogenous gene) by physical obstruction of a polynucleotide sequence (e.g., a promoter, enhancer, repressor, operator, or silencer, insulator, cis-regulatory element, trans-regulatory element, epigenetic modification (e.g., DNA methylation) site, coding sequence).

In some embodiments, a complex can modulate (e.g., increase or decrease) expression and/or activity of a target gene (e.g., target endogenous gene) by recruitment of additional factors effective to suppress or enhance expression of the target gene.

In some embodiments, complexes of the disclosure are used for introducing epigenetic modifications to a target gene (e.g., target endogenous gene) or target gene regulatory sequence (e.g., promoter, enhancer, silencer, insulator, cis-regulatory element, trans-regulatory element, or epigenetic modification (e.g., DNA methylation) site). In some embodiments, complexes of the disclosure are used for producing three-dimensional structures, topologically associating domains, or genomic boundaries comprising a target gene or target gene regulatory sequence (e.g., distal or proximal gene from the target gene).

In some embodiments, a complex comprises a heterologous gene effector and a guide moiety. In some embodiments, a complex comprises one heterologous gene effector and one guide moiety. In some embodiments, a complex comprises two heterologous gene effectors and one guide moiety. In some embodiments, a complex comprises three or more heterologous gene effectors and one guide moiety.

In some embodiments, a complex comprises a heterologous gene effector and a guide nucleic acid. In some embodiments, a complex comprises one heterologous gene effector and one guide nucleic acid. In some embodiments, a complex comprises two heterologous gene effectors and one guide nucleic acid. In some embodiments, a complex comprises three or more heterologous gene effectors and one guide nucleic acid.

Two components present in a complex can be covalently linked, for example, present in a fusion protein, or cross-linked, e.g., treated with a crosslinking agent, or joined by a peptide or non-peptide linker as disclosed herein.

In some embodiments, two components present in a complex are part of the same fusion protein. Components can optionally be joined by a linker, such as a peptide linker or a non-peptide linker.

In some embodiments, a guide moiety or a part thereof (e.g., nuclease, such as dCas9) is joined to a heterologous gene effector by a linker. In some embodiments the guide moiety or part thereof is further joined to a second heterologous gene effector by a second linker that is the same or different. In some embodiments, a guide moiety or a part thereof (e.g., nuclease, such as dCas9) is fused to a heterologous gene effector without a linker.

In some embodiments, a guide moiety or a part thereof (e.g., nuclease, such as dCas9) is joined to an oligomerization domain or dimerization (e.g., heterodimerization) domain by a linker. In some embodiments the guide moiety or part thereof is further joined to a second oligomerization domain or dimerization (e.g., heterodimerization) domain by a second linker that is the same or different. In some embodiments, a guide moiety or a part thereof (e.g., nuclease, such as dCas9) is fused to a second oligomerization domain or dimerization (e.g., heterodimerization) domain without a linker.

In some embodiments, heterologous gene effector is joined to a second heterologous gene effector by a linker. In some embodiments the heterologous gene effector is further joined to a third heterologous gene effector by a second linker that is the same or different. In some embodiments, a heterologous gene effector is fused to a second heterologous gene effector without a linker.

In some embodiments, heterologous gene effector is joined to an oligomerization domain or dimerization (e.g., heterodimerization) domain by a linker. In some embodiments the heterologous gene effector is further joined to a second oligomerization domain or dimerization (e.g., heterodimerization) domain by a second linker that is the same or different. In some embodiments, a heterologous gene effector is fused to a second oligomerization domain or dimerization (e.g., heterodimerization) domain without a linker.

Any suitable linker can be used. A flexible linker can have a sequence containing stretches of glycine and serine residues. The small size of the glycine and serine residues provides flexibility, and allows for mobility of the connected functional domains. The incorporation of serine or threonine can maintain the stability of the linker in aqueous solutions by forming hydrogen bonds with the water molecules, thereby reducing unfavorable interactions between the linker and protein moieties. Flexible linkers can also contain additional amino acids such as threonine and alanine to maintain flexibility, as well as polar amino acids such as lysine and glutamine to improve solubility. A rigid linker can have, for example, an alpha helix-structure. An alpha-helical rigid linker can act as a spacer between protein domains.

A linker sequence can be, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 amino acid residues in length.

In some embodiments, a linker is at least 1, at least 2, at least 3, at least 5, at least 7, at least 9, at least 11, at least 13, at least 15, or at least 20 amino acids. In some embodiments, a linker is at most 5, at most 7, at most 9, at most 11, at most 13, at most 15, at most 20, at most 25, at most 30, at most 40, or at most 50 amino acids.

In some embodiments, non-peptide linkers are used. A non-peptide linker can be, for example a chemical linker. Two parts of a complex of the disclosure can be connected by a chemical linker. Each chemical linker of the disclosure can be alkylene, alkenylene, alkynylene, heteroalkylene, cycloalkylene, heterocycloalkylene, arylene, or heteroarylene, any of which is optionally substituted. In some embodiments, a chemical linker of the disclosure can be an ester, ether, amide, thioether, or polyethyleneglycol (PEG). In some embodiments, a linker can reverse the order of the amino acids sequence in a compound, for example, so that the amino acid sequences linked by the linked are head-to-head, rather than head-to-tail. Non-limiting examples of such linkers include diesters of dicarboxylic acids, such as oxalyl diester, malonyl diester, succinyl diester, glutaryl diester, adipyl diester, pimetyl diester, fumaryl diester, maleyl diester, phthalyl diester, isophthalyl diester, and terephthalyl diester. Non-limiting examples of such linkers include diamides of dicarboxylic acids, such as oxalyl diamide, malonyl diamide, succinyl diamide, glutaryl diamide, adipyl diamide, pimetyl diamide, fumaryl diamide, maleyl diamide, phthalyl diamide, isophthalyl diamide, and terephthalyl diamide. Non-limiting examples of such linkers include diamides of diamino linkers, such as ethylene diamine, 1,2-di(methylamino)ethane, 1,3-diaminopropane, 1,3-di(methylamino)propane, 1,4-di(methylamino)butane, 1,5-di(methylamino)pentane, 1,6-di(methylamino)hexane, and pipyrizine. Non-limiting examples of optional substituents include hydroxyl groups, sulfhydryl groups, halogens, amino groups, nitro groups, nitroso groups, cyano groups, azido groups, sulfoxide groups, sulfone groups, sulfonamide groups, carboxyl groups, carboxaldehyde groups, imine groups, alkyl groups, halo-alkyl groups, alkenyl groups, halo-alkenyl groups, alkynyl groups, halo-alkynyl groups, alkoxy groups, aryl groups, aryloxy groups, aralkyl groups, arylalkoxy groups, heterocyclyl groups, acyl groups, acyloxy groups, carbamate groups, amide groups, ureido groups, epoxy groups, and ester groups.

Two components present in a complex can be non-covalently coupled, for example, by ionic bonds, hydrogen bonds, interactions mediated by oligomerization or dimerization domains disclosed herein, etc.

In some embodiments, a guide moiety or a part thereof (e.g., nuclease, such as dCas9) is joined to a heterologous gene effector by non-covalent coupling. In some embodiments the guide moiety or part thereof is further joined to a second heterologous gene effector by non-covalent coupling. In some embodiments the guide moiety or part thereof is joined to a first heterologous gene effector covalently (e.g., as a fusion protein, optionally with a linker), and the guide moiety or part thereof is further joined to a second heterologous gene effector by non-covalent coupling.

In some embodiments, a guide moiety or a part thereof (e.g., nuclease, such as dCas9) is joined to an oligomerization domain or dimerization (e.g., heterodimerization) domain by non-covalent coupling. In some embodiments the guide moiety or part thereof is further joined to a second oligomerization domain or dimerization (e.g., heterodimerization) domain by non-covalent coupling. In some embodiments, a guide moiety or a part thereof (e.g., nuclease, such as dCas9) is fused to a first oligomerization domain or dimerization (e.g., heterodimerization) domain by covalent coupling (e.g., fused, optionally by a linker) and is joined to a second oligomerization domain or dimerization (e.g., heterodimerization) domain by non-covalent coupling.

In some embodiments, a first component of a guide moiety (e.g., a guide nucleic acid) is joined to a second component of the guide moiety (e.g., nuclease) non-covalently. In some embodiments, a first component of a guide moiety (e.g., a guide nucleic acid) is joined to a second component of the guide moiety (e.g., nuclease) covalently.

Any combination of covalent and non-covalent coupling can be used in a complex of the disclosure, for example, one or more heterologous gene effectors can be fused to a guide moiety non-covalently, and one or more oligomerization domains can be bound to a component of the complex (e.g., nuclease) covalently.

In some embodiments, a polypeptide providing increased or decreased stability is fused to or otherwise associated with a component of a complex of the disclosure, e.g., a guide moiety or a heterologous gene effector. The fused polypeptide can be located at the N-terminus, the C-terminus, or internally within the fusion protein.

In some embodiments, one or more components of a complex of the disclosure is fused to a domain the directs desirable sub-cellular localization, for example, a nuclear localization signal or a protein for targeting to the inner nuclear membrane, outer nuclear membrane, Cajal body, nuclear speckle, nuclear pore complex, PML body, nucleolus, P granule, GW body, stress granule, sponge body, endoplasmic reticulum, mitochondria, etc.

In some embodiments, a complex of the disclosure comprises a first protein linked to a first oligomerization (e.g., dimerization) domain, and a second protein linked to a second oligomerization (e.g., dimerization) domain. In some embodiments, an oligomerization domain or a dimerization domain can comprise a peptide interaction domain, for example, systems utilizing sgRNA2.0, SAM, SunTag, RAB, FLAG-biotin, or inducible oligomerization (e.g., dimerization) systems disclosed herein.

### DELIVERY

One or more genes encoding any of the heterologous polypeptide (e.g., the heterologous gene effectors) and any additional molecule operatively coupled thereto (e.g., the heterologous polynucleotide, such as one or more guide nucleic acid molecules), as disclosed herein, can be integrated into a genome of the cell, in which the aberrant expression of a target gene is to be modified. Alternatively, the one or more genes may not and need not be integrated into the genome of the cell.

Any of the heterologous polypeptide (e.g., the heterologous gene effectors) and any additional molecule operatively coupled thereto (e.g., the heterologous polynucleotide, such as one or more guide nucleic acid molecules) can be introduced (e.g., delivered, expressed, etc.) to a cell by various methods, e.g., viral and non-viral delivery methods. Viral vector delivery systems can include DNA and RNA viruses, which can have either episomal or integrated genomes after delivery to the cell. Non-viral vector delivery systems can include DNA plasmids, RNA (e.g. a transcript of a vector described herein), naked nucleic acid, and nucleic acid complexed with a delivery vehicle, such as a liposome.

RNA or DNA viral based systems can be used to target specific cells and traffic the viral payload to the nucleus of the cell. Viral vectors can be used to treat cells in vitro, and the modified cells can optionally be administered (ex vivo). Alternatively, viral vectors can be administered directly (in vivo) to the subject. Viral based systems can include retroviral, lentivirus, adenoviral, adeno-associated and herpes simplex virus vectors for gene transfer. Integration in the host genome can occur with the retrovirus, lentivirus, and adeno-associated virus gene transfer methods, which can result in long term expression of the inserted transgene.

Non-limiting examples of viral vectors that can be utilized to deliver the heterologous polypeptide and/or heterologous polynucleotide (or one or more genes encoding thereof) can include, but are not limited to, retroviral vectors, lentiviral vectors, adenovirus vectors, poxvirus vectors, herpesvirus vectors, adeno-associated virus (AAV) vectors. Non-limiting examples of AAV vectors can include AAV1, AAV10, AAV106.1/hu.37, AAVI11, AAV114.3/hu.40, AAV12, AAV127.2/hu.41, AAV127.5/hu.42, AAV128.1/hu.43, AAV128.3/hu.44, AAV130.4/hu.48, AAV145.1/hu.53, AAV145.5/hu.54, AAV145.6/hu.55, AAV16.12/hu. 11, AAV16.3, AAV16.8/hu. 10, AAV161.10/hu.60, AAV161.6/hu.61, AAV1-7/rh.48, AAV1-8/rh.49, AAV2, AAV2.5T, AAV2-15/rh.62, AAV223.1, AAV223.2, AAV223.4, AAV223.5, AAV223.6, AAV223.7, AAV2-3/rh.61, AAV24.1, AAV2-4/rh.50, AAV2-5/rh.51, AAV27.3, AAV29.3/bb.1, AAV29.5/bb.2, AAV2G9, AAV-2-pre-miRNA-101, AAV3, AAV3.1/hu.6, AAV3.1/hu.9, AAV3-11/rh.53, AAV3-3, AAV33.12/hu. 17, AAV33.4/hu. 15, AAV33.8/hu. 16, AAV3-9/rh.52, AAV3a, AAV3b, AAV4, AAV4-19/rh.55, AAV42.12, AAV42-10, AAV42-11, AAV42-12, AAV42-13, AAV42-15, AAV42-1b, AAV42-2, AAV42-3a, AAV42-3b, AAV42-4, AAV42-5a, AAV42-5b, AAV42-6b, AAV42-8, AAV42-aa, AAV43-1, AAV43-12, AAV43-20, AAV43-21, AAV43-23, AAV43-25, AAV43-5, AAV4-4, AAV44.1, AAV44.2, AAV44.5, AAV46.2/hu.28, AAV46.6/hu.29, AAV4-8/r11.64, AAV4-8/rh.64, AAV4-9/rh.54, AAV5, AAV52.1/hu.20, AAV52/hu. 19, AAV5-22/rh.58, AAV5-3/rh.57, AAV54.1/hu.21, AAV54.2/hu.22, AAV54.4R/hu.27, AAV54.5/hu.23, AAV54.7/hu.24, AAV58.2/hu.25, AAV6, AAV6.1, AAV6.1.2, AAV6.2, AAV7, AAV7.2, AAV7.3/hu.7, AAV8, AAV-8b, AAV-8h, AAV9, AAV9.11, AAV9.13, AAV9.16, AAV9.24, AAV9.45, AAV9.47, AAV9.61, AAV9.68, AAV9.84, AAV9.9, AAVA3.3, AAVA3.4, AAVA3.5, AAVA3.7, AAV-b, AAVC1, AAVC2, AAVC5, AAVCh.5, AAVCh.5R1, AAVcy.2, AAVcy.3, AAVcy.4, AAVcy.5, AAVCy.5R1, AAVCy.5R2, AAVCy.5R3, AAVCy.5R4, AAVcy.6, AAV-DJ, AAV-DJ8, AAVF3, AAVF5, AAV-h, AAVH-1/hu.1, AAVH2, AAVH-5/hu.3, AAVH6, AAVhE1.1, AAVhER1.14, AAVhEr1.16, AAVhEr1.18, AAVhER1.23, AAVhEr1.35, AAVhEr1.36, AAVhEr1.5, AAVhEr1.7, AAVhEr1.8, AAVhEr2.16, AAVhEr2.29, AAVhEr2.30, AAVhEr2.31, AAVhEr2.36, AAVhEr2.4, AAVhEr3.1, AAVhu.1, AAVhu.10, AAVhu.11, AAVhu.11, AAVhu.12, AAVhu.13, AAVhu.14/9, AAVhu.15, AAVhu.16, AAVhu.17, AAVhu.18, AAVhu.19, AAVhu.2, AAVhu.20, AAVhu.21, AAVhu.22, AAVhu.23.2, AAVhu.24, AAVhu.25, AAVhu.27, AAVhu.28, AAVhu.29, AAVhu.29R, AAVhu.3, AAVhu.31, AAVhu.32, AAVhu.34, AAVhu.35, AAVhu.37, AAVhu.39, AAVhu.4, AAVhu.40, AAVhu.41, AAVhu.42, AAVhu.43, AAVhu.44, AAVhu.44R1, AAVhu.44R2, AAVhu.44R3, AAVhu.45, AAVhu.46, AAVhu.47, AAVhu.48, AAVhu.48R1, AAVhu.48R2, AAVhu.48R3, AAVhu.49, AAVhu.5, AAVhu.51, AAVhu.52, AAVhu.53, AAVhu.54, AAVhu.55, AAVhu.56, AAVhu.57, AAVhu.58, AAVhu.6, AAVhu.60, AAVhu.61, AAVhu.63, AAVhu.64, AAVhu.66, AAVhu.67, AAVhu.7, AAVhu.8, AAVhu.9, AAVhu.t 19, AAVLG-10/rh.40, AAVLG-4/rh.38, AAVLG-9/hu.39, AAVLG-9/hu.39, AAV-LKO1, AAV-LK02, AAVLK03, AAV-LK03, AAV-LK04, AAV-LK05, AAV-LKO6, AAV-LK07, AAV-LK08, AAV-LK09, AAV-LK10, AAV-LK11, AAV-LK12, AAV-LK13, AAV-LK14, AAV-LK15, AAV-LK17, AAV-LK18, AAV-LK19, AAVN721-8/rh.43, AAV-PAEC, AAV-PAEC11, AAV-PAEC12, AAV-PAEC2, AAV-PAEC4, AAV-PAEC6, AAV-PAEC7, AAV-PAEC8, AAVpi.1, AAVpi.2, AAVpi.3, AAVrh.10, AAVrh.12, AAVrh.13, AAVrh.13R, AAVrh.14, AAVrh.17, AAVrh.18, AAVrh.19, AAVrh.2, AAVrh.20, AAVrh.21, AAVrh.22, AAVrh.23, AAVrh.24, AAVrh.25, AAVrh.2R, AAVrh.31, AAVrh.32, AAVrh.33, AAVrh.34, AAVrh.35, AAVrh.36, AAVrh.37, AAVrh.37R2, AAVrh.38, AAVrh.39, AAVrh.40, AAVrh.43, AAVrh.44, AAVrh.45, AAVrh.46, AAVrh.47, AAVrh.48, AAVrh.48, AAVrh.48.1, AAVrh.48.1.2, AAVrh.48.2, AAVrh.49, AAVrh.50, AAVrh.51, AAVrh.52, AAVrh.53, AAVrh.54, AAVrh.55, AAVrh.56, AAVrh.57, AAVrh.58, AAVrh.59, AAVrh.60, AAVrh.61, AAVrh.62, AAVrh.64, AAVrh.64R1, AAVrh.64R2, AAVrh.65, AAVrh.67, AAVrh.68, AAVrh.69, AAVrh.70, AAVrh.72, AAVrh.73, AAVrh.74, AAVrh.8, AAVrh.8R, AAVrh8R, AAVrh8R A586R mutant, AAVrh8R R533A mutant, BAAV, BNP61 AAV, BNP62 AAV, BNP63 AAV, bovine AAV, caprine AAV, Japanese AAV 10, true type AAV (ttAAV), UPENN AAV 10, AAV-LK16, AAAV, AAV Shuffle 100-1, AAV Shuffle 100-2, AAV Shuffle 100-3, AAV Shuffle 100-7, AAV Shuffle 10-2, AAV Shuffle 10-6, AAV Shuffle 10-8, AAV SM 100-10, AAV SM 100-3, AAV SM 10-1, AAV SM 10-2, and AAV SM 10-8. For example, AAVrh.74 can be used as a viral vector to deliver a polynucleotide sequence encoding the heterologous polypeptide and the heterologous polynucleotide (e.g., Cas protein-gene effector fusion and one or more guide nucleic acid molecules).

Methods of non-viral delivery of nucleic acids can include lipofection, nucleofection, microinjection, biolistics, virosomes, liposomes, immunoliposomes, polycation or lipid:nucleic acid conjugates, lipid nanoparticles (LNPs), naked DNA, artificial virions, and agent-enhanced uptake of DNA. Cationic and neutral lipids that are suitable for efficient receptor-recognition lipofection of polynucleotides can be used.

Any of the compositions disclosed herein (or one or more genes encoding any portion of the compositions), such as the heterologous gene effector(s) and/or the guide nucleic acid molecule(s), can be administered by any suitable administration route, including but not limited to, parenteral (e.g., intravenous, intratumoral, subcutaneous, intramuscular, intracerebral, intracerebroventricular, intra-articular, intraperitoneal, or intracranial), intranasal, buccal, sublingual, oral, or rectal administration routes. In some instances, the pharmaceutical composition is formulated for parenteral (e.g., intravenous, intratumoral, subcutaneous, intramuscular, intracerebral, intracerebroventricular, intra-articular, intraperitoneal, or intracranial) administration.

### TARGET GENE(S)

The disclosure provides compositions, methods, and systems for modulating expression of target genes (e.g., target endogenous genes). For example, disclosed herein are complexes that comprise a guide moiety and one or more heterologous gene effectors that can increase or decrease an activity or expression level of a target gene.

In some embodiments, a target gene or regulatory sequence thereof is endogenous to a subject, for example, present in the subject's genome. In some embodiments, a target gene or regulatory sequence thereof is not part of an engineered reporter system.

In some embodiments, a target gene is exogenous to a host subject, for example, a pathogen target gene or an exogenous gene expressed as a result of a therapeutic intervention, such as a gene therapy and/or cell therapy. In some embodiments, a target gene is an exogenous reporter gene. In some embodiments, a target gene is an exogenous synthetic gene.

In some embodiments, a target gene (e.g., target endogenous gene) is a gene that is over-expressed or under-expressed in a disease or condition. In some embodiments, a target gene is a gene that is over-expressed or under-expressed in a heritable genetic disease.

In some embodiments, a target gene (e.g., target endogenous gene) is a gene that is over-expressed or under-expressed in an autoimmune disease. In some embodiments, a target gene is a gene that is over-expressed or under-expressed in Acute disseminated encephalomyelitis, Acute motor axonal neuropathy, Addison's disease, Adiposis dolorosa, Adult-onset Still's disease, Alopecia areata, Ankylosing Spondylitis, Anti-Glomerular Basement Membrane nephritis, Anti-neutrophil cytoplasmic antibody-associated vasculitis, Anti-N-Methyl-D-Aspartate Receptor Encephalitis, Antiphospholipid syndrome, Antisynthetase syndrome, Aplastic anemia, Autoimmune Angioedema, Autoimmune Encephalitis, Autoimmune enteropathy, Autoimmune hemolytic anemia, Autoimmune hepatitis, Autoimmune inner ear disease, Autoimmune lymphoproliferative syndrome, Autoimmune neutropenia, Autoimmune oophoritis, Autoimmune orchitis, Autoimmune pancreatitis, Autoimmune polyendocrine syndrome, Autoimmune polyendocrine syndrome type 2, Autoimmune polyendocrine syndrome type 3, Autoimmune progesterone dermatitis, Autoimmune retinopathy, Autoimmune thrombocytopenic purpura, Autoimmune thyroiditis, Autoimmune urticaria, Autoimmune uveitis, Balo concentric sclerosis, Behçet's disease, Bickerstaff's encephalitis, Bullous pemphigoid, Celiac disease, Chronic fatigue syndrome, Chronic inflammatory demyelinating polyneuropathy, Churg-Strauss syndrome, Cicatricial pemphigoid, Cogan syndrome, Cold agglutinin disease, Complex regional pain syndrome, CREST syndrome, Crohn's disease, Dermatitis herpetiformis, Dermatomyositis, Diabetes mellitus type 1, Discoid lupus erythematosus, Endometriosis, Enthesitis, Enthesitis-related arthritis, Eosinophilic esophagitis, Eosinophilic fasciitis, Epidermolysis bullosa acquisita, Erythema nodosum, Essential mixed cryoglobulinemia, Evans syndrome, Felty syndrome, Fibromyalgia, Gastritis, Gestational pemphigoid, Giant cell arteritis, Goodpasture syndrome, Graves' disease, Graves ophthalmopathy, Guillain-Barré syndrome, Hashimoto's Encephalopathy, Hashimoto Thyroiditis, Henoch-Schonlein purpura, Hidradenitis suppurativa, Idiopathic dilated cardiomyopathy, Idiopathic inflammatory demyelinating diseases, IgA nephropathy, IgG4-related systemic disease, Inclusion body myositis, Inflamatory Bowel Disease (IBD), Intermediate uveitis, Interstitial cystitis, Juvenile Arthritis, Kawasaki's disease, Lambert-Eaton myasthenic syndrome, Leukocytoclastic vasculitis, Lichen planus, Lichen sclerosus, Ligneous conjunctivitis, Linear IgA disease, Lupus nephritis, Lupus vasculitis, Lyme disease, Ménière's disease, Microscopic colitis, Microscopic polyangiitis, Mixed connective tissue disease, Mooren's ulcer, Morphea, Mucha-Habermann disease, Multiple sclerosis, Myasthenia gravis, Myocarditis, Myositis, Neuromyelitis optica, Neuromyotonia, Opsoclonus myoclonus syndrome, Optic neuritis, Ord's thyroiditis, Palindromic rheumatism, Paraneoplastic cerebellar degeneration, Parry Romberg syndrome, Parsonage-Turner syndrome, Pediatric Autoimmune Neuropsychiatric Disorder Associated with Streptococcus, Pemphigus vulgaris, Pernicious anemia, Pityriasis lichenoides et varioliformis acuta, POEMS syndrome, Polyarteritis nodosa, Polymyalgia rheumatica, Polymyositis, Postmyocardial infarction syndrome, Postpericardiotomy syndrome, Primary biliary cirrhosis, Primary immunodeficiency, Primary sclerosing cholangitis, Progressive inflammatory neuropathy, Psoriasis, Psoriatic arthritis, Pure red cell aplasia, Pyoderma gangrenosum, Raynaud's phenomenon, Reactive arthritis, Relapsing polychondritis, Restless leg syndrome, Retroperitoneal fibrosis, Rheumatic fever, Rheumatoid arthritis, Rheumatoid vasculitis, Sarcoidosis, Schnitzler syndrome, Scleroderma, Sjogren's syndrome, Stiff person syndrome, Subacute bacterial endocarditis, Susac's syndrome, Sydenham chorea, Sympathetic ophthalmia, Systemic Lupus Erythematosus, Systemic scleroderma, Thrombocytopenia, Tolosa-Hunt syndrome, Transverse myelitis, Ulcerative colitis, Undifferentiated connective tissue disease, Urticaria, Urticarial vasculitis, Vasculitis, or Vitiligo.

In some embodiments, a target gene (e.g., target endogenous gene) is a gene that is over-expressed or under-expressed in a cancer, for example, acute leukemia, astrocytomas, biliary cancer (cholangiocarcinoma), bone cancer, breast cancer, brain stem glioma, bronchioloalveolar cell lung cancer, cancer of the adrenal gland, cancer of the anal region, cancer of the bladder, cancer of the endocrine system, cancer of the esophagus, cancer of the head or neck, cancer of the kidney, cancer of the parathyroid gland, cancer of the penis, cancer of the pleural/peritoneal membranes, cancer of the salivary gland, cancer of the small intestine, cancer of the thyroid gland, cancer of the ureter, cancer of the urethra, carcinoma of the cervix, carcinoma of the endometrium, carcinoma of the fallopian tubes, carcinoma of the renal pelvis, carcinoma of the vagina, carcinoma of the vulva, cervical cancer, chronic leukemia, colon cancer, colorectal cancer, cutaneous melanoma, ependymoma, epidermoid tumors, Ewings sarcoma, gastric cancer, glioblastoma, glioblastoma multiforme, glioma, hematologic malignancies, hepatocellular (liver) carcinoma, hepatoma, Hodgkin's Disease, intraocular melanoma, Kaposi sarcoma, lung cancer, lymphomas, medulloblastoma, melanoma, meningioma, mesothelioma, multiple myeloma, muscle cancer, neoplasms of the central nervous system (CNS), neuronal cancer, small cell lung cancer, non-small cell lung cancer, osteosarcoma, ovarian cancer, pancreatic cancer, pediatric malignancies, pituitary adenoma, prostate cancer, rectal cancer, renal cell carcinoma, sarcoma of soft tissue, schwanoma, skin cancer, spinal axis tumors, squamous cell carcinomas, stomach cancer, synovial sarcoma, testicular cancer, uterine cancer, or tumors and their metastases, including refractory versions of any of the above cancers, or a combination thereof.

In some embodiments, a target gene (e.g., target endogenous gene) is a differentiation-associated gene, for example, SSEA1, SSEA3/4, SSEA5, TRA1-60/81, TRA1-85, TRA2-54, GCTM-2, TG343, TG30, CD9, CD29, CD133/prominin, CD140a, CD56, CD73, CD90, CD105, OCT4, NANOG, SOX2, CD30, CD50, AHR, Aiolos/IKZF3, CDX4, CREB, DNMT3A, DNMT3B, EGR1, FoxO3, GATA-1, GATA-2, GATA-3, Helios, HES-1, HHEX, HIF-1 alpha/HIF1A, HMGB1/HMG-1, HMGB3, Ikaros, c-Jun, LMO2, LMO4, c-Maf, MafB, MEF2C, MYB, c-Myc, NFATC2, NFIL3/E4BP4, Nrf2, p53, PITX2, PRDM16/MEL1, Prox1, PU.1/Spi-1, RUNX1/CBFA2, SALL4, SCL/Tal1, Smad2, Smad2/3, Smad4, Smad7, Spi-B, STAT Activators, STAT Inhibitors, STAT3, STAT4, STAT5a, STAT6, TSC22, DUX4, DUX4/DUX4c, DUX4c, EBF-1, EBF-2, EBF-3, ETV5, FoxC2, FoxF1, GATA-4, GATA-6, HMGA2, c-Jun, MYF-5, Myocardin, MyoD, Myogenin, NFATC2, p53, Pax3, PDX-1/IPF1, PLZF, PRDM16/MEL1, RUNX2/CBFA1, Smad1, Smad3, Smad4, Smad5, Smad8, Smad9, Snail, SOX2, SOX9, SOX11, STAT Activators, STAT Inhibitors, STAT1, STAT3, TBX18, Twist-1, Twist-2, Brachyury, EOMES, FoxC2, FoxD3, FoxF1, FoxH1, FoxO1/PKHR, GATA-2, GATA-3, GBX2, Goosecoid, HES-1, HNF-3 alpha/FoxA1, c-Jun, KLF2, KLF4, KLF5, c-Maf, Max, MEF2C, MIXL1, MTF2, c-Myc, Nanog, NFkB/IkB Activators, NFkB/IkB Inhibitors, NFkB1, NFkB2, Oct-3/4, Otx2, p53, Pax2, Pax6, PRDM14, Rex-1/ZFP42, SALL1, SALL4, Smad1, Smad2, Smad2/3, Smad3, Smad4, Smad5, Smad8, Snail, SOX2, SOX7, SOX15, SOX17, STAT Activators, STAT Inhibitors, STAT3, SUZ12, TBX6, TCF-3/E2A, THAP11, UTF1, WDR5, WT1, ZNF206, ZNF281, KLF2, KLF4, c-Maf, c-Myc, Nanog, Oct-3/4, p53, SOX1, SOX2, SOX3, SOX15, SOX18, TBX18, ASCL2/Mash2, CDX2, DNMT1, ELF3, Ets-1, FoxM1, FoxN1, GATA-6, Hairless, HNF-4 alpha/NR2A1, IRF6, c-Maf, MITF, Miz-1/ZBTB17, MSX1, MSX2, MYB, c-Myc, Neurogenin-3, NFATC1, NKX3.1, Nrf2, p53, p63/TP73L, Pax2, Pax3, RUNX1/CBFA2, RUNX2/CBFA1, RUNX3/CBFA3, Smad1, Smad2, Smad2/3, Smad4, Smad5, Smad7, Smad8, Snail, SOX2, SOX9, STAT Activators, STAT Inhibitors, STAT3, SUZ12, TCF-3/E2A, TCF7/TCF1, Androgen R/NR3C4, AP-2 gamma, beta-Catenin, beta-Catenin Inhibitors, Brachyury, CREB, ER alpha/NR3A1, ER beta/NR3A2, FoxM1, FoxO3, FRA-1, GLI-1, GLI-2, GLI-3, HIF-1 alpha/HIF1A, HIF-2 alpha/EPAS1, HMGA1B, c-Jun, JunB, KLF4, c-Maf, MCM2, MCM7, MITF, c-Myc, Nanog, NFkB/IkB Activators, NFkB/IkB Inhibitors, NFkB1, NKX3.1, Oct-3/4, p53, PRDM14, Snail, SOX2, SOX9, STAT Activators, STAT Inhibitors, STAT3, TAZ/WWTR1, TBX3, Twist-1, Twist-2, WT1, or ZEB1.

In some embodiments, modulation of the expression level and/or epigenetic level (e.g., methylation level) of the target gene in the target cell (e.g., muscle cell) can effect modification (e.g., upregulation or downregulation) of a downstream gene (e.g., one or more downstream genes) of the target gene. In some cases, the target gene can be encoded by the D4Z4 repeat array (e.g., target gene being DUX4), and the downstream genes that are in turn modified in their expressions (e.g., downregulated) can include, but are not limited to, ZSCAN4, LEUTX, MBD3L2, TRIM48, TRIM43, DEFB103, ZFN217, RNASEL, EIF2AK2, BMP2, SP1 P21, MYC, MURF1, ATROGIN1, CRYM, PRAMEF1, RFPL2, KHDC1, SPRYD5, TPRX1, HSPA2, FGFR3, SLC2A14, ID2, PVRL3, SFRS2B, THOC4, ZNHIT6, DBR1, TFIP11, FBXO33, USP29, TRIM23, SLC34A2, CSAG3, and/or PNMA6B.

In some embodiments, modulation of the expression level and/or epigenetic level (e.g., methylation level) of the target gene in the target cell can effect apoptosis of the target cell (e.g., muscle cell). In some cases, such modulation of the target gene can reduce stress in the target cell. For example, the modulation of the target gene (e.g., DUX4) can effect downregulation of one or more stress-related markers in the target cell. Non-limiting examples of the one or more stress-related markers can include ACTH, glucocorticoid receptor, CRHR-1/2, POMC, prolactin, arginine vasopressin receptor V1a, superoxide dismutase 1, superoxide dismutase 2, peroxiredoxin-3, CCR5, iNOS, eNOS, heme oxygenase-2, cyclooxygenase-2, HSP27, HSP40, HSP60, HSP70, HSP70i, HSP90, HSP110, GRP78/BIP, AIF, annexin II, annexin IV, caspase 1, caspase 2, caspase 3, caspase 6, cytokeratin, E-cadherin, and/or Annexin V, caspase 5, caspase 7, caspase 8, caspase 9, caspase 10, BAD, BAX, BAK, BCL2, BID, PARP-1, NOXA, PUMA, RIPK3, RIPK1, FADD, APAF1, DFF40, DFF45, ROCK. The one or more stress-related markers as disclosed herein can be an apoptotic marker.

In some embodiments, a heterologous gene effector is from a gene product that is a hematopoietic stem cell transcription factor. In some embodiments, a target gene is a mesenchymal stem cell transcription factor. In some embodiments, a target gene is an embryonic stem cell transcription factor. In some embodiments, a target gene is an induced pluripotent stem cell (iPSC) transcription factor. In some embodiments, a target gene is an epithelial stem cell transcription factor. In some embodiments, a target gene is a cancer stem cell transcription factor.

In some embodiments, a target gene is an age-related gene. In some embodiments, a target gene is a senescence-associated protein. In some embodiments, a target gene is a drug target.

In some embodiments, a target gene (e.g., target endogenous gene) is a cancer-related gene. Non-limiting examples of cancer-related genes include A1CF, ABI1, ABL1, ABL2, ACKR3, ACSL3, ACSL6, ACVR1, ACVR2A, AFDN, AFF1, AFF3, AFF4, AKAP9, AKT1, AKT2, AKT3, ALDH2, ALK, AMER1, ANK1, APC, APOBEC3B, AR, ARAF, ARHGAP26, ARHGAP5, ARHGEF10, ARHGEF10L, ARHGEF12, ARID1A, ARID1B, ARID2, ARNT, ASPSCR1, ASXL1, ASXL2, ATF1, ATIC, ATM, ATP1A1, ATP2B3, ATR, ATRX, AXIN1, AXIN2, B2M, BAP1, BARD1, BAX, BAZ1A, BCL10, BCL11A, BCL11B, BCL2, BCL2L12, BCL3, BCL6, BCL7A, BCL9, BCL9L, BCLAF1, BCOR, BCORL1, BCR, BIRC3, BIRC6, BLM, BMP5, BMPR1A, BRAF, BRCA1, BRCA2, BRD3, BRD4, BRIP1, BTG1, BTK, BUB1B, C15orf65, CACNA1D, CALR, CAMTA1, CANT1, CARD11, CARS, CASP3, CASP8, CASP9, CBFA2T3, CBFB, CBL, CBLB, CBLC, CCDC6, CCNB1IP1, CCNC, CCND1, CCND2, CCND3, CCNE1, CCR4, CCR7, CD209, CD274, CD28, CD74, CD79A, CD79B, CDC73, CDH1, CDH10, CDH11, CDH17, CDK12, CDK4, CDK6, CDKN1A, CDKN1B, CDKN2A, CDKN2C, CDX2, CEBPA, CEP89, CHCHD7, CHD2, CHD4, CHEK2, CHIC2, CHST11, CIC, CIITA, CLIP1, CLP1, CLTC, CLTCL1, CNBD1, CNBP, CNOT3, CNTNAP2, CNTRL, COL1A1, COL2A1, COL3A1, COX6C, CPEB3, CREB1, CREB3L1, CREB3L2, CREBBP, CRLF2, CRNKL1, CRTC1, CRTC3, CSF1R, CSF3R, CSMD3, CTCF, CTNNA2, CTNNB1, CTNND1, CTNND2, CUL3, CUX1, CXCR4, CYLD, CYP2C8, CYSLTR2, DAXX, DCAF12L2, DCC, DCTN1, DDB2, DDIT3, DDR2, DDX10, DDX3X, DDX5, DDX6, DEK, DGCR8, DICER1, DNAJB1, DNM2, DNMT3A, DROSHA, DUX4L1, EBF1, ECT2L, EED, EGFR, EIF1AX, EIF3E, EIF4A2, ELF3, ELF4, ELK4, ELL, ELN, EML4, EP300, EPAS1, EPHA3, EPHA7, EPS15, ERBB2, ERBB3, ERBB4, ERC1, ERCC2, ERCC3, ERCC4, ERCC5, ERG, ESR1, ETNK1, ETV1, ETV4, ETV5, ETV6, EWSR1, EXT1, EXT2, EZH2, EZR, FAM131B, FAM135B, FAM47C, FANCA, FANCC, FANCD2, FANCE, FANCF, FANCG, FAS, FAT1, FAT3, FAT4, FBLN2, FBXO11, FBXW7, FCGR2B, FCRL4, FEN1, FES, FEV, FGFR1, FGFR1OP, FGFR2, FGFR3, FGFR4, FH, FHIT, FIP1L1, FKBP9, FLCN, FLI1, FLNA, FLT3, FLT4, FNBP1, FOXA1, FOXL2, FOXO1, FOXO3, FOXO4, FOXP1, FOXR1, FSTL3, FUBP1, FUS, GAS7, GATA1, GATA2, GATA3, GLI1, GMPS, GNA11, GNAQ, GNAS, GOLGA5, GOPC, GPC3, GPC5, GPHN, GRIN2A, GRM3, H3F3A, H3F3B, HERPUD1, HEY1, HIF1A, HIP1, HIST1H3B, HIST1H4I, HLA-A, HLF, HMGA1, HMGA2, HMGN2P46, HNF1A, HNRNPA2B1, HOOK3, HOXA11, HOXA13, HOXA9, HOXC11, HOXC13, HOXD11, HOXD13, HRAS, HSP90AA1, HSP90AB1, ID3, IDH1, IDH2, IGF2BP2, IGH, IGK, IGL, IKBKB, IKZF1, IL2, IL21R, IL6ST, IL7R, IRF4, IRS4, ISX, ITGAV, ITK, JAK1, JAK2, JAK3, JAZF1, JUN, KAT6A, KAT6B, KAT7, KCNJ5, KDM5A, KDM5C, KDM6A, KDR, KDSR, KEAP1, KIAA1549, KIF5B, KIT, KLF4, KLF6, KLK2, KMT2A, KMT2C, KMT2D, KNL1, KNSTRN, KRAS, KTN1, LARP4B, LASP1, LATS1, LATS2, LCK, LCP1, LEF1, LEPROTL1, LHFPL6, LIFR, LMNA, LMO1, LMO2, LPP, LRIG3, LRP1B, LSM14A, LYL1, LZTR1, MACC1, MAF, MAFB, MALAT1, MALT1, MAML2, MAP2K1, MAP2K2, MAP2K4, MAP3K1, MAP3K13, MAPK1, MAX, MB21D2, MDM2, MDM4, MDS2, MECOM, MED12, MEN1, MET, MGMT, MITF, MLF1, MLH1, MLLT1, MLLT10, MLLT11, MLLT3, MLLT6, MN1, MNX1, MPL, MRTFA, MSH2, MSH6, MSI2, MSN, MTCP1, MTOR, MUC1, MUC16, MUC4, MUTYH, MYB, MYC, MYCL, MYCN, MYD88, MYH11, MYH9, MYO5A, MYOD1, N4BP2, NAB2, NACA, NBEA, NBN, NCKIPSD, NCOA1, NCOA2, NCOA4, NCOR1, NCOR2, NDRG1, NF1, NF2, NFATC2, NFE2L2, NFIB, NFKB2, NFKBIE, NIN, NKX2-1, NONO, NOTCH1, NOTCH2, NPM1, NR4A3, NRAS, NRG1, NSD1, NSD2, NSD3, NT5C2, NTHL1, NTRK1, NTRK3, NUMA1, NUP214, NUP98, NUTM1, NUTM2B, NUTM2D, OLIG2, OMD, P2RY8, PABPC1, PAFAH1B2, PALB2, PATZ1, PAX3, PAX5, PAX7, PAX8, PBRM1, PBX1, PCBP1, PCM1, PDCD1LG2, PDE4DIP, PDGFB, PDGFRA, PDGFRB, PER1, PHF6, PHOX2B, PICALM, PIK3CA, PIK3CB, PIK3R1, PIM1, PLAG1, PLCG1, PML, PMS1, PMS2, POLD1, POLE, POLG, POLQ, POT1, POU2AF1, POU5F1, PPARG, PPFIBP1, PPM1D, PPP2R1A, PPP6C, PRCC, PRDM1, PRDM16, PRDM2, PREX2, PRF1, PRKACA, PRKAR1A, PRKCB, PRPF40B, PRRX1, PSIP1, PTCH1, PTEN, PTK6, PTPN11, PTPN13, PTPN6, PTPRB, PTPRC, PTPRD, PTPRK, PTPRT, PWWP2A, QKI, RABEP1, RAC1, RAD17, RAD21, RAD51B, RAF1, RALGDS, RANBP2, RAP1GDS1, RARA, RB1, RBM10, RBM15, RECQL4, REL, RET, RFWD3, RGPD3, RGS7, RHOA, RHOH, RMI2, RNF213, RNF43, ROBO2, ROS1, RPL10, RPL22, RPL5, RPN1, RSPO2, RSPO3, RUNX1, RUNX1T1, S100A7, SALL4, SBDS, SDC4, SDHA, SDHAF2, SDHB, SDHC, SDHD, 44444, 44445, 44448, SET, SETBP1, SETD1B, SETD2, SETDB1, SF3B1, SFPQ, SFRP4, SGK1, SH2B3, SH3GL1, SHTN1, SIRPA, SIX1, SIX2, SKI, SLC34A2, SLC45A3, SMAD2, SMAD3, SMAD4, SMARCA4, SMARCB1, SMARCD1, SMARCE1, SMC1A, SMO, SND1, SNX29, SOCS1, SOX2, SOX21, SPECC1, SPEN, SPOP, SRC, SRGAP3, SRSF2, SRSF3, SS18, SS18L1, SSX1, SSX2, SSX4, STAG1, STAG2, STAT3, STAT5B, STAT6, STIL, STK11, STRN, SUFU, SUZ12, SYK, TAF15, TAL1, TAL2, TBL1XR1, TBX3, TCEA1, TCF12, TCF3, TCF7L2, TCL1A, TEC, TENT5C, TERT, Tet1, Tet2, TFE3, TFEB, TFG, TFPT, TFRC, TGFBR2, THRAP3, TLX1, TLX3, TMEM127, TMPRSS2, TNC, TNFAIP3, TNFRSF14, TNFRSF17, TOP1, TP53, TP63, TPM3, TPM4, TPR, TRA, TRAF7, TRB, TRD, TRIM24, TRIM27, TRIM33, TRIP11, TRRAP, TSC1, TSC2, TSHR, U2AF1, UBR5, USP44, USP6, USP8, VAV1, VHL, VTI1A, WAS, WDCP, WIF1, WNK2, WRN, WT1, WWTR1, XPA, XPC, XPO1, YWHAE, ZBTB16, ZCCHC8, ZEB1, ZFHX3, ZMYM2, ZMYM3, ZNF331, ZNF384, ZNF429, ZNF479, ZNF521, ZNRF3, and ZRSR2.

In some embodiments, a target gene (e.g., target endogenous gene) is an immune cell-related gene, for example, a cytokine, cytokine receptor, chemokine, chemokine receptor, co-inhibitory immune receptor, co-stimulatory immune receptor, immune cell transcription factor, etc.

In some embodiments, a target gene (e.g., target endogenous gene) is a cytokine, for example, 4-1BBL, APRIL, CD153, CD154, CD178, CD70, G-CSF, GITRL, GM-CSF, IFN-α, IFN-β, IFN-γ, IL-1RA, IL-1α, IL-1β, IL-2, IL-3, IL-4, IL-5, IL-6, IL-7, IL-9, IL-10, IL-11, IL-12, IL-13, IL-14, IL-15, IL-16, IL-17, IL-18, IL-20, IL-23, LIF, LIGHT, LT-β, M-CSF, MSP, OSM, OX40L, SCF, TALL-1, TGF-β, TGF-β1, TGF-β2, TGF-β3, TNF-α, TNF-β, TRAIL, TRANCE, or TWEAK.

In some embodiments, a target gene (e.g., target endogenous gene) is a cytokine receptor, for example, A common gamma chain receptor, a common beta chain receptor, an interferon receptor, a TNF family receptor, a TGF-B receptor, Apo3, BCMA, CD114, CD115, CD116, CD117, CD118, CD120, CD120a, CD120b, CD121, CD121a, CD121b, CD122, CD123, CD124, CD126, CD127, CD130, CD131, CD132, CD212, CD213, CD213a1, CD213a13, CD213a2, CD25, CD27, CD30, CD4, CD40, CD95 (Fas), CDw119, CDw121b, CDw125, CDw131, CDw136, CDw137 (41BB), CDw210, CDw217, GITR, HVEM, IL-11R, IL-11Ra, IL-14R, IL-15R, IL-15Ra, IL-18R, IL-18Rα, IL-18Rβ, IL-20R, IL-20Rα, IL-20Rβ, IL-9R, LIFR, LTβR, OPG, OSMR, OX40, RANK, TACI, TGF-βR1, TGF-βR2, TGF-βR3, TRAILR1, TRAILR2, TRAILR3, or TRAILR4.

In some embodiments, a target gene (e.g., target endogenous gene) is a chemokine, for example, ACT-2, AMAC-a, ATAC, ATAC, BLC, CCL1, CCL11, CCL13, CCL14, CCL15, CCL16, CCL17, CCL18, CCL19, CCL2, CCL20, CCL21, CCL22, CCL23, CCL24, CCL25, CCL26, CCL27, CCL3, CCL4, CCL5, CCL7, CCL8, CKb-6, CKb-8, CTACK, CX3CL1, CXCL1, CXCL10, CXCL11, CXCL12, CXCL13, CXCL14, CXCL2, CXCL3, CXCL4, CXCL5, CXCL6, CXCL7, CXCL8, CXCL9, DC-CK1, ELC, ENA-78, eotaxin, eotaxin-2, eotaxin-3, Eskine, exodus-1, exodus-2, exodus-3, fractalkine, GCP-2, GROa, GROb, GROg, HCC-1, HCC-2, HCC-4, I-309, IL-8, ILC, IP-10, I-TAC, LAG-1, LARC, LCC-1, LD78α, LEC, Lkn-1, LMC, lymphoactin, lymphoactin b, MCAF, MCP-1, MCP-2, MCP-3, MCP-4, MDC, MDNCF, MGSA-a, MGSA-b, MGSA-g, Mig, MIP-1d, MIP-1α, MIP-1β, MIP-2a, MIP-2b, MIP-3, MIP-3α, MIP-3β, MIP-4, MIP-4a, MIP-5, MPIF-1, MPIF-2, NAF, NAP-1, NAP-2, oncostatin, PARC, PF4, PPBP, RANTES, SCM-1a, SCM-1b, SDF-1α/β -, SLC, STCP-1, TARC, TECK, XCL1, or XCL2.

In some embodiments, a target gene (e.g., target endogenous gene) is a chemokine receptor, for example, CCR1, CCR2, CCR3, CCR4, CCR5, CCR6, CCR7, CCR8, CCR9, CCR10, CX3CR1, CXCR1, CXCR2, CXCR3, CXCR4, CXCR5, XCR1, or XCR1.

In some embodiments, a target gene (e.g., target endogenous gene) is an activating NK receptor, for example, CD100 (SEMA4D), CD16 (FcgRIIIA), CD160 (BY55), CD244 (2B4, SLAMF4), CD27, CD94- NKG2C, CD94- NKG2E, CD94-NKG2H, CD96, CRTAM, DAP12, DNAM1 (CD226), KIR2DL4, KIR2DS1, KIR2DS2, KIR2DS3, KIR2DS4, KIR2DS5, KIR3DS1, Ly49, NCR, NKG2D (KLRK1, CD314), NKp30 (NCR3), NKp44 (NCR2), NKp46 (NCR1), NKp80 (KLRF1, CLEC5C), NTB-A (SLAMF6), PSGL1, or SLAMF7 (CRACC, CS1, CD319).

In some embodiments, a target gene (e.g., target endogenous gene) is an inhibitory NK receptor, for example, CD161 (NKR-P1A, NK1.1), CD94-NKG2A, CD96, CEACAM1, KIR2DL1, KIR2DL2, KIR2DL3, KIR2DL4, KIR2DL5A, KIR2DL5B, KIR3DL1, KIR3DL2, KIR3DL3, KLRG1, LAIR1, LIR1 (ILT2, LILRB1), Ly49a, Ly49b, NKR-P1A (KLRB1), SIGLEC-10, SIGLEC-11, SIGLEC-14, SIGLEC-16, SIGLEC-3 (CD33), SIGLEC-5 (CD170), SIGLEC-6 (CD327), SIGLEC-7 (CD328), SIGLEC-8, SIGLEC-9 (CD329), SIGLEC-E, SIGLEC-F, SIGLEC-G, SIGLEC-H, or TIGIT.

In some embodiments, a target gene (e.g., target endogenous gene) is a co-inhibitory immune receptor, for example, 2B4, B7-1, BTLA, CD160, CTLA-4, DR6, Fas, LAG3, LAIR1, Ly108, PD-1, PD-L1, PD1H, TIGIT, TIM1, TIM2, or TIM3.

In some embodiments, a target gene (e.g., target endogenous gene) is co-stimulatory immune receptor, for example, 2B4, 4-1BB, CD2, CD4, CD8, CD21, CD27, CD28, CD30, CD40, CD84, CD226, CD355, CRACC, DcR3, DR3, GITR, HVEM, ICOS, Ly9, Ly108, LIGHT, LTβR, OX40, SLAM, TIM1, or TIM2.

In some embodiments, a target gene (e.g., target endogenous gene) is itself a gene effector, such as any of the gene effectors disclosed herein (e.g., a transcription factor disclosed herein).

In some embodiments, a target gene (e.g., target endogenous gene) is an immune cell transcription factor, for example, AP-1, Bcl6, E2A, EBF, Eomes, FoxP3, GATA3, Id2, Ikaros, IRF, IRF1, IRF2, IRF3, IRF3, IRF7, NFAT, NFkB, Pax5, PLZF, PU.1, ROR-gamma-T, STAT, STAT1, STAT2, STAT3, STAT4, STATS, STAT5A, STAT5B, STAT6, T-bet, TCF7, or ThPOK.

In some embodiments, a target gene is a kinase, for example, a tyrosine kinase, or serine/threonine kinase. In some embodiments, a target gene is a phosphatase, for example, a tyrosine phosphatase, or serine/threonine phosphatase.

In some embodiments, a target gene is a receptor. In some embodiments, a target gene is an ion channel. In some embodiments, a target gene is a GPCR. In some embodiments, a target gene is a receptor tyrosine kinase. In some embodiments, a target gene is a ribosomal protein. In some embodiments, a target gene is a membrane protein. In some embodiments, a target gene is a cytoplasmic protein. In some embodiments, a target gene is a nuclear protein. In some embodiments, a target gene is a mitochondrial protein. In some embodiments, a target gene is a ubiquitin ligase. In some embodiments, a target gene is a methyltransferase. In some embodiments, a target gene is a glycosyltransferase. In some embodiments, a target gene is a hydrolase.

In some embodiments, CD45 is a target gene used in compositions and methods of the disclosure (e.g., for gene expression activation screens). In some embodiments, CD45 is not used as a target gene. Compositions and methods disclosed herein to identify complexes that modulate CD45 expression can similarly be modified and adapted to other target genes (e.g., target endogenous genes), including those disclosed herein.

In some embodiments, CD71 is a target gene used in compositions and methods of the disclosure (e.g., for gene expression reduction screens). In some embodiments, CD71 is not used as a target gene. Compositions and methods disclosed herein to identify complexes that modulate CD71 expression can similarly be modified and adapted to other target genes (e.g., target endogenous genes), including those disclosed herein.

### CELLS

As set forth in the claims, systems, methods and complexes for use are applied to muscle cells. Also disclosed herein are compositions, methods, and systems that are applied to cells of various types, and populations thereof. For example, a complex of the disclosure can be used to elicit changes in the expression, epigenetic modification, or activity level of a target gene (e.g., target endogenous gene) in cells of a particular type, or populations thereof. Methods of the disclosure can be used to identify complexes that are capable of eliciting changes in the expression or activity of target genes (e.g., target endogenous genes) in cells of a particular type, or populations thereof.

Disclosed herein, a complex or a heterologous gene effector identified by methods of the disclosure effects a desirable change in expression of a target gene (e.g., target endogenous gene) that is specific to a particular cell type. Disclosed herein, a complex or a heterologous gene effector identified by methods of the disclosure effects a desirable change in expression of a target gene (e.g., target endogenous gene) that is applicable to two or more cell types. Disclosed herein, a complex or a heterologous gene effector identified by methods of the disclosure effects a desirable change in expression of a target gene (e.g., target endogenous gene) that is applicable to three or more cell types. Disclosed herein, a complex or a heterologous gene effector identified by methods of the disclosure effects a desirable change in expression of a target gene (e.g., target endogenous gene) that is applicable to a class of cell types, for example, cell types with overlapping functional roles, that are present in similar tissues, or that are from the same or similar differentiation lineages, e.g., stem cells, immune cells, T cells, T effector cells, etc. Disclosed herein, a complex or a heterologous gene effector identified by methods of the disclosure effects a desirable change in expression of a target gene (e.g., target endogenous gene) that is broadly applicable to a wide variety of cell types, for example, elicits an expression level of a target gene that is above or below a certain threshold for multiple target cell types when introduced to the cells using suitable methods.

Disclosed herein, a composition, complex, system, or method of the disclosure is used to effect a change in the expression, epigenetic modification, or activity level of a target gene in a primary cell. Disclosed herein, a composition, complex, system, or method of the disclosure is used to effect a change in the expression, epigenetic modification, or activity level of a target gene in a cell line. Disclosed herein, a composition, complex, system, or method of the disclosure is used to effect a change in the expression, epigenetic modification, or activity level of a target gene in an immortalized cell.

Disclosed herein, a composition, complex, system, or method of the disclosure is used to effect a change in the expression, epigenetic modification, or activity level of a target gene (e.g., target endogenous gene) in a mammalian cell, for example, a human cell, non-human primate cell, non-rodent mammal cell, non-human mammal cell, swine cell, lagomorph cell, canine cell, etc. Disclosed herein, a composition, complex, system, or method of the disclosure is used to effect a change in the expression, epigenetic modification, or activity level of a target gene in a plant cell, an avian cell, a reptilian cell, a bacterial cell, or an archaeal cell.

A composition, complex, system, or method of the disclosure can be used to effect a change in the expression, epigenetic modification, or activity level of a target gene (e.g., target endogenous gene) in a human cell.

A composition, complex, system, or method of the disclosure can be used to effect a change in the expression, epigenetic modification, or activity level of a target gene (e.g., target endogenous gene) in a stem cell.

A composition, complex, system, or method of the disclosure can be used to effect a change in the expression, epigenetic modification, or activity level of a target gene (e.g., target endogenous gene) in a differentiated cell.

A composition, complex, system, or method of the disclosure can be used to effect a change in the expression, epigenetic modification, or activity level of a target gene (e.g., target endogenous gene) in a disease-associated cell.

A composition, complex, system, or method of the disclosure can be used to effect a change in the expression, epigenetic modification, or activity level of a target gene (e.g., target endogenous gene) in a cancer cell.

A composition, complex, system, or method of the disclosure can be used to effect a change in the expression, epigenetic modification, or activity level of a target gene (e.g., target endogenous gene) in a non-cancer cell.

A composition, complex, system, or method of the disclosure can be used to effect a change in the expression, epigenetic modification, or activity level of a target gene (e.g., target endogenous gene) in a lymphoid cell, such as a B cell, a T cell (Cytotoxic T cell, Natural Killer T cell, Regulatory T cell, T helper cell), Natural killer cell, cytokine induced killer (CIK) cells (see e.g. US20080241194); myeloid cells, such as granulocytes (Basophil granulocyte, Eosinophil granulocyte, Neutrophil granulocyte/Hypersegmented neutrophil), Monocyte/Macrophage, Red blood cell, Reticulocyte, Mast cell, Thrombocyte/Megakaryocyte, Dendritic cell; cells from the endocrine system, including thyroid (Thyroid epithelial cell, Parafollicular cell), parathyroid (Parathyroid chief cell, Oxyphil cell), adrenal (Chromaffin cell), pineal (Pinealocyte) cells; cells of the nervous system, including glial cells (Astrocyte, Microglia), Magnocellular neurosecretory cell, Stellate cell, Boettcher cell, and pituitary (Gonadotrope, Corticotrope, Thyrotrope, Somatotrope, Lactotroph); cells of the Respiratory system, including Pneumocyte (Type I pneumocyte, Type II pneumocyte), Clara cell, Goblet cell, Dust cell; cells of the circulatory system, including Myocardiocyte, Pericyte; cells of the digestive system, including stomach (Gastric chief cell, Parietal cell), Goblet cell, Paneth cell, G cells, D cells, ECL cells, I cells, K cells, S cells; enteroendocrine cells, including enterochromaffm cell, APUD cell, liver cells (e.g., Hepatocyte, or Kupffer cell), Cartilage/bone/muscle; bone cells, including Osteoblast, Osteocyte, Osteoclast, teeth cells, (Cementoblast, Ameloblast); cartilage cells, including Chondroblast, Chondrocyte; skin cells, including Trichocyte, Keratinocyte, Melanocyte (Nevus cell); muscle cells, including Myocyte; urinary system cells, including Podocyte, Juxtaglomerular cell, Intraglomerular mesangial cell/Extraglomerular mesangial cell, Kidney proximal tubule brush border cell, Macula densa cell; reproductive system cells, including Spermatozoon, Sertoli cell, Leydig cell, Ovum; and other cells, including Adipocyte, Fibroblast, Tendon cell, Epidermal keratinocyte, Epidermal basal cell, Keratinocyte of fingernails and toenails, Nail bed basal cell, Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, Hair matrix cell, Wet stratified barrier epithelial cells, Surface epithelial cell of stratified squamous epithelium of cornea, tongue, oral cavity, esophagus, anal canal, distal urethra and vagina, basal cell of epithelia of cornea, tongue, oral cavity, esophagus, anal canal, distal urethra and vagina, Urinary epithelium cell, Exocrine secretory epithelial cells, Salivary gland mucous cell, Salivary gland serous cell, Von Ebner's gland cell in tongue, Mammary gland cell, Lacrimal gland cell, Ceruminous gland cell in ear, Eccrine sweat gland dark cell, Eccrine sweat gland clear cell. Apocrine sweat gland cell, Gland of Moll cell in eyelid, Sebaceous gland cell, Bowman's gland cell in nose, Brunner's gland cell in duodenum, Seminal vesicle cell, Prostate gland cell, Bulbourethral gland cell, Bartholin's gland cell, Gland of Littre cell, Uterus endometrium cell, Isolated goblet cell of respiratory and digestive tracts, Stomach lining mucous cell, Gastric gland zymogenic cell, Gastric gland oxyntic cell, Pancreatic acinar cell, Paneth cell of small intestine, Type II pneumocyte of lung, Clara cell of lung, Hormone secreting cells, Anterior pituitary cells, Somatotropes, Lactotropes, Thyrotropes, Gonadotropes, Corticotropes, Intermediate pituitary cell, Magnocellular neurosecretory cells, Gut and respiratory tract cells, Thyroid gland cells, thyroid epithelial cell, parafollicular cell, Parathyroid gland cells, Parathyroid chief cell, Oxyphil cell, Adrenal gland cells, chromaffin cells, Ley dig cell of testes, Theca interna cell of ovarian follicle, Corpus luteum cell of ruptured ovarian follicle, Granulosa lutein cells, Theca lutein cells, Juxtaglomerular cell, Macula densa cell of kidney, Metabolism and storage cells, Barrier function cells (e.g., Lung, Gut, Exocrine Glands and Urogenital Tract), Kidney, Type I pneumocyte, Pancreatic duct cell (centroacinar cell), Nonstriated duct cell (of sweat gland, salivary gland, mammary gland, etc.), Duct cell (of seminal vesicle, prostate gland, etc.), Epithelial cells lining closed internal body cavities, Ciliated cells with propulsive function, Extracellular matrix secretion cells, Contractile cells; Skeletal muscle cells, stem cell, Heart muscle cells, Blood and immune system cells, Erythrocyte, Megakaryocyte, Monocyte, Connective tissue macrophage (various types), Epidermal Langerhans cell, Osteoclast, Dendritic cell, Microglial cell, Neutrophil granulocyte, Eosinophil granulocyte, Basophil granulocyte, Mast cell, Helper T cell, Suppressor T cell, Cytotoxic T cell, Natural Killer T cell, B cell, Natural killer cell, Reticulocyte, Stem cells and committed progenitors for the blood and immune system (various types), Pluripotent stem cells, Totipotent stem cells, Induced pluripotent stem cells, adult stem cells, Sensory transducer cells, neurons, Autonomic neuron cells, Sense organ and peripheral neuron supporting cells, Central nervous system neurons and glial cells, Lens cells, Pigment cells, Melanocyte, Retinal pigmented epithelial cell, Germ cells, Oogonium/Oocyte, Spermatid, Spermatocyte, Spermatogonium cell, Spermatozoon, Nurse cells, Ovarian follicle cell, Sertoli cell, Thymus epithelial cell, Interstitial cells, Interstitial kidney cells, common myeloid progenitors, common lymphoid progenitors, and stem cells that are differentiated into or are to be differentiated into any cell type disclosed herein.

A composition, complex, system, or method of the disclosure can be used to effect a change in the expression, epigenetic modification, or activity level of a target gene (e.g., target endogenous gene) in a stem cell, for example, an isolated stem cell (e.g., an ESC) or an induced stem cell (e.g., an iPSC).

A composition, complex, system, or method of the disclosure can be used to effect a change in the expression, epigenetic modification, or activity level of a target gene (e.g., target endogenous gene) in a hematopoietic stem cell, for example, a hematopoietic stem cell from a subject, for example, from bone marrow, or peripheral blood (e.g., a mobilized peripheral blood apheresis product, for example, mobilized by administration of GCSF, GM-CSF, mozobil, or a combination thereof).

In some cases, pluripotency of stem cells (e.g., ESCs or iPSCs) can be determined, in part, by assessing pluripotency characteristics of the cells. Pluripotency characteristics can include, but are not limited to: pluripotent stem cell morphology; the potential for unlimited self-renewal; expression of pluripotent stem cell markers including, but not limited to SSEA1, SSEA3/4, SSEA5, TRA1-60/81, TRA1-85, TRA2-54, GCTM-2, TG343, TG30, CD9, CD29, CD133/prominin, CD140a, CD56, CD73, CD90, CD105, OCT4, NANOG, SOX2, CD30 and/or CD50; ability to differentiate to all three somatic lineages (ectoderm, mesoderm and endoderm); ability to form teratomas comprising the three somatic lineages; and/or (vi) formation of embryoid bodies comprising cells from the three somatic lineages.

A composition, complex, system, or method of the disclosure can be used to effect a change in the expression, epigenetic modification, or activity level of a target gene (e.g., target endogenous gene) in an immune cell, for example, lymphocytes, T cells, CD4+ T cells, CD8+ T cells, alpha-beta T cells, gamma-delta T cells, T regulatory cells (Tregs), cytotoxic T lymphocytes, Th1 cells, Th2 cells, Th17 cells, Th9 cells, naive T cells, memory T cells, effector T cells, effector-memory T cells (TEM), central memory T cells (TCM), resident memory T cells (TRM), follicular helper T cells (TFH), Natural killer T cells (NKTs), tumor-infiltrating lymphocytes (TILs), Natural killer cells (NKs), Innate Lymphoid Cells (ILCs), ILC1 cells, ILC2 cells, ILC3 cells, lymphoid tissue inducer (LTi) cells, B cells, B1 cells, B1a cells, B1b cells, B2 cells, plasma cells, B regulatory cells, memory B cells, marginal zone B cells, follicular B cells, germinal center B cells, antigen presenting cells (APCs), monocytes, macrophages, M1 macrophages, M2 macrophages, tissue-associated macrophages, dendritic cells, plasmacytoid dendritic cells, neutrophils, mast cells, basophils, eosinophils, common myeloid progenitors, common lymphoid progenitors, or any combination thereof.

A composition, complex, system, or method of the disclosure can be used to effect a change in the expression, epigenetic modification, or activity level of an engineered cell that is used to manufacture a biologic, for example, an antibody or other protein-based therapeutic.

### EXAMPLES

### Example 1: Regulation of DUX4 expression in a target cell population

A population of lymphoblasts was used as an example target cell population for regulating DUX4 expression level by the compositions and methods disclosed herein. The population of lymphoblasts were contacted by (i) the heterologous actuator moiety coupled to a gene regulator and (ii) a guide RNA (see **Table 1)** designed to direct the heterologous actuator moiety to a target polynucleotide sequence between two CpG islands within a D4Z4 repeat array that encodes DUX4 in the population of lymphoblasts. A number of the guide RNAs was able to allow the heterologous actuator moiety coupled to the gene regulator to complex with its respective target polynucleotide sequence in the population of lymphoblasts, and yield between about 0.2-fold and about 0.8-fold reduction in the DUX4 expression level **(****Figure. 2****).**

**Table 1. Guide RNA molecules used in FIG. 2.**

| **SEQ ID NO** | **Name** | **Sequence** |
|---|---|---|
| SEQ ID NO. 1 | DUX4_1 gRNA | CGCGGGGAGGGTGCTGTCCG |
| SEQ ID NO. 2 | DUX4_2 gRNA | CCATCGCGGTGAGCCCCGGC |
| SEQ ID NO. 3 | DUX4_3 gRNA | GGGCGTCGCCGTTGCCGGGA |
| SEQ ID NO. 4 | DUX4_4 gRNA | GAATGGCGGTGAGCCCCCCT |
| SEQ ID NO. 5 | DUX4_5 gRNA | CGGCTCTCCGGACCTCTCCA |
| SEQ ID NO. 6 | DUX4_6 gRNA | GACCCAGGGCGTCGAGGCCT |
| SEQ ID NO. 7 | DUX4_7 gRNA | TGACGGCGGTCCGCTTTCGC |
| SEQ ID NO. 8 | DUX4_10 gRNA | TCCAGGCATCGCCGCCCGGG |
| SEQ ID NO. 9 | DUX4_11 gRNA | CGGGACGGTCTCGCACACGC |
| SEQ ID NO. 10 | DUX4_12 gRNA | CCTTTACAAGGGCGGCTGGC |
| SEQ ID NO. 11 | DUX4_13 gRNA | CTCTCTGGGCTCCCACGCGT |
| SEQ ID NO. 12 | DUX4_14 gRNA | GTGCGAGACCGTCCCGGCAA |
| SEQ ID NO. 13 | DUX4_15 gRNA | TCTCCCTGCTGCCGACGCGT |
| SEQ ID NO. 14 | DUX4_91 gRNA | GTACGGGTTCCGCTCAAAGC |

### Example 2: Validation of in vitro FSHD model

In order to develop an *in vitro* FSHD model, two immortalized patient-derived FSHD skeletal myoblasts (SkM) cells, 12ABIC (12A) and 15ABIC (15A), were expanded in complete growth media. Upon confluence, the complete growth media was changed to differentiation media conditions for either 2 days or 7 days (D2 or D7 in **FIG. 3A**). The experiment included a negative control which was undifferentiated, proliferating myoblasts (UD in **FIG. 3A**). After differentiation, total RNA was extracted from the myoblasts and qRT-PCR was performed using TaqMan probes for DUX4 and DUX4-target genes ZSAC4, LEUTZ, MBD3L2, TRIM48, and TRIM43. GAPDH was included as an internal reference control for the qRT-pCR measurements and the double delta Ct method was used to calculate the gene fold change. The 12ABIC and 15ABIC cells showed increased DUX4 and DUX4-target gene expression consistent with FSHD presentation in patients.

The 12ABIC and 15ABIC cells were then tested for whether they also showed increased apoptosis consistent with the FSHD phenotype in patients. The 12ABIC and 15ABIC cells, as well as two corresponding healthy control cells 12UBIC and 15VBIC, respectively, were growth and then differentiated for 2 days after staining for an apoptosis marker, Caspase 3. The assay also included DAPI staining as a positive control. The cells were then imaged and analyzed using CellXpress PICO Imager. As shown in **FIG. 3B****,** the 12ABIC and 15ABIC cells had increased apoptosis levels compared to their healthy sibling control myoblasts, 12UBIC and 15VBIC, at day 2 of differentiation. The 12ABIC, 15ABIC, 12UBIC, and 15VBIC cells were grown, differentiated, stained, imaged, and analyzed until day 7 of differentiation using the CellXpress PICO Imager. The percent of apoptotic cells for each cell type was plotted on day 0, 1, 2, and 7 of differentiation **(****FIG. 3C****).** 12ABIC and 15ABIC cells had a higher level of apoptosis at day 2 and day 7 compared to their corresponding healthy controls. This increase in apoptosis during differentiation was consistent with the *in vivo* phenotype of FSHD.

To ensure that the 12ABIC and 15ABIC cells had similar myoblast differentiation compared to their healthy sibling controls, all four cell types were immunostained for Myosin Heavy Chain (MYHC), which was a late muscle gene used as a marker for muscle cell differentiation **(****FIG. 3D****).** The immunostaining assay also included DAPI staining as a positive control. In addition, the four cell types were assayed for the expression of MYHC, MYOG, and MyoMaker (MYMK) via qRT-PCR **(****FIG. 3E****).** GADPH was included in the qRT-PCR was an internal control for the qRT-PCR measurements. MYOG was an essential myogenic regulatory factor that regulates skeletal muscle differentiation and MYMK was a late muscle gene used as a marker for muscle cell differentiation. The results of both the immunostaining and qRT-PCR experiments showed that the 12ABIC and 15ABIC cells had similar differentiation to their corresponding healthy sibling controls.

Overall, the results of these validation experiments showed that 12ABIC and 15ABIC cells presented the *in vivo* phenotypes of FSHD myoblasts and thus were an appropriate *in vitro* model for FSHD.

### Example 3: Targeting of DUX4 for downregulation of expression

In order to target DUX4 for downregulation, numerous gRNAs were designed that spanned over the entire DZ4Z region locus, which included regions coding for long noncoding RNA, DBET, in the 5' end of the D4Z4 locus. The DZ4Z region locus was known to upregulate DUX4 gene expression upon deletion of the repeat units and DBET lncRNA has been shown to positively regulate the expression of DUX4 from the DZ4Z locus. The gRNAs were designed using the ChopChop CRISPR guide design tool. When designing the gRNAs, Hg38 human genome assembly was used with TTTR as the PAM sequence requirement. The map of the gRNAs designed to the DZ4Z locus is shown in **FIG. 4****.**

Once the gRNAs were designed, the different gRNAs were tested with a Cas12f variant construct coupled with a KRAB modulator. 12ABIC cells stably expressing the Cas12f-KRAB effector-modulator were generated after lentiviral transduction of 12ABIC myoblasts. The design of the Cas12f-KRAB effector-modulator vector is shown in **FIG. 5****.** The vector included a muscle-specific promoter (CK8e) to drive the expression of the Cas12f variant effector and the KRAB and DNMT3L domains. A human U6g promoter was included to drive the expression of the sgRNA spacer sequence with scaffold driven by RNA polymerase III. The vector additionally included a modified WPRE and polyadenylation regulatory sequences. The Cas12f-KRAB effector-modulator was labeled with mCherry, so after transduction, mCherry+ cells were sorted for enrichment. Following enrichment, annealed crRNA:trcrRNA constructs for 78 guides were nucleofected in the Cas12f-KRAB effector-modulator-expressing 12ABIC cells. After differentiation of the myoblasts for 7 cells, the cells were assayed for expression of DUX4 **(****FIG. 6A** and **6B****)** and MDB3L2 **(****FIG. 6B****)** using Quantigene assay probes. The relative expression of DUX4 was normalized to expression of control gene HPRT1. The experiments showed that the different gRNAs were capable of downregulating expression of DUX4 and MDB3L2 in cells expressing the Cas12f-KRAB effector-modulator. In addition, the downregulation of DUX4 and MDB3L2 by the different gRNAs were positively correlated **(****FIG. 6B****).**

From the initial screen, six of the gRNAs were further tested. The six gRNAs were transfected into immortalized patient-derived FSHD myoblasts, along with one of two different Cas12f-KRAB effector-modulators. The two different Cas12f-KRAB effector-modulators included one of two different DNMT3L domains (e.g., DNMT3L-Kla or DNMT3L-Klb). Following transfection, the cells were differentiated and expression of DUX4 and DUX4-target genes, MBD3L2, TRIM48, and MYOG, were assayed using qRT-PCR after 17 days **(****FIG. 7A****)** and 18 days **(****FIG. 7B****)** post-transfection to measure for persistence of DUX4 repression. MYOG was included as a positive control to ensure that the differentiation ability of DUX4 sgRNA transfected cells was similar to control sgRNA transfected myoblasts. Overall, it was found that the Cas12f-KRAB-DNMT3L modulators resulted in persistent repression of DUX4 and DUX4-target genes.

In addition to testing the expression of levels of DUX4 and DUX4-target genes in patient-derived myoblasts treated with the Cas12f-KRAB-DNMT3L modulator, the cells were also tested for apoptosis levels of treated cells. The treated cells were stained for apoptotic marker, Caspase-3, 2 days after differentiation. Following staining, the apoptotic-positive cells were counted using a high content imager and the percent positive cells were calculated based on total nuclei stained by DAPI (blue cells). The cells treated with the Cas12f-KRAB-DNMT3L modulator showed a decrease in apoptosis compared to cells transfected with a control sgRNA **(****FIGs. 8A** and **8B****).**

### Example 4: Ex Vivo FSHD model Establishment and Validation

Immortalized healthy sibling control cells and FSHD skeletal myoblasts can be thawed and expanded for *ex vivo* 3D studies. The skeletal myoblasts can be split onto 2D surfaces and then engineered into 3D Mantarray tissues as per established Curi Bio lab protocols as described in Fayazi, M., "Passive-Stretch Induced Skeletal Muscle Injury Platform for Duchenne Muscular Dystrophy Modeling," Archives of Physical Medicine and Rehabilitation, volume 103, issue 3, March 2022, page e26, which is hereby incorporated in its entirety by reference. Briefly, 3D-skeletal myoblasts tissues can be cultured for 7 days to allow for compaction, and then additionally cultured for 14 days. Functional measurements can be taken three-times a week during culture to assess contractile force over a period and stimulated to assess the phenotypic differences in mechanical force, tetanic force, and fatigue **(****FIG. 9****).** Upon establishment of the model, the patient-derived FSHD skeletal myoblasts can be used to test the efficacy of the control and the Cas12f effector-modulator AAV targeting DZ4Z locus for the rescue in 3D tissue morphology, gene expression profile, and mechanical forces assessments.

In some cases, a 3D-skeletal myoblast tissue that is treated with the system, composition or method as disclosed herein (e.g., to modulate expression level or epigenetic level of a gene encoded by a D4Z4 repeat array, such as DUX4) can be characterized by exhibiting (i) enhanced mechanical force (e.g., a maximum mechanical force, an average mechanical force over a period of time), (ii) enhanced tetanic force (e.g., force indicative of a sustained muscle contraction evoked when the motor nerve that innervates a skeletal muscle emits action potentials at a very high rate), and/or (iii) reduced fatigue (e.g., as measured via a contraction against a fixed, immovable object (a static test or isometric measurement), or via a dynamic muscular contraction at a controlled velocity (repeated contractions or isokinetic assessment)), as compared to a control 3D-skeletal myoblast tissue (e.g., which is not treated with the system, composition or method as disclosed herein.

In some cases, the 3D-skeletal myoblast tissue that is treated with the system, composition or method as disclosed herein can be characterized by exhibiting a mechanical force that is greater than that in the control 3D-skeletal myoblast tissue, by at least or up to about 1%, at least or up to about 5%, at least or up to about 10%, at least or up to about 15%, at least or up to about 20%, at least or up to about 30%, at least or up to about 40%, at least or up to about 50%, at least or up to about 60%, at least or up to about 70%, at least or up to about 80%, at least or up to about 90%, at least or up to about 95%, at least or up to about 100%, at least or up to about 120%, at least or up to about 150%, at least or up to about 200%, at least or up to about 300%, at least or up to about 400%, or at least or up to about 500%.

In some cases, the 3D-skeletal myoblast tissue that is treated with the system, composition or method as disclosed herein can be characterized by exhibiting a tetanic force that is greater than that in the control 3D-skeletal myoblast tissue, by at least or up to about 1%, at least or up to about 5%, at least or up to about 10%, at least or up to about 15%, at least or up to about 20%, at least or up to about 30%, at least or up to about 40%, at least or up to about 50%, at least or up to about 60%, at least or up to about 70%, at least or up to about 80%, at least or up to about 90%, at least or up to about 95%, at least or up to about 100%, at least or up to about 120%, at least or up to about 150%, at least or up to about 200%, at least or up to about 300%, at least or up to about 400%, or at least or up to about 500%.

In some cases, the 3D-skeletal myoblast tissue that is treated with the system, composition or method as disclosed herein can be characterized by exhibiting fatigue that is less than that in the control 3D-skeletal myoblast tissue, by at least or up to about 1%, at least or up to about 5%, at least or up to about 10%, at least or up to about 15%, at least or up to about 20%, at least or up to about 30%, at least or up to about 40%, at least or up to about 50%, at least or up to about 60%, at least or up to about 70%, at least or up to about 80%, at least or up to about 90%, at least or up to about 95%, at least or up to about 99%, or at least or up to about 100%.

### Example 5: In vivo FSHD model for DUX4 targeting

On the morning of designated day -7, mice can be anesthetized using 90-200 mg/mg of ketamine and 10 mg/kg of xylazine administered intraperitoneally. The hind-limb of the mouse can be subjected to X-irradiation at 25 Gy at 2.2 Gy/minute over 11-12 minutes. Six days later, 60 uL of 0.3 mg/kg cardiotoxin can be administered along the length of the TA muscle to promote degradation. One day later, 2E10^6 human myoblast cells in 60 uL can be administered along the TA muscle. Isoflurane anesthesia can be used for subsequent cardiotoxin and human myoblast administration. One day after administration of the myoblast cells, the Cas12f modulator vector of the previous examples and a control AAVrh74 vector can be administered via retroorbital injection. At day 4 and day 21, the animals can be euthanized. The TA muscles and other major organs (e.g., heart, lung, liver) can be harvested. The harvested TA muscles can be sectioned, fixed, and H&E stained. The remaining organs can be processed, and total RNA/DNA can be extracted to perform gene expression experiments using qRT-PCR. The gene expression experiments can measure the expression of DUX4 and DUX4-target genes to determine the level of DUX4 repression. The gene expression experiments can measure the expression of one or more downstream genes of DUX4, such as, for example, ZSCAN4, LEUTX, MBD3L2, TRIM48, and/or TRIM43. The gene expression experiments may also examine the enrichment of human myoblasts in the mouse as well as the AAV tropism to specific tissues. The workflow of the experiment is shown in **FIG. 10****.**

**Table 2. Guide RNA molecules for binding a target polynucleotide sequence for modifying expression level or epigenetic level of a gene (e.g., DUX4) encoded by a D4Z4 repeat array in a target cell (e.g., a muscle cell).**

| **SEQ ID NO** | **Name** | **Sequence** |
|---|---|---|
| 45 | DUX4_gRNA | TTTAAAGAGATCTGGGGATCTATA |
| 46 | DUX4_gRNA | TTTAAAGAATGGGAAAATTACGGG |
| 47 | DUX4_gRNA | TTTAACTTGGAAACACAGCGAAGT |
| 48 | DUX4_gRNA | TTTGCCTGTGAGTTCGAATGCACT |
| 49 | DUX4_gRNA | TTTGATGAAGTCTGGCTTACAGCC |
| 50 | DUX4_gRNA | TTTGCATATCTGATGGAGAACTTA |
| 51 | DUX4_gRNA | TTTGGGAATGTGTTTGTGAAGCAC |
| 52 | DUX4_gRNA | TTTGAATATACTGTGGTCATCTCT |
| 53 | DUX4_gRNA | TTTGCACTGGAGCAGAGATGACCA |
| 54 | DUX4_gRNA | TTTATTCTACTCTGCAATCCCCTA |
| 55 | DUX4_gRNA | TTTAAGATTCTGGGAGGGAGAGAA |
| 56 | DUX4_gRNA | TTTATAAATCTATTGTGCCTCAAG |
| 57 | DUX4_gRNA | TTTGATGAGTGCTGTATAGATCCC |
| 58 | DUX4_gRNA | TTTGCTACAGCACTAGTGAAACTG |
| 59 | DUX4_gRNA | TTTACTGAGCCAGTCTTTAAATGC |
| 60 | DUX4_gRNA | TTTATAAAAAATGGCATGACAAGG |
| 61 | DUX4_gRNA | TTTATCAAAAAGCCAAACATTTCA |
| 62 | DUX4_gRNA | TTTGCTCACTGAGAATGCATAAGA |
| 63 | DUX4_gRNA | TTTAAGTTCTCCATCAGATATGCA |
| 64 | DUX4_gRNA | TTTATAAATTCACTACAGAGACAC |
| 65 | DUX4_gRNA | TTTGAAATCTGGAAAGTTCTTAGC |
| 66 | DUX4_gRNA | TTTGTTGATATTTTGCTCATTCGT |
| 67 | DUX4_gRNA | TTTATGTTTTTCTTCCAATGGGGA |
| 68 | DUX4_gRNA | TTTGTGAAGCACCTAGAATCTATA |
| 69 | DUX4_gRNA | TTTAAAGACTGGCTCAGTAAAGGG |
| 70 | DUX4_gRNA | TTTAATTCTCTCCTGAAGGAGATA |
| 71 | DUX4_gRNA | TTTAAATTTCACTCAGTTGTCTCT |
| 72 | DUX4_gRNA | TTTGTGTCTGCTGAGAAGAAAGAT |
| 73 | DUX4_gRNA | TTTGATAAATTGTCTAATGACTAG |
| 74 | DUX4_gRNA | TTTATTTTTTCACCCAGAACAGTA |
| 75 | DUX4_gRNA | TTTGCTGTTGTGTCTCTGTAGTGA |
| 76 | DUX4_gRNA | TTTAAAATATTAGTTTCCAGGACT |
| 77 | DUX4_gRNA | TTTAAATGCTAGATTTGATGAGTG |
| 78 | DUX4_gRNA | TTTAAAAGACTCTATCTCTGAATG |
| 79 | DUX4_gRNA | TTTATGTTCTCACAAGATTCTGGG |
| 80 | DUX4_gRNA | TTTATGCCATTTTCTCCCTCTATT |
| 81 | DUX4_gRNA | TTTGGCATTGCTTTTGGGGATCTG |
| 82 | DUX4_gRNA | TTTAACAAATAAAGATTTTTGCAT |
| 83 | DUX4_gRNA | TTTAAAAATAACGAATGAGCAAAA |
| 84 | DUX4_gRNA | TTTGAAATACAGTATTTCCCAGAT |
| 85 | DUX4_gRNA | TTTGGGGATCTGGGAAAATCTGTG |
| 86 | DUX4_gRNA | TTTGGCTTTTTGATAAATTGTCTA |
| 87 | DUX4_gRNA | TTTGCTCATTCGTTATTTTTAAAT |
| 88 | DUX4_gRNA | TTTATAAAACTCAGTTATTATATT |
| 89 | DUX4_gRNA | TTTAAAAACCCAACAGAAATCATA |
| 90 | DUX4_gRNA | TTTGTGAATAATATATGTTCAATT |
| 91 | DUX4_gRNA | TTTATCTCTTTGTTGATATTTTGC |
| 92 | DUX4_gRNA | TTTAGATTCTATTGTATATTTTCT |
| 93 | DUX4_gRNA | TTTAAAAAGAATAGAGGGAGAAAA |
| 94 | DUX4_gRNA | TTTATTTTAATCTTTGAAAGTCTT |
| 95 | DUX4_gRNA | TTTATTTGTTAAAATTCAGTTTCT |
| 96 | DUX4_gRNA | TTTGTTAAAATTCAGTTTCTGAAT |
| 97 | DUX4_gRNA | TTTAATCTTTGAAAGTCTTTATTT |
| 98 | DUX4_gRNA | TTTACAAGGGCGGCTGGCTGGGTG |
| 99 | DUX4_gRNA | TTTGTCCCGGAGGAAACCGCCCAC |
| 100 | DUX4_gRNA | TTTGCCCTCCGCAAGGCGGCCTGT |
| 101 | DUX4_gRNA | TTTGGTTTCCGCGTGGCTTTGCCC |
| 102 | DUX4_gRNA | TTTAAAAAAAAAAATCACAAGGCA |
| 103 | DUX4_gRNA | TTTGAAAGTCTTTATTTTTTTCTA |
| 104 | DUX4_gRNA | TTTACAAGGGCGGCTGGCTGGCTG |
| 105 | DUX4_gRNA | TTTAAAAATAGTTTTTATCTCTTT |
| 106 | DUX4_gRNA | TTTATTTTTTTCTAATTTTTGAAA |
| 107 | DUX4_gRNA | TTTGCCCGGGTGCGGAGGCCAGCG |
| 108 | DUX4_gRNA | TTTAGGACGCGGGGTTGGGACGGG |
| 109 | DUX4_gRNA | TTTGCCCGGGTGCGGAGGCCACCG |
| 110 | DUX4_gRNA | TTTGGAACCTGGCAAGGAGAGCGA |
| 111 | DUX4_gRNA | TTTGCGGGCAGCCGCCTGGGCTGT |
| 112 | DUX4_gRNA | TTTGGCTCGGGGTCCAAACGAGTC |
| 113 | DUX4_gRNA | TTTGAGCGGAACCCGTACCCGGGC |
| 114 | DUX4_gRNA | TTTGGACCCCGAGCCAAAGCGAGG |
| 115 | DUX4_gRNA | TTTGCTCCCGGAGCTCTGCGGGCA |
| 116 | DUX4_gRNA | TTTACTCCCGGAGCTCTGCGGGCA |
| 117 | DUX4_gRNA | TTTGGTTTCAGAATGAGAGGTCAC |
| 118 | DUX4_gRNA | TTTACAAGAGAAAAACAAAAAACC |
| 119 | DUX4_gRNA | TTTGAGAAGGATCGCTTTCCAGGC |
| 120 | DUX4_gRNA | TTTGTTTTTCTCTTGTAAATTTTT |
| 121 | DUX4_gRNA | TTTAATAGGGTTTTTTGTTTTTCT |
| 122 | DUX4_gRNA | TTTGTCCGGAGGAAACCGCCCACT |
| 123 | DUX4_gRNA | TTTGACCGCCAGGAGCTCCGCGCT |
| 124 | DUX4_gRNA | TTTACATGAGGTTCTACTACATAC |
| 125 | DUX4_gRNA | TTTGGATTCGGGTTCAGGTTAAGA |
| 126 | DUX4_gRNA | TTTAGGGTTAGGGTAGTGTAAATA |
| 127 | DUX4_gRNA | TTTGGCTTATAGGGGCTTTGTGAG |
| 128 | DUX4_gRNA | TTTGTGGTAAAGAGTTGTGATTCT |
| 129 | DUX4_gRNA | TTTGGCCTACAGGGGGCTTTGTGA |
| 130 | DUX4_gRNA | TTTATTCACTAAATACAAATCACA |
| 131 | DUX4_gRNA | TTTATCAGTGTAATTATTAGTCAT |
| 132 | DUX4_gRNA | TTTGCAGAGATATGTCACAATCCC |
| 133 | DUX4_gRNA | TTTGGTCTAGTTTTATCAACAGAG |
| 134 | DUX4_gRNA | TTTGTCCAGTATGCTGCGGGTTGT |
| 135 | DUX4_gRNA | TTTGTTTCCTGCAATATGTCACAA |
| 136 | DUX4_gRNA | TTTACACTACCCTAACCCTAAACC |
| 137 | DUX4_gRNA | TTTGGAACGTAGGATGTTTCCATT |
| 138 | DUX4_gRNA | TTTATGACTAATAATTACACTGAT |
| 139 | DUX4_gRNA | TTTAGCAGGAACACACTACCTTTC |
| 140 | DUX4_gRNA | TTTATCAACAGAGCTAGTATTTAC |
| 141 | DUX4_gRNA | TTTAGCCTCTGCCTACAGGAGGCA |
| 142 | DUX4_gRNA | TTTACATCTCCTGAGTGAGCATTG |
| 143 | DUX4_gRNA | TTTGTCCATGATTTAGCAGGAACA |
| 144 | DUX4_gRNA | TTTGTCATGAGAGATGTGGCAGGA |
| 145 | DUX4_gRNA | TTTGGGGGACGTGCTCCTTCTGCA |
| 146 | DUX4_gRNA | TTTAAGATGAAGCCCCTTTGCTCC |
| 147 | DUX4_gRNA | TTTACCACAAACAACACAGCTTCA |
| 148 | DUX4_gRNA | TTTGTTGTGTGTGTAATGAGAACA |
| 149 | DUX4_gRNA | TTTAAGGAAAATATGCTAATTTTA |
| 150 | DUX4_gRNA | TTTAGATTCATATGGGAATACTGA |
| 151 | DUX4_gRNA | TTTATATTACACTATTACTTAATA |
| 152 | DUX4_gRNA | TTTAGCTGAGGGAGATTGAGTGAC |
| 153 | DUX4_gRNA | TTTAGAATGCTACCTATTGCCTTC |
| 154 | DUX4_gRNA | TTTGCTCCTTCCTTAAGGATGTCT |
| 155 | DUX4_gRNA | TTTGTCTTCAAAGAATGGCCTTGG |
| 156 | DUX4_gRNA | TTTGGAGTTTAAAATTAGCATATT |
| 157 | DUX4_gRNA | TTTAGCTTTCTGGAACCTGGTATG |
| 158 | DUX4_gRNA | TTTACTGATCAACCAGATGATGTA |
| 159 | DUX4_gRNA | TTTAAAATTAGCATATTTTCCTTA |
| 160 | DUX4_gRNA | TTTGGTGATATATGACACAGAGAT |
| 161 | DUX4_gRNA | TTTGATTCTGATGTAAGAAATGAT |
| 162 | DUX4_gRNA | TTTGCATCTCTGTGTCATATATCA |
| 163 | DUX4_gRNA | TTTAAACTCCAAATACTTATGAAT |
| 164 | DUX4_gRNA | TTTGAAGACAAACATGTCTTAATA |
| 165 | DUX4_gRNA | TTTGCCTAGACAGCGTCGGAAGGT |
| 166 | DUX4_gRNA | TTTATTATTAGTAATAATGTGAAA |
| 167 | DUX4_gRNA | TTTGTATTTAGTGAATAAAAACAA |
| 168 | DUX4_gRNA | TTTGTTTTTATTCACTAAATACAA |
| 169 | DUX4_gRNA | TTTAGATCACCTAGGTGATCAGTG |
| 170 | DUX4_gRNA | TTTGTTCTTATTTTAAGGAAAATA |
| 171 | DUX4_gRNA | TTTAGTGAATAAAAACAAACAAAA |
| 172 | DUX4_gRNA | TTTGTTTGTTTTTATTCACTAAAT |
| 173 | DUX4_gRNA | TTTAGGCAGATCCTAGAAAAGAGT |
| 174 | DUX4_gRNA | TTTGCATCTTTTGTGTGATGAGTG |
| 175 | DUX4_gRNA | TTTAATATATCTCTGAACTAATCA |
| 176 | DUX4_gRNA | TTTGTCTAGGCTCTGCTTACTTGG |
| 177 | DUX4_gRNA | TTTAGGGTTAGGGTTAGGGTTATG |
| 178 | DUX4_gRNA | TTTGACATATGTCTGCACTGATGA |
| 179 | DUX4_gRNA | TTTGCCCGCTTCCTGGCTAGACCT |
| 180 | DUX4_gRNA | TTTGTCTAGGCTCTGGCTACACAG |
| 181 | DUX4_gRNA | TTTGTGTGATGAGTGCAGAGATAT |
| 182 | DUX4_gRNA | TTTAGGGTTAGGGTTAGGGTTAGG |
| 183 | DUX4_gRNA | TTTGTCTAGGCTCTGCCTACATAG |
| 184 | DUX4_gRNA | TTTAACATATCTCTACACTGATCA |
| 185 | DUX4_gRNA | TTTGCCTATGGGGGCAATGTGACA |
| 186 | DUX4_gRNA | TTTGAGATATCTCTGCACTGATCA |
| 187 | DUX4_gRNA | TTTGTGATATATATTTCCACTGCT |
| 188 | DUX4_gRNA | TTTGAGCAGTGGAAATATATATCA |
| 189 | DUX4_gRNA | TTTACATAACTTCGGTGATCAGTG |
| 190 | DUX4_gRNA | TTTAGGCACAGCTTAGACAAGCGT |
| 191 | DUX4_gRNA | TTTGTCAAGGATATGGCTACAGGG |
| 192 | DUX4_gRNA | TTTGTGAGATATCTCTGCACTGAT |
| 193 | DUX4_gRNA | TTTGTGACATACTTCTGTACTGAT |
| 194 | DUX4_gRNA | TTTGCTCTGATCACCCAGGTGATG |
| 195 | DUX4_gRNA | TTTGTTGATCAGTTCAGAGATGTG |
| 196 | DUX4_gRNA | TTTGTCTACGCTCTGCCTATGGGG |
| 197 | DUX4_gRNA | TTTGTCTACAGGGGGCTTTGTGAT |
| 198 | DUX4_gRNA | TTTGTCGAAATTCCCTGTAGGCAG |
| 199 | DUX4_gRNA | TTTGTGACATACCTTTGCTCTGAT |
| 200 | DUX4_gRNA | TTTAGGCAGAGCTTAGACTAGAGT |
| 201 | DUX4_gRNA | TTTGTCTAGGCTCTGTCTACGGGG |
| 202 | DUX4_gRNA | TTTGACATATCTCTGCACTGTTAA |
| 203 | DUX4_gRNA | TTTGGCAGAGCCTAGACAAGGGTT |
| 204 | DUX4_gRNA | TTTGCCTACAGAAGGCTTTGTGAC |
| 205 | DUX4_gRNA | TTTGTCTACAGGGGGCTTTGTGAC |
| 206 | DUX4_gRNA | TTTGACACAATGCCCCCATAGACA |
| 207 | DUX4_gRNA | TTTGTGACTTCTCTCTGCACTGAT |
| 208 | DUX4_gRNA | TTTGTGACATAACTCTGCACTAAT |
| 209 | DUX4_gRNA | TTTGACATAGCTCTGCACAGATCA |
| 210 | DUX4_gRNA | TTTAAGCAGAGCCTAGACAATAGT |
| 211 | DUX4_gRNA | TTTGTGACATATCTTTGCACTGAT |
| 212 | DUX4_gRNA | TTTGCCTACAGGGGACATTGTGAC |
| 213 | DUX4_gRNA | TTTGTCTAGGCTCTGCCTAAGGGG |
| 214 | DUX4_gRNA | TTTGTCATCAGTTCAGGGATATGT |
| 215 | DUX4_gRNA | TTTGCACTGATCACCCAGGAGATG |
| 216 | DUX4_gRNA | TTTGTGACACATCTCTGCACTGAT |
| 217 | DUX4_gRNA | TTTGTGACATATCCCTGCAATGAT |
| 218 | DUX4_gRNA | TTTATAAGCACTGCCTACAGGGAA |
| 219 | DUX4_gRNA | TTTGTGACATATTTCTGCACTGAT |
| 220 | DUX4_gRNA | TTTGTGACATATCACTGCACTGAT |
| 221 | DUX4_gRNA | TTTGACATATCTCTGCACTGATCA |
| 222 | DUX4_gRNA | TTTGTGACATATCTATGCACTGAT |
| 223 | DUX4_gRNA | TTTATGACTTATCCCTGCACTGAT |
| 224 | DUX4_gRNA | TTTGTGACATATCTCTGCACAGAT |
| 225 | DUX4_gRNA | TTTGTGACATATCTCTGCACTGAT |
| 226 | DUX4_gRNA | TTTGAGATGGAGTCTTGCTCTGTT |
| 227 | DUX4_gRNA | TTTGTATTTTTAGTAGAGACGAGG |
| 228 | DUX4_gRNA | TTTGCTATCAAAAGCTTGGGTCAA |
| 229 | DUX4_gRNA | TTTAAAAGAACACTTGCGGTGTTT |
| 230 | DUX4_gRNA | TTTAAAAGATCTCTGTGGCCAGGC |
| 231 | DUX4_gRNA | TTTGAACTATAGATACAGCAGAAG |
| 232 | DUX4_gRNA | TTTAAAATATTTAACATTTAGCCC |
| 233 | DUX4_gRNA | TTTGATAGCAAAAGGTAGAAAAGA |
| 234 | DUX4_gRNA | TTTGGAGAATGAAAACGTGCAGTA |
| 235 | DUX4_gRNA | TTTGAAGGAGATCTCACAAACAGG |
| 236 | DUX4_gRNA | TTTAGAAAGGAAACAGGCTGGAAA |
| 237 | DUX4_gRNA | TTTGCTGCAAAATAAATACACGCT |
| 238 | DUX4_gRNA | TTTATCATCTATCTATCTACCTCC |
| 239 | DUX4_gRNA | TTTGTTTGAACTATAGATACAGCA |
| 240 | DUX4_gRNA | TTTGCAACTGTGGGTTTTCCAGCC |
| 241 | DUX4_gRNA | TTTAAAACAAGAACTCTTGTAGGA |
| 242 | DUX4_gRNA | TTTGCACCTTAAATCTGTGAAATC |
| 243 | DUX4_gRNA | TTTGTCGCTTCAAACACCGCAAGT |
| 244 | DUX4_gRNA | TTTACATCCATGATTTTTCACTGT |
| 245 | DUX4_gRNA | TTTGTCCATCTCACCTCTCCAGAT |
| 246 | DUX4_gRNA | TTTGAAGGTTAGAACTAGTGGTCT |
| 247 | DUX4_gRNA | TTTAAGGTGCAAAAAGTCACTGGG |
| 248 | DUX4_gRNA | TTTACTTACAAACGCAGACTGTGT |
| 249 | DUX4_gRNA | TTTGTCACAGCAGCCTTTGTCGCT |
| 250 | DUX4_gRNA | TTTGAAGCGACAAAGGCTGCTGTG |
| 251 | DUX4_gRNA | TTTGTTTCCACATTACAGAGTGGG |
| 252 | DUX4_gRNA | TTTGCCACACCTTGTTTTAGAAAG |
| 253 | DUX4_gRNA | TTTAATCTCAGTGACAGGGGAACA |
| 254 | DUX4_gRNA | TTTAAAAGAATTATATCAACCTTT |
| 255 | DUX4_gRNA | TTTGCCTCATCTTTGTTTGAACTA |
| 256 | DUX4_gRNA | TTTATGATGTGATGGAGAATTCCT |
| 257 | DUX4_gRNA | TTTACTAATCTGCTTATTACCCAC |
| 258 | DUX4_gRNA | TTTGTGAGATCTCCTTCAAATACT |
| 259 | DUX4_gRNA | TTTGTGCATTGTCTGTTACTGTGT |
| 260 | DUX4_gRNA | TTTGCAGCAAACATTTACATCCAT |
| 261 | DUX4_gRNA | TTTACTATCTGTCTTTTCTACCTT |
| 262 | DUX4_gRNA | TTTATGCCTGGCCTTTCCATCCTT |
| 263 | DUX4_gRNA | TTTGGTTTTTGAAGGTTAGAACTA |
| 264 | DUX4_gRNA | TTTAAATGGTAGAGTGAACATACA |
| 265 | DUX4_gRNA | TTTGCTTTGCAACTGTGGGTTTTC |
| 266 | DUX4_gRNA | TTTAGAAAGCTCAGGTTTATGATG |
| 267 | DUX4_gRNA | TTTAAAAAAAATTCCCTTTCACTG |
| 268 | DUX4_gRNA | TTTAAATTTTCCAAGCCATATGGT |
| 269 | DUX4_gRNA | TTTGGTTCATGAAATTTTCAGTTT |
| 270 | DUX4_gRNA | TTTGTTTTAATCTCAGTGACAGGG |
| 271 | DUX4_gRNA | TTTAAAACAAGAGTCAGCAAATAT |
| 272 | DUX4_gRNA | TTTGTTGAGAAAAAATGAGTTGGA |
| 273 | DUX4_gRNA | TTTATTTTGCAGCAAACATTTACA |
| 274 | DUX4_gRNA | TTTGCTGACTCTTGTTTTAAAAGA |
| 275 | DUX4_gRNA | TTTGTTGTTTGCTTTTCTGTGGGG |
| 276 | DUX4_gRNA | TTTGCTTTTCTGTGGGGTTTTTGT |
| 277 | DUX4_gRNA | TTTAACATTTAGCCCTTTGCAGAA |
| 278 | DUX4_gRNA | TTTGACTTCTTCCTCTGTTTTTTTG |
| 279 | DUX4_gRNA | TTTGAAAATAATTAAGCAATATCT |
| 280 | DUX4_gRNA | TTTAGCCCTTTGCAGAAAATATTT |
| 281 | DUX4_gRNA | TTTAAAAAAAGCAAACAACAACAA |
| 282 | DUX4_gRNA | TTTGCTTTTTTTAAAAAAAATTCC |
| 283 | DUX4_gRNA | TTTGTAAGTAAAGTTGTAATGGGA |
| 284 | DUX4_gRNA | TTTGTTTGTTGTTTGCTTTTCTGT |
| 285 | DUX4_gRNA | TTTGCAGAAAATATTTGCTGACTC |
| 286 | DUX4_gRNA | TTTGGGATGCCGAGGCTAGCCGAT |
| 287 | DUX4_gRNA | TTTGTTGTTGTTGTTTGCTTTTTT |
| 288 | DUX4_gRNA | TTTGTTTGTTTGTTGTTTGCTTTT |
| 289 | DUX4_gRNA | TTTAGTAGAGACGAGGTTTCACTG |
| 290 | DUX4_gRNA | TCTAATGACTAGATTCTTTCTCTC |
| 291 | DUX4_gRNA | TCTGGCCTTGTCCGTGACGTTTAA |
| 292 | DUX4_gRNA | TCTATAGCCTGGACTATTGCTGTC |
| 293 | DUX4_gRNA | TCTGTTATAGTTTTGCTCACTGAG |
| 294 | DUX4_gRNA | TCTGGAAACAGTTTGCACTGGAGC |
| 295 | DUX4_gRNA | TCTGGGGATCTATACAGCACTCAT |
| 296 | DUX4_gRNA | TCTATATGACTCCATCATGTCCTT |
| 297 | DUX4_gRNA | TCTGGCTTACAGCCTGTCCACTGC |
| 298 | DUX4_gRNA | TCTGCAATCCCCTAAGGCTTTTTC |
| 299 | DUX4_gRNA | TCTGGACTTCGCTGTGTTTCCAAG |
| 300 | DUX4_gRNA | TCTGCTCCAGTGCAAACTGTTTCC |
| 301 | DUX4_gRNA | TCTAGTCATTAGACAATTTATCAA |
| 302 | DUX4_gRNA | TCTGTGCACACTTCTGGAGACCCT |
| 303 | DUX4_gRNA | TCTATACAGCACTCATCAAATCTA |
| 304 | DUX4_gRNA | TCTACTCTGCAATCCCCTAAGGCT |
| 305 | DUX4_gRNA | TCTGGGAGGATTTTGCCTGTGAGT |
| 306 | DUX4_gRNA | TCTGGAAAGTTCTTAGCATCCCCG |
| 307 | DUX4_gRNA | TCTAGCATTTAAAGACTGGCTCAG |
| 308 | DUX4_gRNA | TCTATTTTCCTTGCTGTAACAGAG |
| 309 | DUX4_gRNA | TCTAGTAGTTAACAACCTCAGCTT |
| 310 | DUX4_gRNA | TCTGAATGTATTGGTCTACTTGAT |
| 311 | DUX4_gRNA | TCTGCTCCATTGTTTGCTGTTGTG |
| 312 | DUX4_gRNA | TCTATCTCTGAATGTATTGGTCTA |
| 313 | DUX4_gRNA | TCTGTTACAGCAAGGAAAATAGAA |
| 314 | DUX4_gRNA | TCTGCTGAGAAGAAAGATGAGTGT |
| 315 | DUX4_gRNA | TCTGATGGAGAACTTAAAATAATC |
| 316 | DUX4_gRNA | TCTGGAGACCCTTGTCATGCCATT |
| 317 | DUX4_gRNA | TCTGACTTGAGGCACAATAGATTT |
| 318 | DUX4_gRNA | TCTAGGTGCTTCACAAACACATTC |
| 319 | DUX4_gRNA | TCTGTGGTATTGCAGTTTCACTAG |
| 320 | DUX4_gRNA | TCTGTAGTGAATTTATAAAACTCA |
| 321 | DUX4_gRNA | TCTATTGTATATTTTCTTCCCCAG |
| 322 | DUX4_gRNA | TCTGCCTTCTCTGTGTGCCTTGTG |
| 323 | DUX4_gRNA | TCTACTGTTTTAATTCTCTCCTGA |
| 324 | DUX4_gRNA | TCTGGGAGGGAGAGAAAAAGCCTT |
| 325 | DUX4_gRNA | TCTGGGTGAAAAAATAAACTGCAG |
| 326 | DUX4_gRNA | TCTGGGAAAATCTGTGCACACTTC |
| 327 | DUX4_gRNA | TCTATTGTGCCTCAAGTCAGAAGT |
| 328 | DUX4_gRNA | TCTGACTAGTTTGGCATTGCTTTT |
| 329 | DUX4_gRNA | TCTGGGAAATACTGTATTTCAAAA |
| 330 | DUX4_gRNA | TCTATTCTTTTTAAAAGACTCTAT |
| 331 | DUX4_gRNA | TCTGAAATAATGTTTATGCCATTT |
| 332 | DUX4_gRNA | TCTGTCCGGCCCCACCACCACCAC |
| 333 | DUX4_gRNA | TCTGCACCAATGAAAAAAAAATTT |
| 334 | DUX4_gRNA | TCTAAAATACATTGAGAAAAAATT |
| 335 | DUX4_gRNA | TCTATGATTTCTGTTGGGTTTTTA |
| 336 | DUX4_gRNA | TCTAATTTTTGAAATACAGTATTT |
| 337 | DUX4_gRNA | TCTGAATATTTATGTTTTTCTTCC |
| 338 | DUX4_gRNA | TCTGTTGGGTTTTTAAAAATAGTT |
| 339 | DUX4_gRNA | TCTGTGTGCCTTGTGATTTTTTTT |
| 340 | DUX4_gRNA | TCTACTTGATGGTGTCCAGTAAGT |
| 341 | DUX4_gRNA | TCTGTGAACCGCGCGGGTGAAGAC |
| 342 | DUX4_gRNA | TCTGTGAACCGCGCGGGTGAAAAC |
| 343 | DUX4_gRNA | TCTGGCGGGCCGCGTCTCCCGGGC |
| 344 | DUX4_gRNA | TCTAGGTCTCCCGTTCCTCTCTCC |
| 345 | DUX4_gRNA | TCTACGTGGAAATGAACGAGAGCC |
| 346 | DUX4_gRNA | TCTGTCTTTCCCTCCGTTCCTCCC |
| 347 | DUX4_gRNA | TCTGCCCGCCTTCCCTCCCGCCTG |
| 348 | DUX4_gRNA | TCTGCCCCTGCCGCGCGGAGGCGG |
| 349 | DUX4_gRNA | TCTGCGCCCCCGCGCCACCGTCGC |
| 350 | DUX4_gRNA | TCTGGGCTCCCACGCGTCGGCAGC |
| 351 | DUX4_gRNA | TCTGGCCAGCTCCTCCCGGGCGGC |
| 352 | DUX4_gRNA | TCTGCCCCTGCCGCGCGGAGGCGT |
| 353 | DUX4_gRNA | TCTGCCCGCGTCCGTCCGTGAAAT |
| 354 | DUX4_gRNA | TCTGCCGTCGCGGCCTGGCTGGGC |
| 355 | DUX4_gRNA | TCTAGGAGAGGTTGCGCCTGCTGC |
| 356 | DUX4_gRNA | TCTGCAGCAGGCGCAACCTCTCCT |
| 357 | DUX4_gRNA | TCTGCGTTCCGCCGCCAGGCGCTC |
| 358 | DUX4_gRNA | TCTAGGCCCGGTGAGAGACTCCAC |
| 359 | DUX4_gRNA | TCTGGTCTTCTACGTGGAAATGAA |
| 360 | DUX4_gRNA | TCTGCAGTGTGGCCGGTTTGGAAC |
| 361 | DUX4_gRNA | TCTAGGTCTAGGCCCGGTGAGAGA |
| 362 | DUX4_gRNA | TCTGCACTCCCCTGCGGCCTGCTG |
| 363 | DUX4_gRNA | TCTGGGGTCTCGCTCTGGTCTTCT |
| 364 | DUX4_gRNA | TCTGGTGGCGATGCCCGGGTACGG |
| 365 | DUX4_gRNA | TCTGGGATCCGGTGACGGCGGTCC |
| 366 | DUX4_gRNA | TCTGCTGGAGGAGCTTTAGGACGC |
| 367 | DUX4_gRNA | TCTGCCGGCGCGGCCTGGCTGGGC |
| 368 | DUX4_gRNA | TCTGGGATCCCCGGGATGCCCAGG |
| 369 | DUX4_gRNA | TCTGCCCGGGCTGCTCCCACAGCC |
| 370 | DUX4_gRNA | TCTGAATCCTGGACTCCGGGAGGC |
| 371 | DUX4_gRNA | TCTGCGGGCACCCGGAAACATGCA |
| 372 | DUX4_gRNA | TCTGGACCCTGGGCTCCGGAATGC |
| 373 | DUX4_gRNA | TCTGGTTTCAGAATCGAAGGGCCA |
| 374 | DUX4_gRNA | TCTGAAACCAAATCTGGACCCTGG |
| 375 | DUX4_gRNA | TCTGAAACCAGATCTGAATCCTGG |
| 376 | DUX4_gRNA | TCTGTCTCTCCCTCCGTTCCTCCC |
| 377 | DUX4_gRNA | TCTGGGCTCCCACGCATCGGCAGC |
| 378 | DUX4_gRNA | TCTGGTTTCAGAATTGAAGGGCCA |
| 379 | DUX4_gRNA | TCTGTTCTCATTACACACACAACA |
| 380 | DUX4_gRNA | TCTAGGCAAACCTGGATTAGAGTT |
| 381 | DUX4_gRNA | TCTAAACCTTGTATGGGCTTTGCC |
| 382 | DUX4_gRNA | TCTACGGCAGCTTTGACATATGTC |
| 383 | DUX4_gRNA | TCTAATCCAGGTTTGCCTAGACAG |
| 384 | DUX4_gRNA | TCTGGCTGAATGTCTCCCCCCACC |
| 385 | DUX4_gRNA | TCTACACTCTGTCTACGGCAGCTT |
| 386 | DUX4_gRNA | TCTGTCTACGGCAGCTTTGACATA |
| 387 | DUX4_gRNA | TCTAGTCTTTTCCTATGTGGGTTT |
| 388 | DUX4_gRNA | TCTACTATGGAGTTCTGAAACACA |
| 389 | DUX4_gRNA | TCTGTCTTTGCCCGCTTCCTGGCT |
| 390 | DUX4_gRNA | TCTAGGTTCAGTCTACTATGGAGT |
| 391 | DUX4_gRNA | TCTAGGCTTTGGCCTACAGGGGGC |
| 392 | DUX4_gRNA | TCTGCAGCCTGTAGCTCCTGGGGA |
| 393 | DUX4_gRNA | TCTATCACAGTGCCCCCATAGGCA |
| 394 | DUX4_gRNA | TCTGGCTTCATTTTGGGGGACGTG |
| 395 | DUX4_gRNA | TCTATAGGATCCACAGGGAGGGGG |
| 396 | DUX4_gRNA | TCTGACACATCTCTGAACTGATCA |
| 397 | DUX4_gRNA | TCTATGTTCTTCACTGCCTCATAC |
| 398 | DUX4_gRNA | TCTGGGCGATCAGTGCAGAGAGAA |
| 399 | DUX4_gRNA | TCTGTCTGTCTTTGCCCGCTTCCT |
| 400 | DUX4_gRNA | TCTGTCTGGCTTCATTTTGGGGGA |
| 401 | DUX4_gRNA | TCTGTGGACAGTTTCTCCTCATGG |
| 402 | DUX4_gRNA | TCTGTTGATAAAACTAGACCAAAA |
| 403 | DUX4_gRNA | TCTAAACACTACTCTGCTATTAGT |
| 404 | DUX4_gRNA | TCTGTGTTCAGTATTCCCATATGA |
| 405 | DUX4_gRNA | TCTACGGGGGCATTGTGACATATC |
| 406 | DUX4_gRNA | TCTGTGCAGAGATATGTCACAAAG |
| 407 | DUX4_gRNA | TCTGAAATTGTCATGCAGTGACTC |
| 408 | DUX4_gRNA | TCTAAGCCTCGTGGTTAGTGGGGA |
| 409 | DUX4_gRNA | TCTGTAGATCTCTGCCATTCATAA |
| 410 | DUX4_gRNA | TCTGCCTAAGCTTGAGTGAGTCAC |
| 411 | DUX4_gRNA | TCTGCCTCTCAAGAAATTCCTGCC |
| 412 | DUX4_gRNA | TCTGATGTAAGAAATGATGCTCAC |
| 413 | DUX4_gRNA | TCTGCCATTCATAAGTATTTGGAG |
| 414 | DUX4_gRNA | TCTGAACCTAGACAGGAGTTACAT |
| 415 | DUX4_gRNA | TCTAGTTTTATCAACAGAGCTAGT |
| 416 | DUX4_gRNA | TCTGTTTAGAATGCTACCTATTGC |
| 417 | DUX4_gRNA | TCTATCCAGAAGGCAATAGGTAGC |
| 418 | DUX4_gRNA | TCTATATCCAGCCTCATCTATTTC |
| 419 | DUX4_gRNA | TCTACTACATACCAGGTTCCAGAA |
| 420 | DUX4_gRNA | TCTGGAACCTGGTATGTAGTAGAA |
| 421 | DUX4_gRNA | TCTGGATAGAATCACAACTCTTTA |
| 422 | DUX4_gRNA | TCTAAACAGAGATCCTTTTTTTTT |
| 423 | DUX4_gRNA | TCTGCATCTCCCAGAGCCAGCCTG |
| 424 | DUX4_gRNA | TCTGGGAAGCTGACAATCCATCAG |
| 425 | DUX4_gRNA | TCTGGGAGATGCAGAAGGAGCACG |
| 426 | DUX4_gRNA | TCTACAGATTTGATTCTGATGTAA |
| 427 | DUX4_gRNA | TCTGTGTCATATATCACCAAATCT |
| 428 | DUX4_gRNA | TCTGAAACACATCTGCACTGATCA |
| 429 | DUX4_gRNA | TCTACAGGGGATATTGTGACATAT |
| 430 | DUX4_gRNA | TCTATTTCTGCTCCTCCTCCTTAT |
| 431 | DUX4_gRNA | TCTGCTCCTCCTCCTTATTTTCCT |
| 432 | DUX4_gRNA | TCTAGGTGATGTAACTCTTGTCCA |
| 433 | DUX4_gRNA | TCTGGTTGATCAGTAAAGAGATAT |
| 434 | DUX4_gRNA | TCTGCTATTAGTAGCTGTGTGACC |
| 435 | DUX4_gRNA | TCTGATCACCCAGGTGATGTAACT |
| 436 | DUX4_gRNA | TCTAGGTGATGTAACTCTTGTCTA |
| 437 | DUX4_gRNA | TCTGTAGGCAGAGCCTAGACAAGA |
| 438 | DUX4_gRNA | TCTACTGGGAGCATTGTGACATAT |
| 439 | DUX4_gRNA | TCTAGCCAGGAAGCGGGCAAAGAC |
| 440 | DUX4_gRNA | TCTGGGTGATCTTTGCAGAGATAT |
| 441 | DUX4_gRNA | TCTAGACAAGAGTTACATCTCCTG |
| 442 | DUX4_gRNA | TCTGACATATCTCTGCACTGATCA |
| 443 | DUX4_gRNA | TCTGGGTCATCAGTGCAGACATAT |
| 444 | DUX4_gRNA | TCTATACTCTGCCTGCAGGGACAT |
| 445 | DUX4_gRNA | TCTGCAATGATCACTCATGTGATG |
| 446 | DUX4_gRNA | TCTACCCTCTGCCTACAGGGGGCG |
| 447 | DUX4_gRNA | TCTGTGCCCTTGTTCTTCCGTGAA |
| 448 | DUX4_gRNA | TCTGCACTCATCACACAAAAGATG |
| 449 | DUX4_gRNA | TCTAAGCTCTGCCTACAGGGGCGT |
| 450 | DUX4_gRNA | TCTGCTTACTTGGGGGATTGTGAC |
| 451 | DUX4_gRNA | TCTGCCTGCAGGGACATTTTGAGA |
| 452 | DUX4_gRNA | TCTGTCTACTGGGAGCATTGTGAC |
| 453 | DUX4_gRNA | TCTAGGCTCTGCTTACTTGGGGGA |
| 454 | DUX4_gRNA | TCTAGGCTCCGCCCACAGGGGGCA |
| 455 | DUX4_gRNA | TCTACACGAGAATTTTAACATATC |
| 456 | DUX4_gRNA | TCTAGGCTCTGTCTACTGGGAGCA |
| 457 | DUX4_gRNA | TCTGTCTACACGAGAATTTTAACA |
| 458 | DUX4_gRNA | TCTGCCTACTGGGGCGTAGTGACA |
| 459 | DUX4_gRNA | TCTAGACTCGACCTACAGGGGCTT |
| 460 | DUX4_gRNA | TCTAGGCTCTGCTTACGGGGGTAT |
| 461 | DUX4_gRNA | TCTAGGCCCTGCCTACAAGGGAAT |
| 462 | DUX4_gRNA | TCTGCCTACAGGGGACATTGTGAC |
| 463 | DUX4_gRNA | TCTAGGTTCAGACTACAGGAGCGT |
| 464 | DUX4_gRNA | TCTGTACGGATCACCTGGGTTATG |
| 465 | DUX4_gRNA | TCTGCCTATGGGGGCATTGCAACA |
| 466 | DUX4_gRNA | TCTGCCTACAGAGGGCATTGTGGC |
| 467 | DUX4_gRNA | TCTGTCACAATGCCCCTGTAGGCA |
| 468 | DUX4_gRNA | TCTAGGTGATGTAACTCTTGCTTA |
| 469 | DUX4_gRNA | TCTGCTTACGGGGGTATTGTGACA |
| 470 | DUX4_gRNA | TCTGCACTGATCAGCCCAGGGAGG |
| 471 | DUX4_gRNA | TCTGCCTACATGGGCATTCTGACA |
| 472 | DUX4_gRNA | TCTGTGTATGGGGGCTTTCTGACA |
| 473 | DUX4_gRNA | TCTGCCTATGGGGGCACTGTGATA |
| 474 | DUX4_gRNA | TCTGCCTACTGGAGCATTGTGACA |
| 475 | DUX4_gRNA | TCTAAGCTGTGCCTAAAGGGGAAT |
| 476 | DUX4_gRNA | TCTAGGCTCTGTCTACACGAGAAT |
| 477 | DUX4_gRNA | TCTGCCTACATAGGCATTGTGACA |
| 478 | DUX4_gRNA | TCTGTCTACGGGGGCATTGTGACA |
| 479 | DUX4_gRNA | TCTGCCTACTGGGGGCATTGTTAC |
| 480 | DUX4_gRNA | TCTAGGCTCTGCCTACTGGCGGCA |
| 481 | DUX4_gRNA | TCTAAGACAAGCGTCCATCACCTG |
| 482 | DUX4_gRNA | TCTGCAAAGATCACCCAGATGATG |
| 483 | DUX4_gRNA | TCTGCCCTTATGACCCAGGTGATG |
| 484 | DUX4_gRNA | TCTATGCACTGATCTCTGAGGTGA |
| 485 | DUX4_gRNA | TCTGAGGTGATTCAACTCTTGTCT |
| 486 | DUX4_gRNA | TCTGCCTACTGGCGGCATTGTCAC |
| 487 | DUX4_gRNA | TCTAGGCTCTGCCTATGGGGGCAC |
| 488 | DUX4_gRNA | TCTGCACTGATAACCTAGGTGATG |
| 489 | DUX4_gRNA | TCTAGGCTCTGCCTACATAGGCAT |
| 490 | DUX4_gRNA | TCTAGGCAGAGTATAGAGAAGAGT |
| 491 | DUX4_gRNA | TCTAGGCTCTGCCTACAGAGGGCA |
| 492 | DUX4_gRNA | TCTGAACTAATCATCCAGGAGATG |
| 493 | DUX4_gRNA | TCTGCCTACAGAGGGCGTTGTGAC |
| 494 | DUX4_gRNA | TCTAGGCCCCACCTACAGGGGGTA |
| 495 | DUX4_gRNA | TCTACAGGGGGCTTTGTGATATAT |
| 496 | DUX4_gRNA | TCTGCCTACAGGGGGCGTTGTGAA |
| 497 | DUX4_gRNA | TCTGCCTACAGGAGGCATTGTGAC |
| 498 | DUX4_gRNA | TCTATATCTGCCTACTGGCGGCAT |
| 490 | DUX4_gRNA | TCTGCACTGATCACCCTGAGGAGG |
| 491 | DUX4_gRNA | TCTGCCTAAGGGGGCATTGTGACG |
| 492 | DUX4_gRNA | TCTAAGCTCTGCCTACAGGAGCTT |
| 493 | DUX4_gRNA | TCTAGGCTCTGCCTACACGGGAAT |
| 494 | DUX4_gRNA | TCTGCCTACAGGGGCATTGTGACG |
| 495 | DUX4_gRNA | TCTGCCTATGGGGGCATTGCGACA |
| 496 | DUX4_gRNA | TCTACGCTCTGCCTATGGGGGCAT |
| 497 | DUX4_gRNA | TCTGCCTACGGGGCATAGTGACAT |
| 498 | DUX4_gRNA | TCTAGGCTCTGTGTATGGGGGCTT |
| 499 | DUX4_gRNA | TCTGCCTATGGGGGCTTTGTGACA |
| 500 | DUX4_gRNA | TCTGTGTATGGGGGCTTTGTGACA |
| 501 | DUX4_gRNA | TCTAGGCTCTGCCAAAAGGGGGCA |
| 502 | DUX4_gRNA | TCTGCTTACAGGGTGCTTTGTGAC |
| 503 | DUX4_gRNA | TCTACGCTCTGCCTACAGGAGGCT |
| 504 | DUX4_gRNA | TCTAGTCTCTGCCTACAGAGGGCG |
| 505 | DUX4_gRNA | TCTGGCTACACAGCATTGTGACAT |
| 506 | DUX4_gRNA | TCTAGGCTCTGGCTACACAGCATT |
| 507 | DUX4_gRNA | TCTGCCTATAGGGGGCCTTGTGAC |
| 508 | DUX4_gRNA | TCTAGGCTCTGCCTACTGGAGCAT |
| 509 | DUX4_gRNA | TCTGCCTAGAGGGGGATTTGTGAC |
| 510 | DUX4_gRNA | TCTGCCTACAGGGGCATTGTGATA |
| 511 | DUX4_gRNA | TCTGTAGGCAGAGACTAGAAAAGA |
| 512 | DUX4_gRNA | TCTGCCTACAGGGGCATTGCGATG |
| 513 | DUX4_gRNA | TCTGCCTGCAGGGGCATTGTGAAA |
| 514 | DUX4_gRNA | TCTGCCTACATGGGCATTGTGACA |
| 515 | DUX4_gRNA | TCTGCCTACAGGGGGTATTGTGAA |
| 516 | DUX4_gRNA | TCTAGGCTCTGCCTATGGGGGCTT |
| 517 | DUX4_gRNA | TCTGCCAAAAGGGGGCATTGTGAC |
| 518 | DUX4_gRNA | TCTACAGGGATTTTTGTGACATAT |
| 519 | DUX4_gRNA | TCTATACTCTGCCTAGAGGGGGAT |
| 520 | DUX4_gRNA | TCTATGGGGGCATTGTGTCAAATA |
| 521 | DUX4_gRNA | TCTGAACTGATCAACAAAGTGATG |
| 522 | DUX4_gRNA | TCTAGGCTCTGCCTACAGGGGGCA |
| 523 | DUX4_gRNA | TCTGCCTACTGGAGACATTGTGAC |
| 524 | DUX4_gRNA | TCTGCCTACTGGCGGCATTGTGGC |
| 525 | DUX4_gRNA | TCTGCTTACAGGGGGCATTGTGAC |
| 526 | DUX4_gRNA | TCTGCCTATAGGGGCATTGTGACA |
| 527 | DUX4_gRNA | TCTAGGATCTGCCTAAAGGGACTT |
| 528 | DUX4_gRNA | TCTGTCTACAGGGATTTTTGTGAC |
| 529 | DUX4_gRNA | TCTAGGCTCTGCCTACTGGGGGCA |
| 530 | DUX4_gRNA | TCTGCACAGATCATCTAGGTGATG |
| 531 | DUX4_gRNA | TCTACAGGGGGCTTTGTGACATAT |
| 532 | DUX4_gRNA | TCTAGGCTCTGTCTACGGGGGCAT |
| 533 | DUX4_gRNA | TCTAGGCTCTGTCTACAGGGATTT |
| 534 | DUX4_gRNA | TCTGTGCAGAGCTATGTCAAAACG |
| 535 | DUX4_gRNA | TCTGCACTGATGACCCAGATGATG |
| 536 | DUX4_gRNA | TCTGCTTACAGGGGGTATTGTGAC |
| 537 | DUX4_gRNA | TCTGCCTACACGGGAATTCTCACA |
| 538 | DUX4_gRNA | TCTGCCTACAGGGGCGTTTTGACA |
| 539 | DUX4_gRNA | TCTGCACTAATCATCCAGGTGATG |
| 540 | DUX4_gRNA | TCTATGCTCTGCCTACAGGGGGCA |
| 541 | DUX4_gRNA | TCTAGGCACTGCCTACAGGGGACA |
| 542 | DUX4_gRNA | TCTGTGCTCTGCCTACAGGGGACA |
| 543 | DUX4_gRNA | TCTGTCTATGGGGGCATTGTGTCA |
| 544 | DUX4_gRNA | TCTGCACTGTTAACCGAGGTGATG |
| 545 | DUX4_gRNA | TCTGCCTACAGGGGGCATTGTGAA |
| 546 | DUX4_gRNA | TCTGCAGTGATCACGCAGGTGATG |
| 547 | DUX4_gRNA | TCTGCCTAAAGGGACTTTGTGACA |
| 548 | DUX4_gRNA | TCTGCCTACAGGAGGCTTTATGAC |
| 549 | DUX4_gRNA | TCTGCCTATGGGGGCATAGTGACA |
| 550 | DUX4_gRNA | TCTGTAGGCAAAGCCCATACAAGG |
| 551 | DUX4_gRNA | TCTGCACTGATCACCTAGGTCATA |
| 552 | DUX4_gRNA | TCTGCCTACAGGGGGCTTGTGACA |
| 553 | DUX4_gRNA | TCTAGGCTCTGCCTACTGGAGACA |
| 554 | DUX4_gRNA | TCTGCCTACAGGGGGCATTGTGAC |
| 555 | DUX4_gRNA | TCTGCACTGATCCCCAAGGTGATG |
| 556 | DUX4_gRNA | TCTGCACTGATCAACTAGGTGATG |
| 557 | DUX4_gRNA | TCTAGGCTCTGCTTACAGGGGGTA |
| 558 | DUX4_gRNA | TCTGCTTAAAGGGGCCTTGTCACA |
| 559 | DUX4_gRNA | TCTGAACTGATCAACCAAGTGATG |
| 560 | DUX4_gRNA | TCTGCCTAAAGGGGCATTGTGACA |
| 561 | DUX4_gRNA | TCTGCCTACTGGGGACATTGTGAC |
| 562 | DUX4_gRNA | TCTGCCTACAGGGGCGTTTTCACA |
| 563 | DUX4_gRNA | TCTGCACTGATCCCGAGGTGATCC |
| 564 | DUX4_gRNA | TCTGCCTACAGTGGCATTGTGACA |
| 565 | DUX4_gRNA | TCTGCAATGATCACCCAGGTGATG |
| 566 | DUX4_gRNA | TCTGCCCTGATCACCCAGGTGATG |
| 567 | DUX4_gRNA | TCTGCCTACAGGGGCATTGCAATG |
| 568 | DUX4_gRNA | TCTAGGCTGTGCCCACAGGGGGAT |
| 569 | DUX4_gRNA | TCTAGGCTCTGCCTACAGGGGCTT |
| 570 | DUX4_gRNA | TCTAGGCTCTGCTTAAAGGGGCCT |
| 571 | DUX4_gRNA | TCTGCACTGATCACTCAGGTGATG |
| 572 | DUX4_gRNA | TCTGCCTATGGGGGCATTGTGACA |
| 573 | DUX4_gRNA | TCTAAGCTCTGCCTAAAGGGGCAT |
| 574 | DUX4_gRNA | TCTGTCACAATGCCCCTTTAGGCA |
| 575 | DUX4_gRNA | TCTAGGCTCTGCCTAAGGGGGCAT |
| 576 | DUX4_gRNA | TCTGCACTGATAACCCAGGTGATG |
| 577 | DUX4_gRNA | TCTGCACTGATCATCTAGGTGATG |
| 578 | DUX4_gRNA | TCTGCCTACAGGGGAATTGTGAGA |
| 579 | DUX4_gRNA | TCTAAGCTCTGCCTACAGGGGCAT |
| 580 | DUX4_gRNA | TCTGCCTACAGGGTGCTTTGTGAC |
| 581 | DUX4_gRNA | TCTAGTCTAAGCTCTGCCTAAAGG |
| 582 | DUX4_gRNA | TCTGCACTGATCACCGAAGTTATG |
| 583 | DUX4_gRNA | TCTGCACTGATCTCCCAGGTGCTG |
| 584 | DUX4_gRNA | TCTGGGATTTGTCTACAGGGGGCT |
| 585 | DUX4_gRNA | TCTGCCTACAGGGGCTTTGTGACA |
| 586 | DUX4_gRNA | TCTGCCTACAGGAGCTTTGTGACA |
| 587 | DUX4_gRNA | TCTGCACTGATCACCCAGGAGACG |
| 588 | DUX4_gRNA | TCTGCCTACAGGGGCATTGTGACA |
| 589 | DUX4_gRNA | TCTAGGCTCTGCCTACAGGGGGCT |
| 590 | DUX4_gRNA | TCTGCACTGATCACCTAGGTCATG |
| 591 | DUX4_gRNA | TCTGCACTGATCACTTAGGTGATG |
| 592 | DUX4_gRNA | TCTAGGATCTGCCTACAGGGGGTA |
| 593 | DUX4_gRNA | TCTGCACTGATCGCCCAGATGATG |
| 594 | DUX4_gRNA | TCTAGGATCTGCCTACAGGGTGCT |
| 595 | DUX4_gRNA | TCTGCACTGATCACCCAAGTAATG |
| 596 | DUX4_gRNA | TCTAGGCTCTGCCTACAGTGGCAT |
| 597 | DUX4_gRNA | TCTAGGCTCTGCCTACAGGGGCGT |
| 598 | DUX4_gRNA | TCTGGAGTAGCTGGGACTACAGTC |
| 599 | DUX4_gRNA | TCTGGGATCTGCTTACAGGGGGCA |
| 600 | DUX4_gRNA | TCTAGGATCTGCTTACAGGGTGCT |
| 601 | DUX4_gRNA | TCTGCACTGATCACCTTGGTGATG |
| 602 | DUX4_gRNA | TCTGCACTGATCACCCAGGTGACT |
| 602 | DUX4_gRNA | TCTGGGCTCTGCCTACAGGGGCAT |
| 604 | DUX4_gRNA | TCTAGGCTCTGCCTACAGGGGCAT |
| 605 | DUX4_gRNA | TCTGTTGCCCGGGCTGGAATGCAG |
| 606 | DUX4_gRNA | TCTGCACTGATCACCTAGGTGATG |
| 607 | DUX4_gRNA | TCTGTACTGATCACCCAGGTGATG |
| 608 | DUX4_gRNA | TCTGGGCTTTGTCTACAGGGGGCT |
| 609 | DUX4_gRNA | TCTACACTGATCACCTAAGTGATG |
| 610 | DUX4_gRNA | TCTACACTGATCACACAGGTGATG |
| 611 | DUX4_gRNA | TCTGCACTGATCACCTAAGTGATG |
| 612 | DUX4_gRNA | TCTGCACTGATCACCCAGGTGAAG |
| 613 | DUX4_gRNA | TCTGCACAGATCACCCAGGTGATG |
| 614 | DUX4_gRNA | TCTGCACTGATCACCGAGGTGATG |
| 615 | DUX4_gRNA | TCTGCACTGATCACCCAGGTGGTG |
| 617 | DUX4_gRNA | TCTGCACTGATCACCCAGGGGATG |
| 618 | DUX4_gRNA | TCTGCACTGATCACCCAGGTAATG |
| 619 | DUX4_gRNA | TCTGCACTGATCACCCAGGTGATA |
| 620 | DUX4_gRNA | TCTGCACTGATCAACCAGGTGATG |
| 621 | DUX4_gRNA | TCTGCACTGATCACCCAGGTCATG |
| 622 | DUX4_gRNA | TCTGCACTGATCACCCAGGCGATG |
| 623 | DUX4_gRNA | TCTGCACTGATCACCCAAGTGATG |
| 624 | DUX4_gRNA | TCTACTAAAAATACAAAAAAATTA |
| 625 | DUX4_gRNA | TCTGCACTGATCACCCAGGTGATG |
| 626 | DUX4_gRNA | TCTACACTGATCACCCAGGTGATG |
| 627 | DUX4_gRNA | TCTACCTCAGATGAGATATTGCTT |
| 628 | DUX4_gRNA | TCTGTCTCGGAATGAAATGAATTC |
| 629 | DUX4_gRNA | TCTATAGTTCAAACAAAGATGAGG |
| 630 | DUX4_gRNA | TCTGAGGTAGAATGTTTCTAGTGG |
| 631 | DUX4_gRNA | TCTGCTGTATCTATAGTTCAAACA |
| 632 | DUX4_gRNA | TCTATACTGCTTGACCCAAGCTTT |
| 633 | DUX4_gRNA | TCTAGCGTGTATTTATTTTGCAGC |
| 634 | DUX4_gRNA | TCTGAAACGTGGTATCTGGAGAGG |
| 635 | DUX4_gRNA | TCTACCTTTTGCTATCAAAAGCTT |
| 636 | DUX4_gRNA | TCTAGGAACAGTAAGAGGACCTTG |
| 637 | DUX4_gRNA | TCTGGAGAATTCATTTCATTCCGA |
| 638 | DUX4_gRNA | TCTGCTTATTACCCACTCTGTAAT |
| 639 | DUX4_gRNA | TCTGAGGGAGAAAAACTAATCTTT |
| 640 | DUX4_gRNA | TCTGTTACTGTGTGCAAGGTGAAG |
| 641 | DUX4_gRNA | TCTAGTGGTTGTGTTCTGAGGGAG |
| 642 | DUX4_gRNA | TCTGCTTTTGGTTCATGAAATTTT |
| 643 | DUX4_gRNA | TCTGGAGAGGTGAGATGGACAAAG |
| 644 | DUX4_gRNA | TCTGGGTCACAGCTATATTAGAGC |
| 645 | DUX4_gRNA | TCTGTTTCTAGCGTGTATTTATTT |
| 646 | DUX4_gRNA | TCTAATATAGCTGTGACCCAGATG |
| 647 | DUX4_gRNA | TCTGCTTCACTTCAATAACAGCCT |
| 648 | DUX4_gRNA | TCTGGAACAGCTATGTACTTTCTT |
| 649 | DUX4_gRNA | TCTGAAATCCTTTTATGCCTGGCC |
| 650 | DUX4_gRNA | TCTGTAATGTGGAAACAAATTATT |
| 651 | DUX4_gRNA | TCTGACACAGTCTGCGTTTGTAAG |
| 652 | DUX4_gRNA | TCTGGGATTCTTCTGCTGGAAAAA |
| 653 | DUX4_gRNA | TCTAAGAAGTCTGGGATTCTTCTG |
| 654 | DUX4_gRNA | TCTACCATTTAAAACAAGAACTCT |
| 655 | DUX4_gRNA | TCTGCTGGAAAAATAAGTTTGTTG |
| 656 | DUX4_gRNA | TCTGTGAAATCCTCATGTTTTCTT |
| 657 | DUX4_gRNA | TCTAAAGTATATTACTCTGCTTTT |
| 658 | DUX4_gRNA | TCTAACCTTCAAAAACCAAACCTG |
| 659 | DUX4_gRNA | TCTGGCTACTTTCATGGTATAATG |
| 660 | DUX4_gRNA | TCTATCTGTTTACTATCTGTCTTT |
| 661 | DUX4_gRNA | TCTGTTTACTATCTGTCTTTTCTA |
| 662 | DUX4_gRNA | TCTAAAACAAGGTGTGGCAAACTA |
| 663 | DUX4_gRNA | TCTGTTTTCTGGAACAGCTATGTA |
| 664 | DUX4_gRNA | TCTGTCTTTTCTACCTTTTGCTAT |
| 665 | DUX4_gRNA | TCTAGTTTTGCCTCATCTTTGTTT |
| 666 | DUX4_gRNA | TCTGCGTTTGTAAGTAAAGTTGTA |
| 667 | DUX4_gRNA | TCTGCAAAGGGCTAAATGTTAAAT |
| 668 | DUX4_gRNA | TCTATCTATCTGTTTACTATCTGT |
| 669 | DUX4_gRNA | TCTGTGGGGTTTTTGTTGTTGTTG |
| 670 | DUX4_gRNA | TCTACCTCCTATCATCTATCTATC |
| 671 | DUX4_gRNA | TCTATCTACCTCCTATCATCTATC |
| 672 | DUX4_gRNA | TCTATCTATCTACCTCCTATCATC |
| 673 | DUX4_gRNA | TCTGTGGCCAGGCGTGGTGGCTCA |
| 674 | DUX4_gRNA | TCTGTTTTTTGTTTGTTTGTTGTT |
| 675 | DUX4_gRNA | TCTGTAATCCCAGCACTTTGGGAT |
| 676 | DUX4_gRNA | TCGAACTCACAGGCAAAATCCTCC |
| 677 | DUX4_gRNA | TCGGTATCCCCCTTTACTGAGCCA |
| 678 | DUX4_gRNA | TCGAATGCACTTTAAGATTCTGGG |
| 679 | DUX4_gRNA | TCGGGTCTTCACCCGCGCGGTTCA |
| 680 | DUX4_gRNA | TCGGGTGGTTCGGGGCAGGGCCGT |
| 681 | DUX4_gRNA | TCGGGTTTTCACCCGCGCGGTTCA |
| 682 | DUX4_gRNA | TCGGCCTCGCGCCGCGTTGCAGGG |
| 683 | DUX4_gRNA | TCGGGTTGCCGTCGGGTCTTCACC |
| 684 | DUX4_gRNA | TCGGCATGGCCAGCCTTTCGGGGG |
| 685 | DUX4_gRNA | TCGGCAGCAGGGAGAAACCAGCCT |
| 686 | DUX4_gRNA | TCGGGTGGTTCGGGGCAGGGCGGT |
| 687 | DUX4_gRNA | TCGGCCTCCGGGAGTAGCGGGACC |
| 688 | DUX4_gRNA | TCGGAAGAGGCCGCCTCGCTGGAA |
| 689 | DUX4_gRNA | TCGAGGCCTGGGGCCGGCCGGCGG |
| 690 | DUX4_gRNA | TCGGGTTGCCGTCGGGTTTTCACC |
| 691 | DUX4_gRNA | TCGGGGGCCGGAGAGACGTGAGCA |
| 692 | DUX4_gRNA | TCGGGGGCCGGCTCTCCGGACCTC |
| 692 | DUX4_gRNA | TCGGTGGCCTCCGCACCCGGGCAA |
| 694 | DUX4_gRNA | TCGACGCCCTGGGTCCCTTCCGGG |
| 695 | DUX4_gRNA | TCGGACAGCACCCTCCCCGCGGAA |
| 696 | DUX4_gRNA | TCGGGAGGGCCATCGCGGTGAGCC |
| 697 | DUX4_gRNA | TCGGGGCAGGGCCGTGGCCTCTCT |
| 698 | DUX4_gRNA | TCGGGGTCCAAACGAGTCTCCGTC |
| 699 | DUX4_gRNA | TCGGCCCTGGCCCGGGAGACGCGG |
| 700 | DUX4_gRNA | TCGGCATTCCGGAGCCCAGGGTCC |
| 701 | DUX4_gRNA | TCGGAGGAGCAGGGCGGTCTGGGA |
| 702 | DUX4_gRNA | TCGGGGCAGGGCGGTGGCCTCTCT |
| 703 | DUX4_gRNA | TCGAAGGGCCAGGCACCCGGGACA |
| 704 | DUX4_gRNA | TCGATTCTGAAACCAGATCTGAAT |
| 705 | DUX4_gRNA | TCGGAAGGTGGGGGGAGACATTCA |
| 706 | DUX4_gRNA | TCGAGTCTAGACAAGAGTTACATC |
| 707 | DUX4_gRNA | TCGGGTTCAGGTTAAGAGTTAGGG |
| 708 | DUX4_gRNA | TCGGTGATCAGTGCAGAGATACGT |
| 709 | DUX4_gRNA | TCGACAAATCTCTGCACTGATCAC |
| 710 | DUX4_gRNA | TCGAAATTCCCTGTAGGCAGTGCT |
| 711 | DUX4_gRNA | TCGGTGATCAGTGCAGATGTGTTT |
| 712 | DUX4_gRNA | TCGACCTACAGGGGCTTTGTGACA |
| 713 | DUX4_gRNA | TCGGTGATCAATGCAGCGATATGT |
| 714 | DUX4_gRNA | TCGGTTAACAGTGCAGAGATATGT |
| 715 | DUX4_gRNA | TCGGGATCAGTGCAGAGATATGTC |
| 716 | DUX4_gRNA | TCGGAATGAAATGAATTCTCCAGA |
| 717 | DUX4_gRNA | TCGGCTAGCCTCGGCATCCCAAAG |
| 718 | DUX4_gRNA | TCGGCATCCCAAAGTGCTGGGATT |

It shall be understood that different aspects of the invention can be appreciated individually, collectively, or in combination with each other. Various aspects of the invention described herein may be applied to any of the particular applications disclosed herein. The compositions of matter disclosed herein in the composition section of the present disclosure may be utilized in the method section including methods of use and production disclosed herein, or vice versa.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense.

## Claims

1. A system for regulating aberrant expression of a target gene in a muscle cell, comprising:
a heterologous polypeptide comprising a deactivated nuclease, wherein the deactivated nuclease has a length that is less than or equal to 750 amino acids and is a modified variant of Un1Cas12f1; and
a guide nucleic acid molecule configured to form a complex with the heterologous polypeptide,
wherein the guide nucleic acid molecule exhibits specific binding to a target polynucleotide sequence at or adjacent to, a D4Z4 repeat array in the muscle cell, wherein, upon formation of the complex, the complex is capable of binding the target polynucleotide sequence, to effect modification of an expression level and/or a methylation level of the target gene in the muscle cell,
wherein the target gene is within the D4Z4 repeat array.

2. The system of claim 1, wherein upon formation of the complex, the modified expression level and/or methylation level of the target gene in the muscle cell is sustained for at least about 2 days.

3. The system of claim 2, wherein
(i) the modified expression level and/or methylation level of the target gene is sustained for at least about 3 days, 4 days, 5 days, 6 days, 1 week, 2 weeks, 2 weeks, 4 weeks, or 2 months, or
(ii) the modified expression level and/or methylation level of the target gene is sustained for at least about 17 days, or
(iii) the modified expression level and/or methylation level of the target gene is sustained for at least about 18 days.

4. The system of claim 1, wherein
(i) the muscle cell is in a subject having or is suspected of having facioscapulohumeral muscular dystrophy (FSHD), or
(ii) the target gene is Dux4.

5. The system of claim 1, wherein
the nuclease comprises an amino acid sequence that is at least 90% identical to the polypeptide sequence of SEQ ID NO: 44.

6. The system of claim 1, wherein the heterologous polypeptide further comprises a transcriptional regulator.

7. The system of claim 6, wherein the transcriptional regulator comprises at least one methyltransferases.

8. The system of claim 7, wherein
(i) the transcriptional regulator comprises at least one DNA Methyltransferases (DNMT), optionally wherein the transcriptional regulator comprises DNMT-A or DNMT-L, or
(ii) the transcriptional regulator comprises (i) DNMT-A or DNMT-L and (ii) KRAB or a variant of KRAB, or
(iii) the transcriptional regulator comprises (i) DNMT-L and (ii) KRAB or a variant of KRAB, or
(iv) the transcriptional regulator comprises DNMT-A, DNMT-L, and KRAB or a variant of KRAB.

9. The system of claim 6, wherein
(i) the transcriptional regulator comprises DNMT-L or KRAB or variant of KRAB, or
(ii) the transcriptional regulator comprises KRAB or a variant of KRAB, or
(iii) the transcriptional regulator comprises a plurality of different transcriptional regulators.

10. The system of claim 1, wherein
(i) the modification of the expression level and/or the methylation level of the target gene effects downregulation of a downstream gene of the target gene, wherein the downstream gene comprises one or more members selected from the group consisting of ZSCAN4, LEUTX, MBD3L2, TRIM48, and TRIM43, or
(ii) the modification of the expression level and/or the methylation level of the target gene effects downregulation of an apoptosis marker in the muscle cell, optionally wherein the apoptosis marker comprises Caspase 3, or
(iii) the complex effects the modification of the expression level of the target gene in the muscle gene, optionally wherein the modification of the expression level results in downregulation of the target gene, or
(iv) the complex effects the modification of the methylation level of the target gene in the muscle gene, optionally wherein the modification of the methylation level results in downregulation of the target gene.

11. A composition comprising the system of any one of the preceding claims.

12. A viral vector comprising the system of any one of claims 1-10, optionally wherein the viral vector comprises an adeno-associated virus (AAVs), a retrovirus, a lentivirus, a poxvirus, or an adenovirus.

13. The viral vector of claim 12, wherein the viral vector comprises an adeno-associated virus (AAVs), a retrovirus, a lentivirus, a poxvirus, or an adenovirus, optionally wherein the AAV comprises a AAV serotype RH74 AAV.

14. An *in vitro* or *ex vivo* method for regulating aberrant expression of a target gene in a muscle cell, comprising:
(a) contacting the muscle cell with the complex of the heterologous polypeptide and the guide nucleic acid molecule of the system of any one of claims 1-10; and
(b) upon the contacting, binding the target gene with the complex to effect modification of the expression level and/or the methylation level of the target gene in the muscle cell.

15. The complex of the heterologous polypeptide and the guide nucleic acid molecule of the system of any one of claims 1-10 for use in a method for regulating aberrant expression of a target gene in a muscle cell, comprising:
(a) contacting the muscle cell with the complex of the heterologous polypeptide and the guide nucleic acid molecule of the system; and
(b) upon the contacting, binding the target gene with the complex to effect modification of the expression level and/or the methylation level of the target gene in the muscle cell,
optionally wherein the contacting comprises injecting a composition comprising the complex to a subject in need thereof, wherein the subject has or is suspected of having facioscapulohumeral muscular dystrophy (FSHD).

## Patentansprüche

1. System zum Regulieren von abnormaler Expression eines Ziel-Gens in einer Muskelzelle, das Folgendes umfasst:
ein heterologes Polypeptid, das eine deaktivierte Nuklease umfasst, wobei die deaktivierte Nuklease eine Länge aufweist, die kleiner oder gleich 750 Aminosäuren ist, und eine modifizierte Variante von Un1Cas12f1 ist, und
ein Nukleinsäure-Leitmolekül, das dazu ausgelegt ist, einen Komplex mit dem heterologen Polypeptid zu bilden;
wobei das Nukleinsäure-Leitmolekül in der Muskelzelle an einem D4Z4-Wieder-holungs-Array oder benachbart dazu eine spezifische Bindung mit einer Ziel-Polynukleotid-Sequenz eingeht, wobei nach der Bildung des Komplexes der Komplex dazu in der Lage ist, die Ziel-Polynukleotid-Sequenz zu binden, eine Modifikation des Expressions-Ausmaßes und/oder des Methylierungs-Ausmaßes des Ziel-Gens in der Muskelzelle zu bewirken;
wobei das Ziel-Gen innerhalb des D4Z4-Wiederholungs-Arrays vorliegt.

2. System nach Anspruch 1, wobei das modifizierte Expressions-Ausmaß und/oder Methylierungs-Ausmaß des Ziel-Gens in der Muskelzelle nach der Bildung des Komplexes zumindest etwa 2 Tage lang aufrechterhalten wird.

3. System nach Anspruch 2, wobei:
(i) das modifizierte Expressions-Ausmaß und/oder Methylierungs-Ausmaß des Ziel-Gens zumindest etwa 3 Tage, 4 Tage, 5 Tage, 6 Tage, 1 Woche, 2 Wochen, 3 Wochen, 4 Wochen oder 2 Monate lang aufrechterhalten wird oder
(ii) das modifizierte Expressions-Ausmaß und/oder Methylierungs-Ausmaß des Ziel-Gens zumindest etwa 17 Tage lang aufrechterhalten wird oder
(iii) das modifizierte Expressions-Ausmaß und/oder Methylierungs-Ausmaß des Ziel-Gens zumindest etwa 18 Tage lang aufrechterhalten wird.

4. System nach Anspruch 1, wobei:
(i) die Muskelzelle in einem Individuum vorliegt, das fazioskapulohumerale muskuläre Dystrophie (FSHD) aufweist oder bei dem diese vermutet wird oder
(ii) das Ziel-Gen Dux4 ist.

5. System nach Anspruch 1, wobei:
die Nuklease eine Aminosäure-Sequenz umfasst, die zumindest zu 90 % identisch mit der Polypeptid-Sequenz von SEQ ID NO: 44 ist.

6. System nach Anspruch 1, wobei das heterologe Polypeptid weiters einen Transkriptions-Regler umfasst.

7. System nach Anspruch 6, wobei der Transkriptions-Regler zumindest eine Methyltransferase umfasst.

8. System nach Anspruch 7, wobei:
(i) der Transkriptions-Regler zumindest eine DNA-Methyltransferase (DNMT) umfasst, wobei der Transkriptions-Regler gegebenenfalls DNMT-A oder DNMT-L umfasst, oder
(ii) der Transkriptions-Regler (i) DNMT-A oder DNMT-L und (ii) KRAB oder eine Variante von KRAB umfasst oder
(iii) der Transkriptions-Regler (i) DNMT-L und (ii) KRAB oder eine Variante von KRAB umfasst oder
(iv) der Transkriptions-Regler DNMT-A, DNMT-L und KRAB oder eine Variante von KRAB umfasst.

9. System nach Anspruch 6, wobei:
(i) der Transkriptions-Regler DNMT-L oder KRAB oder eine Variante von KRAB umfasst oder
(ii) der Transkriptions-Regler KRAB oder eine Variante von KRAB umfasst oder
(iii) der Transkriptions-Regler eine Vielzahl verschiedener Transkriptions-Regler umfasst.

10. System nach Anspruch 1, wobei:
(i) die Modifikation des Expressions-Ausmaßes und/oder des Methylierungs-Ausmaßes des Ziel-Gens die Herunterregulierung eines Stromab-Gens des Ziel-Gens bewirkt, wobei das Stromab-Gen ein oder mehrere Elemente umfasst, die aus der aus ZSCAN4, LEUTX, MBD3L2, TRIM48 und TRIM43 bestehenden Gruppe ausgewählt sind, oder
(ii) die Modifikation des Expressions-Ausmaßes und/oder des Methylierungs-Ausmaßes des Ziel-Gens die Herunterregulierung eines Apoptose-Markers in der Muskelzelle bewirkt, wobei der Apoptose-Marker gegebenenfalls Caspase 3 umfasst, oder
(iii) der Komplex die Modifikation des Expressions-Ausmaßes des Ziel-Gens in dem Muskelgen bewirkt, wobei die Modifikation des Expressions-Ausmaßes zur Herunterregulierung des Ziel-Gens führt, oder
(iv) der Komplex die Modifikation des Methylierungs-Ausmaßes des Ziel-Gens in dem Muskelgen bewirkt, wobei die Modifikation des Methylierungs-Ausmaßes zur Herunterregulierung des Ziel-Gens führt.

11. Zusammensetzung, die ein System nach einem der vorangegangenen Ansprüche umfasst.

12. Virusvektor, der ein System nach einem der Ansprüche 1 bis 10, wobei der Virusvektor gegebenenfalls ein adenoassoziiertes Virus (AAVs), ein Retrovirus, ein Lentivirus, ein Pockenvirus oder ein Adenovirus umfasst.

13. Virusvektor nach Anspruch 12, wobei der Virusvektor ein adenoassoziiertes Virus (AAV), ein Retrovirus, ein Lentivirus, ein Pockenvirus oder ein Adenovirus umfasst, wobei das AAV einen AAV-Serotyp RH74-AAV umfasst.

14. In-vitro- oder Ex-vivo-Verfahren zum Regulieren von abnomaler Expression eines Ziel-Gens in einer Muskelzelle, das Folgendes umfasst:
(a) das In-Kontakt-Bringen der Muskelzelle mit dem Komplex aus dem heterologen Polypeptid und dem Nukleinsäure-Leitmolekül eines Systems nach einem der Ansprüche 1 bis 10; und
(b) das Binden des Ziel-Gens an den Komplex nach dem In-Kontakt-Bringen, um eine Modifikation des Expressions-Ausmaßes und/oder des Methylierungs-Ausmaßes des Ziel-Gens in der Muskelzelle zu bewirken.

15. Komplex aus dem heterologen Polypeptid und dem Nukleinsäure-Leitmolekül eines Systems nach einem der Ansprüche 1 bis 10 zur Verwendung in einem Verfahren zum Regulieren von abnomaler Expression eines Ziel-Gens in einer Muskelzelle, das Folgendes umfasst:
(a) das In-Kontakt-Bringen der Muskelzelle mit dem Komplex aus dem heterologen Polypeptid und dem Nukleinsäure-Leitmolekül des Systems und
(b) das Binden des Ziel-Gens an den Komplex nach dem In-Kontakt-Bringen, um eine Modifikation des Expressions-Ausmaßes und/oder des Methylierungs-Ausmaßes des Ziel-Gens in der Muskelzelle zu bewirken;
wobei das Kontaktieren gegebenenfalls das Injizieren einer Zusammensetzung, die den Komplex umfasst, einem bedürftigen Individuum umfasst, wobei das Individuum an faziokapulohumeraler muskulärer Dystrophie (FSHD) leidet oder diese bei ihm vermutet wird.

## Revendications

1. Système de régulation d'une expression aberrante d'un gène cible dans une cellule musculaire, comprenant :
un polypeptide hétérologue comprenant une nucléase désactivée, dans lequel la nucléase désactivée présente une longueur inférieure ou égale à 750 acides aminés et est une variante modifiée de Un1Cas12f1 ; et
une molécule d'acide nucléique guide configurée pour former un complexe avec le polypeptide hétérologue,
dans lequel la molécule d'acide nucléique guide présente une liaison spécifique à une séquence polynucléotidique cible au niveau d'un réseau de répétitions D4Z4 dans la cellule musculaire ou adjacente à celui-ci, dans lequel, lors de la formation du complexe, le complexe est capable de se lier à la séquence polynucléotidique cible, pour effectuer une modification d'un niveau d'expression et/ou d'un niveau de méthylation du gène cible dans la cellule musculaire,
dans lequel le gène cible est à l'intérieur du réseau de répétitions D4Z4.

2. Système selon la revendication 1, dans lequel lors de la formation du complexe, le niveau d'expression et/ou le niveau de méthylation modifié du gène cible dans la cellule musculaire est maintenu pendant au moins environ 2 jours.

3. Système selon la revendication 2, dans lequel :
(i) le niveau d'expression et/ou le niveau de méthylation modifié du gène cible est maintenu pendant au moins environ 3 jours, 4 jours, 5 jours, 6 jours, 1 semaine, 2 semaines, 2 semaines, 4 semaines ou 2 mois, ou
(ii) le niveau d'expression et/ou le niveau de méthylation modifié du gène cible est maintenu pendant au moins environ 17 jours, ou
(iii) le niveau d'expression et/ou le niveau de méthylation modifié du gène cible est maintenu pendant au moins environ 18 jours.

4. Système selon la revendication 1, dans lequel :
(i) la cellule musculaire est chez un sujet présentant ou étant suspecté de présenter une dystrophie musculaire facio-scapulo-humérale (FSHD), ou
(ii) le gène cible est Dux4.

5. Système selon la revendication 1, dans lequel :
la nucléase comprend une séquence d'acides aminés qui est au moins 90 % identique à la séquence polypeptidique de SEQ ID NO :44.

6. Système selon la revendication 1, dans lequel le polypeptide hétérologue comprend en outre un régulateur transcriptionnel.

7. Système selon la revendication 6, dans lequel le régulateur transcriptionnel comprend au moins une méthyltransférase.

8. Système selon la revendication 7, dans lequel :
(i) le régulateur transcriptionnel comprend au moins une ADN méthyltransférase (DNMT), facultativement dans lequel le régulateur transcriptionnel comprend DNMT-A ou DNMT-L, ou
(ii) le régulateur transcriptionnel comprend (i) DNMT-A ou DNMT-L et (ii) KRAB ou un variant de KRAB, ou
(iii) le régulateur transcriptionnel comprend (i) DNMT-L et (ii) KRAB ou un variant de KRAB, ou
(iv) le régulateur transcriptionnel comprend DNMT-A, DNMT-L et KRAB ou un variant de KRAB.

9. Système selon la revendication 6, dans lequel :
(i) le régulateur transcriptionnel comprend DNMT-L ou KRAB ou un variant de KRAB, ou
(ii) le régulateur transcriptionnel comprend KRAB ou un variant de KRAB, ou
(iii) le régulateur transcriptionnel comprend une pluralité de régulateurs transcriptionnels différents.

10. Système selon la revendication 1, dans lequel :
(i) la modification du niveau d'expression et/ou du niveau de méthylation du gène cible entraîne une régulation à la baisse d'un gène en aval du gène cible, le gène en aval comprenant un ou plusieurs membres choisis dans le groupe comprenant ZSCAN4, LEUTX, MBD3L2, TRIM48 et TRIM43, ou
(ii) la modification du niveau d'expression et/ou du niveau de méthylation du gène cible entraîne une régulation à la baisse d'un marqueur d'apoptose dans la cellule musculaire, facultativement dans lequel le marqueur d'apoptose comprend de la caspase 3, ou
(iii) le complexe entraîne la modification du niveau d'expression du gène cible dans le gène musculaire, dans lequel la modification du niveau d'expression entraîne facultativement une régulation à la baisse du gène cible, ou
(iv) le complexe entraîne la modification du niveau de méthylation du gène cible dans le gène musculaire, facultativement dans lequel la modification du niveau de méthylation entraîne une régulation à la baisse du gène cible.

11. Composition comprenant le système selon l'une quelconque des revendications précédentes.

12. Vecteur viral comprenant le système selon l'une quelconque des revendications 1 à 10, facultativement dans lequel le vecteur viral comprend un virus adéno-associé (AAV), un rétrovirus, un lentivirus, un poxvirus ou un adénovirus.

13. Vecteur viral selon la revendication 12, dans lequel le vecteur viral comprend un virus adéno-associé (AAV), un rétrovirus, un lentivirus, un poxvirus ou un adénovirus, facultativement dans lequel l'AAV comprend un AAV de sérotype RH74.

14. Procédé *in vitro* ou *ex vivo* pour réguler l'expression aberrante d'un gène cible dans une cellule musculaire, comprenant les étapes consistant à :
(a) mettre en contact la cellule musculaire avec le complexe du polypeptide hétérologue et de la molécule d'acide nucléique guide du système selon l'une quelconque des revendications 1 à 10 ; et
(b) lors de la mise en contact, lier le gène cible au complexe pour entraîner une modification du niveau d'expression et/ou du niveau de méthylation du gène cible dans la cellule musculaire.

15. Complexe du polypeptide hétérologue et de la molécule d'acide nucléique guide du système selon l'une quelconque des revendications 1 à 10, pour utilisation dans un procédé de régulation de l'expression aberrante d'un gène cible dans une cellule musculaire, comprenant les étapes consistant à :
(a) mettre en contact la cellule musculaire avec le complexe du polypeptide hétérologue et de la molécule d'acide nucléique guide du système ; et
(b) lors de la mise en contact, lier le gène cible au complexe pour effectuer une modification du niveau d'expression et/ou du niveau de méthylation du gène cible dans la cellule musculaire,
facultativement dans lequel la mise en contact comprend une injection d'une composition comprenant le complexe à un sujet qui en a besoin, dans lequel le sujet présente ou est suspecté de présenter une dystrophie musculaire facio-scapulo-humérale (FSHD).
